(19) Europäisches Patentamt
European Patent Office
Office européen des brevets

(11) EP 2 350 655 B1

(12) EUROPEAN PATENT SPECIFICATION

(45) Date of publication and mention of the grant of the patent:
**21.12.2016 Bulletin 2016/51**

(21) Application number: **09825245.5**

(22) Date of filing: **28.10.2009**

(51) Int Cl.:
*G01N 33/53* (2006.01) *G01N 33/569* (2006.01)
*G01N 33/574* (2006.01) *G01N 33/68* (2006.01)

(86) International application number:
**PCT/US2009/062382**

(87) International publication number:
**WO 2010/053788 (14.05.2010 Gazette 2010/19)**

(54) **METHODS FOR USING ANTIBODIES AND ANALOGS THEREOF**

VERFAHREN ZUR VERWENDUNG VON ANTIKÖRPERN UND ANALOGA DARAUS

PROCÉDÉS D'UTILISATION D'ANTICORPS ET DE LEURS ANALOGUES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.10.2008 US 197601 P**
**21.09.2009 US 563330**
**27.10.2009 US 606818**

(43) Date of publication of application:
**03.08.2011 Bulletin 2011/31**

(73) Proprietor: **ICB International, Inc.**
**San Diego, CA 92130 (US)**

(72) Inventors:
- **BHATT, Ram, S.**
  **San Diego,CA 92130 (US)**
- **BHATT, Rishi**
  **San Diego,CA 92130 (US)**

(74) Representative: **Schiweck, Weinzierl & Koch**
**European Patent Attorneys**
**Landsberger Straße 98**
**80339 München (DE)**

(56) References cited:

| | |
|---|---|
| **EP-A1- 1 978 035** | **WO-A1-2006/054991** |
| **US-A- 5 800 988** | **US-A1- 2005 009 050** |
| **US-A1- 2005 152 896** | **US-A1- 2006 246 477** |
| **US-A1- 2007 117 151** | **US-A1- 2007 160 616** |

- **SAERENS D ET AL: "Engineering camel single-domain antibodies and immobilization chemistry for human prostate-specific antigen sensing", ENGINEERING CAMEL SINGLE-DOMAIN ANTIBODIES AND IMMOBILIZATION CHEMISTRY FOR HUMAN PROSTATE-SPECIFIC ANTIGEN SENSING,, vol. 77, no. 23, 1 December 2005 (2005-12-01), pages 7547-7555, XP002670982, DOI: DOI:10.1021/AC051092J**
- **DIRK SAERENS ET AL: "Identification of a universal VHH framework to graft non-canonical antigen-binding loops of camel single-domain antibodies", JOURNAL OF MOLECULAR BIOLOGY, ACADEMIC PRESS, UNITED KINGDOM, vol. 352, no. 3, 23 September 2005 (2005-09-23), pages 597-607, XP002629673, ISSN: 0022-2836, DOI: 10.1016/J.JMB.2005.07.038 [retrieved on 2005-07-27]**
- **SHERWOOD LAURA J ET AL: "Rapid assembly of sensitive antigen-capture assays for Marburg virus, using in vitro selection of llama single-domain antibodies, at biosafety level 4.", THE JOURNAL OF INFECTIOUS DISEASES 15 NOV 2007 LNKD- PUBMED:17940952, vol. 196 Suppl 2, 15 November 2007 (2007-11-15), pages S213-S219, XP002673501, ISSN: 0022-1899**
- **RYAN SHANNON ET AL: "Single-domain antibody-nanoparticles: promising architectures for increased staphylococcus aureus detection specificity and sensitivity.", BIOCONJUGATE CHEMISTRY 21 OCT 2009 LNKD-PUBMED:19751063, vol. 20, no. 10, 21 October 2009 (2009-10-21), pages 1966-1974, XP002673502, ISSN: 1520-4812**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- EGLETON ET AL.: 'Development of Neuropeptide Drugs that Cross the Blood-Brain Barrier.' NEURORX: THE JOURNAL OF THE AMERICAN SOCIETY FOR EXPERIMENTAL NEUROTHERAPEUTICS vol. 2, no. 1, January 2005, pages 44 - 53, XP025343798

## Description

## FIELD OF THE INVENTION

This invention relates to the use of single-domain heavy-chain only camelid antibodyanalogs without the light-chains.

## BACKGROUND OF THE INVENTION

The occurrence of various cancers and diseases usually involves pathological antigens, altered protein expression, and/or distribution [Nature, 422, 226 (2003)]. The detection of low levels of certain protein biomarkers can be extremely useful for the diagnosis, prognosis and treatment of specific cancers and other diseases for effective disease control and/or treatment.

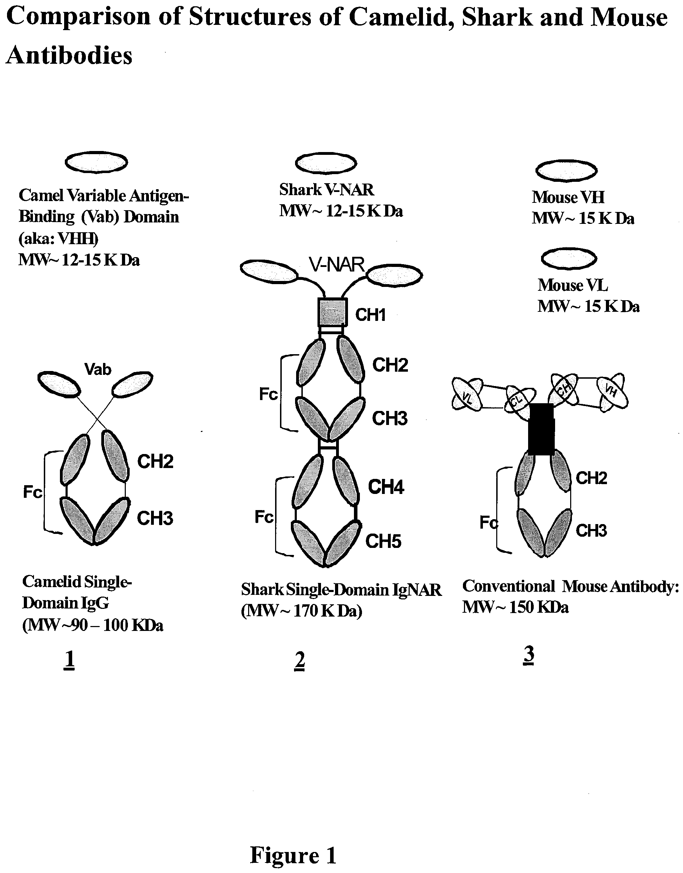

A class of naturally occurring antibodies have been identified from camelids and sharks. In addition to classical heterotetrameric antibodies, camelids and sharks also produce so called "incomplete antibodies" without the light-chains. Their structure is shown in figure 1.

Thus, two types of antibodies exist in camels, dromedaries and llamas: one a conventional hetero-tetramer having two heavy and two light chains (MW -160 K Da), and the other consisting of only two heavy chains, devoid of light chains (MW -90 KDa) and also deprived of constant region CHI.

In addition to camelid antibodies having only two heavy chains and devoid of light chains, distinctly unconventional antibody isotype was identified in the serum of nurse sharks (Ginglymostoma cirratum) and wobbegong sharks (Orectolobus maculatus). The antibody was called the immunoglobulin (Ig) new antigen receptors (IgNARs). They are disulfide-bonded homodimers consisting of five constant domains (CNAR) and one variable domain (VNAR). There is no light chain, and the individual variable domains are independent in solution and do not appear to associate across a hydrophobic interface. Like the Vab domain of camelid antibodies, the variable antigen-binding domain , known as V-NAR, of single-domain shark antibodies is also stable by itself and has a molecular weight of about 15 KDa (Greenberg, A. S., Avila, D., Hughes, M., Hughes, A., McKinney, E. & Flajnik, M. F., Nature, 374, 168-173 (1995); Mol. Immunol. 38, 313-326, (2001); Comp. Biochem. Physiol. B., 15, 225 (1973)). There are three different types of IgNARs characterized by their time of appearance in shark development, and by their disulfide bond pattern [Diaz, M., Stanfield, R. L., Greenberg, A. S. & Flajnik, M. F., Immunogenetics 54, 501-512 (2002); Nuttall, S. D., Krishnan, U. V., Doughty, L., Pearson, K., Ryan, M. T., Hoogenraad, N. J., Hattarki, M., Carmichael, J. A., Irving, R. A. & Hudson, P. J., Eur. J. Biochem., 270, 3543-3554 (2003)].

**Relevant References: Foreign and US Patents:**

| | |
|---|---|
| US Patent Application US 2010/0092470 September 21, 2009 | Antibodies, Analogs and Uses Thereof |
| WO 2010/033913 September 21,2009 | Antibodies, Analogs and Uses thereof |
| US Patent 7371849 (May, 2008) | Methods of constructing camel antibody libraries. |
| US Patent 6838254 B1 (Jan., 2005) | Production of antibodies or fragments thereof derived from heavy-chain immunoglobulins of camelidae. |
| US Patent 6765087 (July, 2004) | Immunoglobulins devoid of light chains. |
| US Patent 6005079 (Dec, 1999) | Immunoglobulins devoid of light chains. |
| US Patent 5800988 (Sept., 1998) | Immunoglobulins devoid of light chains. |
| WO/2002/048193 (June, 2002) | Camelidae Antibody Arrays. |
| EP 1264885 (Dec, 2002) | Antibody library. |
| WO/2001/090190 (Nov., 2001) | Single-domain antigen-binding antibody fragments derived from llama antibodies. |
| WO/2000/043507 (July, 2000) | Methods for producing antibody fragments. |
| EP 1024191 (August, 2000) | Production of chimeric antibodies from segment repertoires and displayed on phage. |
| WO/1999/042077 (August, 1999) | Recognition molecules interacting specifically with the active site or cleft of a target molecule. |

(continued)

| | |
|---|---|
| US Patent Application US 2010/0092470 September 21, 2009 | Antibodies, Analogs and Uses Thereof |
| US 2007/0117151 | Method and apparatus for detecting an analyte using a specific substrate, Here a camelid VHH (full VHH) bound to a solid support is disclosed. |
| EP 1978 035 | Anti-amyloid antibodies and their use in diagnosis and therapy. Here specific camelid VHH antbodies are disclosed. |
| US 2006/0246477 | Method for generating variable domain Sequences of heavy chain antibodies. Here a method for generation of Vabs of VHHs is disclosed. |
| US 5,800,988 | Immunoglobulins devoid of light chains Here heavy chain only antibodies are disclosed. |

**Other References:**

Azwai SM et al, Serology of Orthopoxvirus Camel Infection in Dromedary camels, Comp. Immun. Microbiol. Infect. Dis., 19, 65 (1996).

Kelly PJ, et al, Isolation and characterization of immunoglobulin-G and IgG Subclasses of the African elephant, Comp. Immun. Microbiol. Infect. Dis., 2J_, 65 (1998).

Linden Richard van der et al., Induction of immune responses and molecular cloning of the heavy-chain antibody repertoire of Lama glama, J. Immun. Methods, 240, 185 (2000).

Rivera H et al., Serological survey of viral antibodies in the Peruvian alpacas, Am. J. Vet. Res.,48, 189(1987).

Kumar M et al., Biochemical studies on Indian Camels: Blood proteins and lipids, J. Sci. Industrial Res., 20c, 236 (1961).

Ungar-Waron H., Elias E., et al, Dromedary IgG: Prurification, characterization and quantitation in Sera of Dams and newborns, Isr. J. Vet. Med., 43 (3), 198 (1987).

Azwai SM et al., The isolation and characterization of camel immunoglobulin classes and subclasses, J. Comp. Path., 109, 187 (1993).

Hamers-Casterman C, Atarhouch T., et al., Naturally occurring antibodies devoid of light chains, Nature, 363, 446 (1993).

Zhang J, Tanha J. et al., Pentamerization of single-domain antibodies from phage libraries: a novel strategy for the rapid generation of high-avidity antibody reagents, J. Mol. Biol, 335, 49 (2004).

Saerens D et al., Engineering Camel Single-Domain Antibodies and Immobilization Chemistry for Human Prostate-Specific Antigen Sensing, Anal. Chem. 77:7547-7555 (2005). Here camelid VHH boind to biotin are disclosed.

Saerens D. et al., Identification of a Universal VHH Framework to Graft Non-canonical Antigen-binding Loops of Camel Single-domain antibodies; JMB 352:597-607 (2005). Here a scaffold for antibody production is disclosed.

Sherwood, L J, Rapid assembly of Sensitive Antigen-Capturi Assays for Marburg Virus, Using In Vitro Selection of Llama Single-Domain Antibodies, at Biosafety level 4; JID 296(Suppl 2):S213-219 (2007). Here specific Llama VHHs for detectin of the Marburg virus are disclosed.

Ryan S, Single-Domain Antibody-Nanoparticles: Promising Architechtures for Increased Staphylococcus aureus Detection Specificity and Sensitivity; Bioconjugate Chem 20:1966-1974 (2009). Here the use of VHHs with supramagnetic particles is disclosed.

## SUMMARY OF THE INVENTION

The present invention relates to an ultrasensitive and ultraspecific method for the detection of antigens and useful for diagnosing human diseases using camelid heavy chain only antibody analogs.

Specifically, the sub-nano-antibody analogs of the invention have the structure:

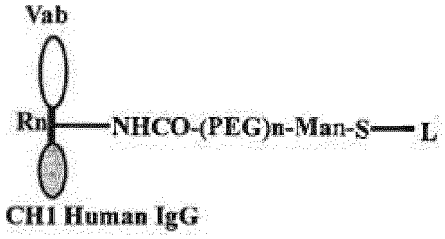

wherein:

(i) Vab is one camelid variable region of a single-domain heavy chain antibody lacking light chains,
(ii) Rn represents all of the hinge region of camelid single domain heavy chain antibodies,
(iii) n is 1-50,
(iv) Man is Maleic Anhydride and
(v) **S- L** is a thiolated ligand; wherein L is a solid matrix.

In one aspect, the disclosure provides a method for detecting the presence or absence of an antigen in a sample. The method includes a) obtaining a sample suspected of having said antigen, b) detecting the presence or absence of the antigen in the sample utilizing a polypeptide in which the polypeptide comprises all or a portion of at least one variable antigen-binding (Vab) domain of camelid and/ or shark single-domain heavy chain antibodies lacking light-chains, at least ten contiguous amino acids derived from a source other than camelid and/ or shark single-domain heavy chain antibodies lacking light-chains and the polypeptide comprises at least one binding site for an antigen. The polypeptide binds specifically to the antigen and the binding is indicative of the presence of the antigen.

In another aspect, the disclosure provides a method for detecting the presence or absence of an antigen in a sample. The method includes a) obtaining a sample suspected of having said antigen, b) detecting the presence or absence of the antigen in the sample utilizing a composition having at least two polypeptides, in which each of the polypeptides includes all or a portion of at least one variable (Vab) domain of camelid and or shark single domain heavy chain antibody lacking light chain, all or a portion of at least one hinge region of camelid and or shark single domain heavy chain antibody lacking light chain in which at least one of the polypeptide includes at least one binding site for an antigen, and the polypeptides are linked to each other through at least one linker. The polypeptide binds specifically to the antigen and the binding is indicative of the presence or absence of the antigen. In one disclosure, at least one linker is a peptide bond. In another disclosure, at least one linker is other than a peptide bond. In one disclosure, the polypeptides of the composition include at least three, at least four, at least five or more variable antigen-binding (Vab) domains of camelid and or shark single domain heavy chain antibody. In some embodiments, the polypeptide may include one or more substitutions or deletions of the native amino acids.

Also disclosed is a method to improve the biodistribution and retention of the heavy chain only camelid and shark antibodies without light-chains and their analogs. In one disclosure, the molecular weight is greater than 15 to 17 KDa and can enter a cell or cross blood brain barrier (BBB), they are retained inside the cell to be diagnostically/therapeutically efficacious. In some cases, the molecular weight of the antibodies and their analogs are between ~ 30 to 60 KDa, more preferably 40 to 60 KDa, ideally ~ 55 KDa. In one example the disclosure encompasses the synthesis of a polypeptide with two or more variable antigen-binding domains to generate the polypeptide with a MW ~ 30 to 60 KDa, more preferably 40 to 60 KDa, ideally - 55 KDa. The polypeptide comprises camelid Vab domains and /or shark V-NAR domains, in which such constructions/preparations are performed either chemically and/or via recombinant DNA methods.

In another aspect, the disclosure provides a method for detecting an organism or a cell in a sample. The method includes a) obtaining a sample suspected of having such cell or organism, b) detecting the presence or absence of one or more antigens associated with the organism or a cell by utilizing the polypeptides or compositions of the above aspects of the invention such that the polypeptides or the compositions bind specifically to one or more antigens associated with the cell or organism and the binding is indicative of the presence or absence of a cell or organism in the sample. In some embodiments, the organism is a pathogenic organism such as bacteria or virus.

In another aspect, the disclosure provides a method for diagnosing an individual with one or more diseases.

The method includes: a) obtaining a sample of bodily fluid from the individual; b) detecting the presence or absence of one or more biomarkers associated with the disease in which the detection comprises utilizing a polypeptide in which the polypeptide comprises all or a portion of at least one variable antigen-binding (Vab) domain of camelid and/ or shark single-domain heavy chain antibodies lacking light-chains, at least ten contiguous amino acids derived from a source other than camelid and/ or shark single-domain heavy chain antibodies lacking light-chains, the polypeptide binds specifically to at least one of said biomarkers and the binding of the polypeptide to one or more of the biomarkers is indicative of the presence of one or more biomarkers in the sample; c) identifying the individual as having the disease when one or more biomarkers are present in the individual's sample. In some embodiments, the method further includes determining the amount of one or more biomarkers in the sample and comparing the amount to reference values. An amount higher or lower than the reference value is indicative of a disease. In some embodiments, the reference values are the levels of the biomarkers in an individual without such one or more diseases.

In one disclosure, the polypeptide described above comprises at least two variable antigen-binding (Vab) domains of camelid and/or shark single-domain heavy- chain antibody lacking the light chains. In another disclosure, the polypeptide described above includes at least three, at least four or more variable (Vab) domains of camelid and shark heavy chain only antibody. The polypeptide may include one or more substitutions or deletions of the native amino acids. In some example, at least two variable antigen-binding (Vab) domains bind to two different antigens. The polypeptide described above may include all or a portion of at least one hinge region of camelid and /or shark single domain heavy chain antibody lacking light chain.

In one disclosure of all of the above aspects, the polypeptide includes all or a portion of at least one camelid and or shark single domain heavy chain constant domain 2 (CH2). In one disclosure of all of the above aspects, the polypeptide includes all or a portion of at least one camelid and or shark single domain heavy chain constant domain 3 (CH3). In one example of all of the above aspects, at least one amino acid at positions 37, 44, 45, and 47 of the Vab region is selected from the group consisting of serine, glutamine, tyrosine, histidine, asparagine, threonine, aspartic acid, glutamic acid, lysine and arginine. In some cases, the polypeptide may include one or more substitutions or deletions of the native amino acids.

In some cases of the above aspects, the polypeptide may include domains (such as variable domain or constant domain) from at least two different species such as camelid and shark, or two different camelid species such as llama, camel, alpaca and dromedaries. In some of the above aspects, the polypeptide may have improved cellular uptake, blood brain barrier permeability, biodistribution and retention.

In some embodiments the polypeptide disclosed above is immobilized on a solid support prior to binding to said antigen. In some embodiments the polypeptide of the above aspect of the invention binds to the antigen to form a complex and the complex is immobilized on a solid support. In one embodiment, the immobilization is achieved by covalent attachment of the polypeptide to the solid surface through a spacer. In one embodiment, the length of the spacer is 1-100 nm in length. In one embodiment, the length of the spacer is 1-50 nm. In another embodiment, the length is 20 nm.

In the above aspects of the invention, the polypeptide is linked to at least one entity other than an antibody. The entity can be solid support. Also disclosed are: radioisotope, enzyme, detectable label, ligand, fluorophore, biotin, digoxegenin, avidin, streptavidin, Fc region of IgGs, a therapeutic agent, toxin, hormone, peptide, protein, vector, siRNA, micro-RNA or nucleic acid. In some embodiments, the solid support can be beads, biosensors, nanoparticles, microchannels, microarrays, and microfluidic devices, glass slides, glass chambers, or gold particles. In some examples, the enzyme can be alkaline phosphatase (AP), horse-raddish-peroxidase (HRP), Luciferase, and beta-galactosidase. In some embodiments, the bead can be 1-200 micrometer in diameter, preferably 1-10 micrometer in diameter.

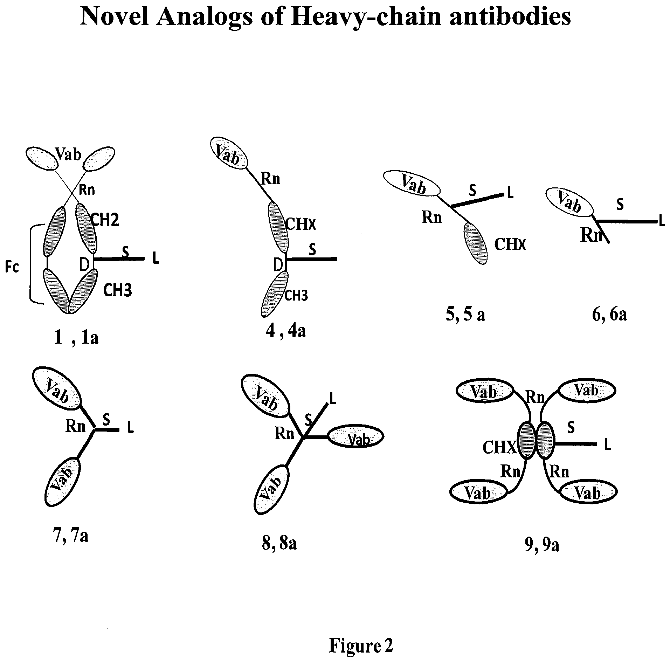

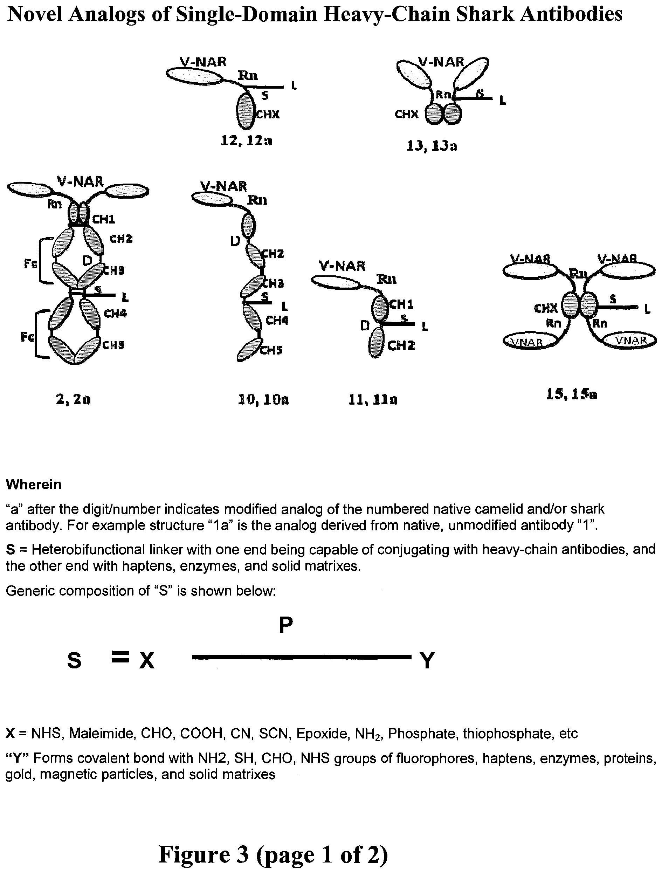

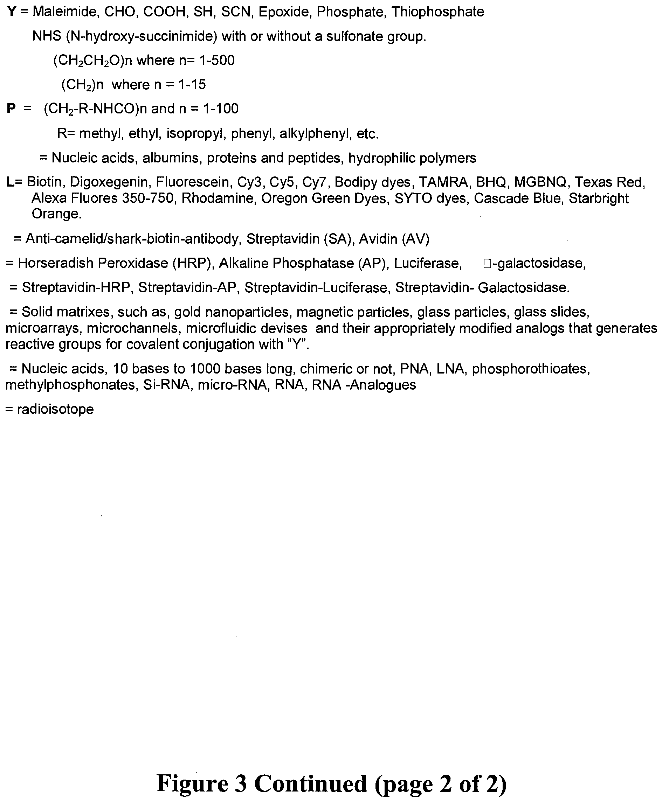

Disclosed are polypeptides or the compositions of the above aspects having structures 1, 1a, 4, 4a, 5, 5a, 6, 6a, 7,7a, 8, 8a, 9, 9a, 10, 10a (Figure 2), wherein "a" represents analog of the unmodified parent antibody. For example, 1, 1a represent native unmodified structure 1 without "S" and "L" whereas la contains modified structure 1 comprising of "S" and "L". Also, "CHX" in figures 2 and 3 represents at least ten contiguous amino acids derived from a source other than camelid and/ or shark single-domain heavy chain antibodies lacking light-chains; "S" represents a linker; "Rn" represents all or a portion of at least one camelid or shark hinge region of single domain heavy chain antibody; "L" represents an entity linked to the polypeptide, and Vab represents camelid or shark variable region of single domain heavy chain antibody, "D" represents at least two amino acids comprising at least one charged amino acid between the two domains of the camelid and shark antibodies.

Also disclosed are polypeptides or compositions of the above aspects having structures 2, 2a, 11, 11a, 12, 12a, 13, 13a, 14, 14a, and 15, 15a. (Figure 3), wherein "a" represents analog of the unmodified parent antibody. For example, 2, represents native unmodified structure 2 and 2a represents modified structure with "S" and "L" Also, "CHX" in figures 2 and 3 represents at least ten contiguous amino acids derived from a source other than camelid and/ or shark single-domain heavy chain antibodies lacking light-chains; "S" represents a linker; "Rn" represents all or a portion of at least one camelid or shark hinge region of single domain heavy chain antibody; L represents an entity linked to the polypeptide, and Vab represents camelid or shark variable region of single domain heavy chain antibody, "D" represents

at least two amino acids comprising at least one charged amino acid, VNAR represents shark variable antigen-binding region of single domain heavy chain only shark antibody without the light-chains. CHI, CH2, CH3, CH4 and CH5 represent five constant domains of shark antibodies.

Also disclosed is a generic composition of the antibody polypeptide is represented by:

**[Vab]m- S-L**

in which

Vab = Variable antigen-binding domain of camelid and/or shark single domain heavy chain antibodies;

m = 1 to 10, preferably 2 to 5 such that the MW is approximately between 15 to 65 KDa for optimal biodistribution and retention in the body;

"S" is selected from the group consisting of groups I and II in which group I includes 120 amino acids of the hinge region of camelid and /or shark single domain heavy chain antibodies comprising at least one lysine and /or cysteine, and group II includes hetrobifunctional linker with one end being capable of covalent binding with amino- or aldehyde group of single-domain antibodies, and the other end with an entity "L";

"L" represents an entity linked to Vab domain. "L" can be a detectable label, enzyme or protein (for example, horse radish peroxidase, alkaline phosphatase, luciferase, beta-galactosidase, and streptavidin), antibody, nucleic acid (for example, DNA, Modified DNA, Locked-DNA, PNA (Peptide Nucleic Acids), RNA, Si-RNA, Micro-RNA(MiRNA), mRNA, RNA-Conjugates/ Modifications), radionucleotides (for example, Fluorine-18, Gallium-67, Krypton-81m, Rubidium-82, Technetium-99m, Indium-Ill, Iodine-123, Xenon-133, and Thallium-201, Yttrium-90, and Iodine-131), toxins (for example, Immunotoxins, Ricin, Saporin, Maytansinoid, and Calicheamicin), solid support (for example, Microchannels, Microfluidic Device, Microarrays, Biosensors, Glass Slides, Glass Chambers, Magnetic Beads, and Gold Nanoparticles), and therapeutic agents (for example, nucleolytic enzymes, antibiotics, and chemotherapeutic agents such as Paclitaxel its derivatives).

In one example, the generic composition of "S" is S = X-P-Y in which X can be of NHS (N-Hydroxy-Succinimide), sulfo-NHS, CHO, COOH, CN, SCN, epoxide, phosphate and other moieties capable of forming covalent bond with NH2 groups of single-domain antibodies; Y can be maleimido, NHS, sulfo-NHS, SH, COOH, SCN, NH2, and epoxide, capable of forming a covalent bond with the thiol group of the detectable label; P can be (CH2CH20)n, wherein n- 1-500; DNA, modified DNA, modified RNA; $(CH_2)n^1$, wherein $n^1$ = 1-15; $(Ra\text{-}NHCO)n^2$, wherein $n^2$ = 1-100; Ra = charged amino acid; nucleic acids; nylon, polystyrene; polypropylene; protein; and chimeric protein-nucleic acids.

In some embodiments, the disease may be cancer, Parkinson's disease, Alzheimer's disease, AIDS, Lyme disease, malaria, SARS, Down syndrome, anthrax, salmonella or bacterial botulism, staphylococcus aureus. In some embodiments, the cancer can be lung cancer, bladder cancer, gastric cancer, ovarian cancer, brain cancer, breast cancer, prostate cancer, cervical cancer, ovarian cancer, oral cancer, colorectal cancer, leukemia, childhood neuroblastoma, or Non-Hodgkin's lymphoma.

In some embodiments of the above aspects of the invention, the polypeptide can bind specifically one or more biomarkers. Exemplary biomarkers include AMACR, TMPRSS2-ERG, HAAH, APP, Aβ42, ALZAS, Tau, gamma secretase, beta secretase, PEDF, BDNF, Cystatin C, VGF nerve growth factor inducible, APO-E, GSK-3 binding protein, TEM1, PGD2, EGFR, ESR-1, HER-2/neu, P53, RAS, SMAD4, Smad7, TNF-alfa, HPV, tPA, PCA-3, Mucin, Cadherin-2, FcRn alpha chain, cytokeratins 1-20, Apo-H, Celuloplasmin, Apo AII, VGF, Vif, LEDGF/p75, TS101, gp120, CCR5, HIV protease, HIV integrase, Bacillus anthracis protein, NadD (Nicotinate Mononucletide Adenyltransferase), Plasmodium falciparum, cGMP directed phosphodiestrase, chain B of Clostridium botulinum neurotroxin type E protein, Borrelia VlsE protein, ACE2 receptor, SFRS4, or SAMP.

In some embodiments of the above aspects of the invention, the polypeptide may specifically bind to the biomarkers associated with Alzheimer's disease. Exemplary biomarkers associated with Alzheimer's disease include but are not limited to Amyloid-beta, ALZAS, Tau, Cyclophilin-D (Cyp-D), Abeta binding alcohol dehydrogenase (ABAD), N-methyl-D-aspartate receptor (NMDAR), mSOD1, mHTT (mutant huntingtin), 3-NP, phosphatidylserine (PtDS), MPTP, integrin α4β1, integrin- α4β7, PPAR-γ, MAdCAM-1, DJ-1, Bax-1, PEDF, HPX, Cystatin-C, Beta-2-Microglobulin, BDNF, Tau-Kinase, γ-Secretase, β-Secretase, Apo-E4, and VGF-Peptide, TOM, hPReP, PLSCR1, integrin- DJ-1, and enzymes involved in the mitochondrial and myelin dysfunction.

In some embodiments of the above aspects of the invention, the polypeptide may specifically bind to the

biomarkers associated with Parkinson's disease. Exemplary biomarkers associated with Parkinson's disease include but are not limited to Apo-H, Cerulopasmin, Chromogranin-B, VDBP, Apo-E, Apo-AII, and alfa-Synuclein.

In some embodiments of the above aspects of the invention, the polypeptide may specifically bind to the biomarkers associated with brain cancer. Exemplary biomarkers associated with brain cancer include but are not limited to TEM1, Plasmalemmal Vesicle (PV-1), Prostaglandin D Synthetase, and (PGD-S).

In some embodiments of the above aspects of the invention, the polypeptide may specifically bind to the biomarkers associated with HIV/AIDS. Exemplary biomarkers associated with HIV/AIDS include but are not limited to gp120, Vif, LEDGF/p75, TS101, HIV-Integrase, HIV-Reverse Transcriptase, HIV-Protease, CCR5, and CXCR4.

In some embodiments of the above aspects of the invention, the polypeptide may specifically bind to the biomarkers associated with lung cancer. Exemplary biomarkers associated with lung cancer include but are not limited to KRAS, Ki67, EGFR, KLKB1, EpCAM, CYFRA21-1, tPA, ProGRP, Neuron-specific Enolase (NSE), and hnRNP.

In some embodiments of the above aspects of the invention, the polypeptide may specifically bind to the biomarkers associated with prostate cancer. Exemplary biomarkers associated with prostate cancer include but are not limited to AMACR, PC A3, TMPRSS2-ERG, HEPSIN, B7-H3, SSeCKs, EPCA-2, PSMA, BAG-1, PSA, MUC6, hK2, PCA1, PCNA, RKIP, and c-HGK.

In some embodiments of the above aspects of the invention, the polypeptide may specifically bind to the biomarkers associated with breast cancer. Exemplary biomarkers associated with breast cancer include but are not limited to EGFR, EGFRT790M, HER-2, Notch-4, ALDH-1, ESR1, SBEM, HSP70, hK-10, MSA, p53, MMP-2, PTEN, Pepsinigen-C, Sigma-S, Topo-11-alfauKPA, BRCA-1, BRCA-2, SCGB2Al,and SCGB1D2.

In some embodiments of the above aspects of the invention, the polypeptide may specifically bind to the biomarkers associated with colorectal cancer. Exemplary biomarkers associated with colorectal cancer include but are not limited to SMAD4, EGFR, KRAS, p53, TS, MSI-H, REGIA, EXTL3, plK3CA, VEGF, HAAH, EpCAM, TEM8, TK1, STAT-3, SMAD-7, beta-Catenin, CK20, MMP-1, MMP-2, MMP-7,9,11, and VEGF-D.

In some embodiments of the above aspects of the invention, the polypeptide may specifically bind to the biomarkers associated with ovarian cancer. Exemplary biomarkers associated with ovarian cancer include but are not limited to CD24, CD34, EpCAM, hK8, 10, 13, CKB, Cathesin B, M-CAM, c-ETSI, and EMMPRIN.

In some embodiments of the above aspects of the invention, the polypeptide may specifically bind to the biomarkers associated with cervical cancer. Exemplary biomarkers associated with cervical cancer include but are not limited to HPV, CD34, ERCC1, Beta-CF, Id-1, UGF, SCC, p16, p21WAF1, PP-4, and TPS.

In some embodiments of the above aspects of the invention, the polypeptide may specifically bind to the biomarkers associated with bladder cancer. Exemplary biomarkers associated with bladder cancer include but are not limited to CK18, CK20, BLCal, BLCA-4, CYFRA21-1, TFT, BTA, Survivin, UCA1, UPII, FAS, and DD23.

In some embodiments of the above aspects of the invention, the polypeptide may specifically bind to the biomarkers associated with a pathogenic bacteria. Exemplary pathogenic bacteria include but are not limited to Clostridium Botulinum (Bacterial Botulism), Bacillus Anthracis (Anthrax), Salmonella Typhi (Typhoid Fever), Treponema Pallidum (Syphilis), Plasmodinum (Malaria), Chlamadyia (STDs), Borrelia B (Lyme disease), Staphyloccus Aureus, Tetanus, Meningococcal Meningitis (Bacterial Meningitis), and Mycobacterium tuberculosis (Tuberculosis, TB), and NadD (Nicotinate Mononucleotide Adenyltransferase, an enzyme involved in inducing resistance to antibiotics).

In some embodiments of the above aspects of the invention, the polypeptide may specifically bind to the biomarkers associated with a pathogenic virus. Exemplary pathogenic virus include but are not limited to Pandemic Flu Virus H1N1 strain, Influenza virus H5N1 strain, Hepatitis B virus (HBV) antigen OSt-577, HBV core antigen HBcAg (HBV), HBV antigen Wnt-1, Hepatitis C Virus (HCV) antigen Wnt-1, and HCV RNA (HCV).

In some embodiments, the antibody is produced using chemical methods as described in pending US Patent ApplicationUS 2010/0092470. Briefly, the method includes a chemical synthesis of a polypeptide comprising one, two, or more variable antigen-binding (Vab) domains using the parent antibody produced from camelid and /or shark as a starting material for generating the polypeptide with one or more Vab domains.

Also disclosed is a method for generating polypeptides comprising multivalent variable antigen-binding domains improving binding affinity between antibody and its antigen.

The antibody can be produced using recombinant DNA methods as described in pending US Patent Application published as US 2010/0092470. Briefly, the method includes isolating the RNA from lymphocytes, reverse-transcription with oligo-dT priming, amplification of the generated cDNA encoding the camelid or shark antibody, inserting the amplified DNA in a phage-display vector, transforming the E.Coli cells, and selecting the clones that express highly specific antibodies.

The term "antibody" as used herein refers to immunoglobulin G (IgG) having only heavy chains without the light-chain and also constant domain 1 (CHI) in case of camelid antibodies. The shark antibody without the light-chains is known in the art as shark IgNAR. An antibody of this invention can be monoclonal or polyclonal.

The term "analog" within the scope of the term "antibody" include those produced by digestion with various proteases, those produced by chemical cleavage, chemical coupling, chemical conjugation, and those produced recombinantly, so long as the fragment remains capable of specific binding to a target molecule. Analogs within the scope of the term include antibodies (or fragments thereof) that have been modified in sequence, but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (see, e.g., Marasco (ed.), Intracellular Antibodies: Research and Disease Applications, Springer-Verlag New York, Inc. (1998) (ISBN: 3540641513). As used herein, antibodies can be produced by any known technique, including harvest from cell culture of native B lymphocytes, harvest from culture of hybridomas, recombinant expression systems, and phage display.

The terms "heavy chain only antibody" and "single domain heavy chain antibody" has been used herein interchangeably in the context of camelid and shark antibodies and refer to camelid immunoglobulin G (IgG) and shark IgNAR having only heavy chains without the heavy chain constant domain 1 (CHI) and further lacking the light chain such as camelids IgG2 and IgG3 and shark IgNAR. Heavy chain only antibody can be monoclonal or polyclonal.

The term "improved biodistribution and retention" as used herein in the context of polypeptides, antibodies and its analogs refers to polypeptides, antibodies and its analogs that can cross cell membrane and blood brain barrier (BBB) and have greater thermal and chemical stability than conventional immunoglobulin G with heavy and light chains. Typically such polypeptides, antibodies and its analogs have molecular weight between 25 to 90 KDa, preferably between 30 to 60 KDa. In some embodiments, the molecular weight is at least 25 KDa, 30 KDa, 35 KDa, 40 KDa, 45 KDa, 50 KDa, 55 KDa, 60 KDa, 65 KDa, 70 KDa, 75 KDa, 80 KDa, 85 KDa, or 90 KDa. Although larger and smaller molecular weights are possible.

The term "specifically binds to" as used herein in the context of an antibody or its analogs refers to binding of an antibody or its analogs specifically to an epitope such that the antibody or its analog can distinguish between two proteins with and without such epitope.

The terms "biomarker" and antigen is used interchangeably and refer to a molecule or group of molecules comprised of nucleic acids, carbohydrates, lipids, proteins, peptides, enzymes and antibodies which is associated with a disease, physiological condition, or an organism. An organism can be pathogenic or nonpathogenic. A biomarker may not necessarily be the reason for a disease or a physiological condition. An amount of a biomarker may be increased or decreased in disease or a physiological condition.

The term "camelid" as used herein refers to members of the biological family Camelidae in the Order: Artiodactyla, Suborder: Tylopoda. Exemplary members of this group include camels, dromedaries, llamas, alpacas, vicunas, and guanacos.

The term "shark" as used herein refers to members that belong to the super order Selachimorpha in the subclass Elasmobranchii in the class Chondrichthyes. There are more than 400 species of sharks known. Exemplary members of the class Chondrichthyes include great white sharks, houndsharks, cat sharks, hammerhead sharks, blue, tiger, bull, grey reef, blacktip reef, Caribbean reef, blacktail reef, whitetip reef, oceanic whitetip sharks, zebra sharks, nurse sharks, wobbegongs, bramble sharks, dogfish, roughsharks, and prickly sharks.

The term "a portion of in the context of antibodies such as camelid and shark heavy chain only antibodies and their analogs, or human antibodies means at least 2, 5, 10, 15, 20, 25, 30, 35, 40, 45, 50, 75, 100, 125, 150, 200, 250, 300, 350, 400 or more amino acids.

The term "a portion of in the context of hinge region of camelid and shark single domain heavy chain antibodies means at least 1, 2, 5, 15, 20, 25, 30, 35, 40, 45, 50, 75, 100, 125, 150, 200, or more amino acids of the hinge region.

The terms "diagnose" or "diagnosis" as used herein refers to the act or process of identifying or determining a disease or condition in an organism or the cause of a disease or condition by the evaluation of the signs and symptoms of the disease or disorder. Usually, a diagnosis of a disease or disorder is based on the evaluation of one or more factors and/or symptoms that are indicative of the disease. That is, a diagnosis can be made based on the presence, absence or amount of a factor which is indicative of presence or absence of the disease or condition. Each factor or symptom that is considered to be indicative for the diagnosis of a particular disease does not need be exclusively related to the particular disease; i.e. there may be differential diagnoses that can be inferred from a diagnostic factor or symptom. Likewise, there may be instances where a factor or symptom that is indicative of a particular disease is present in an individual that does not have the particular disease.

The term "reference value" as used herein means a value which can be used for comparison with a biomarker under investigation. In one case, a reference value may be the level of a biomarker under investigation from one or more individuals without any known disease. In another case, a reference value may be the level of the biomarker in an individual’s sample collected at a different time.

"Sample" or "patient sample" as used herein includes biological samples such as cells, tissues, bodily fluids, and stool. "Bodily fluids" may include, but are not limited to, blood, serum, plasma, saliva, cerebral spinal fluid, pleural fluid, tears, lactal duct fluid, lymph, sputum, urine, amniotic fluid, and semen. A sample may include a bodily fluid that is "acellular". An "acellular bodily fluid" includes less than about 1% (w/w) whole cellular material. Plasma or serum are examples of acellular bodily fluids. A sample may include a specimen of natural or synthetic origin.

The term "body fluid" or "bodily fluid" as used herein refers to any fluid from the body of an animal. Examples of body fluids include, but are not limited to, plasma, serum, blood, lymphatic fluid, cerebrospinal fluid, synovial fluid, urine, saliva, mucous, phlegm and sputum. A body fluid sample may be collected by any suitable method. The body fluid sample may be used immediately or may be stored for later use. Any suitable storage method known in the art may be used to store the body fluid sample; for example, the sample may be frozen at about 20°C to about -70°C. Suitable body fluids are acellular fluids. "Acellular" fluids include body fluid samples in which cells are absent or are present in such low amounts that the peptidase activity level determined reflects its level in the liquid portion of the sample, rather than in the cellular portion. Typically, an acellular body fluid contains no intact cells. Examples of acellular fluids include plasma or serum, or body fluids from which cells have been removed.

The term "enzyme linked immunosorbent assay" (ELISA) as used herein refers to an antibody-based assay in which detection of the antigen of interest is accomplished via an enzymatic reaction producing a detectable signal. ELISA can be run as a competitive or noncompetitive format. ELISA also includes a 2-site or "sandwich" assay in which two antibodies to the antigen are used, one antibody to capture the antigen and one labeled with an enzyme or other detectable label to detect captured antibody-antigen complex. In a typical 2-site ELISA, the antigen has at least one epitope to which unlabeled antibody and an enzyme-linked antibody can bind with high affinity. An antigen can thus be affinity captured and detected using an enzyme-linked antibody. Typical enzymes of choice include alkaline phosphatase or horseradish peroxidase, both of which generated a detectable product upon digestion of appropriate substrates.

The term "label" as used herein, refers to any physical molecule directly or indirectly associated with a specific binding agent or antigen which provides a means for detection for that antibody or antigen. A "detectable label" as used herein refers any moiety used to achieve signal to measure the amount of complex formation between a target and a binding agent. These labels are detectable by spectroscopic, photochemical, biochemical, immunochemical, electromagnetic, radiochemical, or chemical means, such as fluorescence, chemifluoresence, or chemiluminescence, electrochemiluminescence or any other appropriate means. Suitable detectable labels include fluorescent dye molecules or fluorophores.

The terms "polypeptide," "protein," and "peptide" are used herein interchangeably to refer to amino acid chains in which the amino acid residues are linked by peptide bonds or modified peptide bonds. The amino acid chains can be of any length of greater than two amino acids. Unless otherwise specified, the terms "polypeptide," "protein," and "peptide" also encompass various modified forms thereof. Such modified forms may be naturally occurring modified forms or chemically modified forms. Examples of modified forms include, but are not limited to, glycosylated forms, phosphorylated forms, myristoylated forms, palmitoylated forms, ribosylated forms, acetylated forms, ubiquitinated forms, etc. Modifications also include intramolecular crosslinking and covalent attachment to various moieties such as lipids, flavin, biotin, polyethylene glycol or derivatives thereof, etc. In addition, modifications may also include cyclization, branching and cross-linking. Further, amino acids other than the conventional twenty amino acids encoded by genes may also be included in a polypeptide.

The term "detectable label" as used herein in the context of antibody or its analogs refers to a molecule or a compound or a group of molecules or a group of compounds associated with a binding agent such as an antibody or its analogs, secondary antibody and is used to identify the binding agent bound to its target such as an antigen, primary antibody. A detectable label can also be used in to detect nucleic acids. In such cases a detectable label may be incorporated into a nucleic acid during amplification reactions or a detectable label may be associated a probe to detect the nucleic acid.

The terms "ultrasensitive" or "ultrasensitivity" as used herein in the context of antibodies refers to the detection of fewer than 200 molecules of the pathogenic proteins from patient's sample.

"Detecting" as used herein in context of detecting a signal from a detectable label to indicate the presence of a nucleic acid of interest in the sample (or the presence or absence of a protein of interest in the sample) does not require the method to provide 100% sensitivity and/or 100% specificity. As is well known, "sensitivity" is the probability that a test is positive, given that the person has a genomic nucleic acid sequence, while "specificity" is the probability that a test is negative, given that the person does not have the genomic nucleic acid sequence. A sensitivity of at least 50% is preferred, although sensitivities of at least 60%, at least 70%>, at least 80%, at least 90% and at least 99% are clearly more preferred. A specificity of at least 50% is preferred, although specificity of at least 60%, at least 70%, at least 80%, at least 90% and at least 99% are clearly more preferred. Detecting also encompasses assays with false positives and false negatives. False negative rates may be 1%, 5%, 10%, 15%, 20% or even higher. False positive rates maybe 1%, 5%, 10%, 15%, 20% or even higher.

The term "about" as used herein in reference to quantitative measurements or values, refers to the indicated value plus or minus 10%.

"Nucleic acid" as used herein refers to an oligonucleotide, nucleotide or polynucleotide, and fragments or portions thereof, which may be single or double stranded, and represent the sense or antisense strand. A nucleic acid may include DNA or RNA, and may be of natural or synthetic origin and may contain deoxyribonucleotides, ribonucleotides, or nucleotide analogs in any combination.

Non-limiting examples of polynucleotides include a gene or gene fragment, genomic DNA, exons, introns, mRNA, tRNA, rRNA, ribozymes, cDNA, recombinant polynucleotides, branched polynucleotides, plasmids, vectors, isolated DNA of any sequence, isolated RNA of any sequence, synthetic nucleic acid, nucleic acid probes and primers. Polynucleotides may be natural or synthetic. Polynucleotide may comprise modified nucleotides, such as methylated nucleotides and nucleotide analogs, uracyl, other sugars and linking groups such as fluororibose and thiolate, and nucleotide branches. A nucleic acid may be modified such as by conjugation, with a labeling component. Other types of modifications included in this definition are caps, substitution of one or more of the naturally occurring nucleotides with an analog, and introduction of chemical entities for attaching the polynucleotide to other molecules such as proteins, metal ions, labeling components, other polynucleotides or a solid support.

Nucleic acid may include nucleic acid that has been amplified (e.g., using polymerase chain reaction).

A fragment of a nucleic acid generally contains at least about 15, 20, 25, 30, 35, 40, 45, 50, 75, 100, 200, 300, 400, 500, 1000 nucleotides or more. Larger fragments are possible and may include about 2,000, 2,500, 3,000, 3,500, 4,000, 5,000 7,500, or 10,000 bases.

"Gene" as used herein refers to a DNA sequence that comprises control and coding sequences necessary for the production of an RNA, which may have a non-coding function (e.g., a ribosomal or transfer RNA) or which may include a polypeptide or a polypeptide precursor. The RNA or polypeptide may be encoded by a full length coding sequence or by any portion of the coding sequence so long as the desired activity or function is retained.

"cDNA" as used herein refers to complementary or copy polynucleotide produced from an RNA template by the action of RNA-dependent DNA polymerase activity (e.g., reverse transcriptase). cDNA can be single stranded, double stranded or partially double stranded.

cDNA may contain unnatural nucleotides. cDNA can be modified after being synthesized. cDNA may comprise a detectable label.

As used herein, "subject" or "individual" is meant a human or any other animal that has cells. A subject can be a patient, which refers to a human presenting to a medical provider for diagnosis or treatment of a disease. A human includes pre and post natal forms.

The term "patient" as used herein, refers to one who receives medical care, attention or treatment. As used herein, the term is meant to encompass a person diagnosed with a disease as well as a person who may be symptomatic for a disease but who has not yet been diagnosed.

The term "vector or phagemid" as used herein refers to a recombinant DNA or RNA plasmid or virus that comprises a heterologous polynucleotide capable of being delivered to a target cell, either in vitro, in vivo or ex-vivo. The heterologous polynucleotide can comprise a sequence of interest and can be operably linked to another nucleic acid sequence such as promoter or enhancer and may control the transcription of the nucleic acid sequence of interest. As used herein, a vector need not be capable of replication in the ultimate target cell or subject. The term vector may include expression vector and cloning vector.

Suitable expression vectors are well-known in the art, and include vectors capable of expressing a polynucleotide operatively linked to a regulatory sequence, such as a promoter region that is capable of regulating expression of such DNA. Thus, an expression vector refers to a recombinant DNA or RNA construct, such as a plasmid, a phage, recombinant virus or other vector that, upon introduction into an appropriate host cell, results in expression of the inserted DNA. Appropriate expression vectors include those that are replicable in eukaryotic cells and/or prokaryotic cells and those that remain episomal or those which integrate into the host cell genome.

The term "promoter" as used herein refers to a segment of DNA that controls transcription of polynucleotide to which it is operatively linked. Promoters, depending upon the nature of the regulation, may be constitutive or regulated. Exemplary eukaryotic promoters contemplated for use in the practice of the present invention include the SV40 early promoter, the cytomegalovirus (CMV) promoter, the mouse mammary tumor virus (MMTV) steroid-inducible promoter, Moloney murine leukemia virus (MMLV) promoter. Exemplary promoters suitable for use with prokaryotic hosts include T7 promoter, beta-lactamase promoter, lactose promoter systems, alkaline phosphatase promoter, a tryptophan (trp) promoter system, and hybrid promoters such as the lac promoter.

The term "antibody" as used herein refers to immunoglobulin G (IgG) having only heavy chains without the heavy chain constant domain 1 (CHI) and also lacking the light chain such as in shark IgNAR and camelids IgG2 and IgG3. Antibody can be monoclonal or polyclonal.

The term "analog" within the scope of the term "antibody" include those produced by digestion with various proteases, those produced by chemical cleavage, chemical coupling, chemical conjugation, and those produced recombinantly, so long as the fragment remains capable of specific binding to a target molecule. Analogs within the scope of the term include antibodies (or fragments thereof) that have been modified in sequence, but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (see, e.g., Marasco (ed.), Intracellular Antibodies: Research and Disease Applications, Springer-Verlag New York, Inc. (1998) (ISBN: 3540641513). As used herein, antibodies can be produced by any known technique, including

harvest from cell culture of native B lymphocytes, harvest from culture of hybridomas, recombinant expression systems, and phage display.

The terms "heavy chain only antibody" and "single domain heavy chain antibody" has been used herein interchangeably in the context of camelid and shark antibodies and refer to camelid immunoglobulin G (IgG) and shark IgNAR having only heavy chains without the heavy chain constant domain 1 (CHI) and further lacking the light chain such as camelids IgG2 and IgG3 and shark IgNAR. Heavy chain only antibody can be monoclonal or polyclonal.

Unless otherwise specified, the terms "a" or "an" mean "one or more" throughout this application.

**BRIEF DESCRIPTION OF THE FIGURES**

FIG. 1 shows structural differences between camel, shark, and mouse immunoglobulins (IgGs). The notations CHI, CH2, CH3, CH4, CH5 represent constant domain 2, 3, 4 of single domain heavy chain antibody of the respective species. The notations Vab and VNAR represent variable domain of camelid and shark single domain heavy chain antibodies respectively.

FIG. 2 shows the structure of exemplary analogs of camelid single-domain antibodies without the light-chains: mini-antibody 1 and its analogs 1a; micro-antibody 4 and its analogs 4a; sub-nano-antibody 5 and its analogs 5a; nano-antibody 6 and its analogs 6a; dimeric nano-antibody 7 and its analogs 7a; trimeri-nanoantibody 8 and its analogs; and tetrameric nano-antibody 9 and its analogs 9a. The notation "Rn" represents all or portion of the hinge region of camelid or shark single domain antibodies. CHX represents segment of human IgG CHI domain or CH2 domain of camelid antibody. "S" stands for a spacer or linker. "L" is a ligand.

FIG. 3 shows the structure of exemplary analogs of shark single-domain antibodies without the light-chains: Hark IgNAR 2 and its analogs 2a; shark mini-antibody 11 and its analogs 11a; shark micro-antibody 12 and its analogs 12a; shark sub-nano-antibody 13 and its analogs 13a; shark dimeric nano-antibody 14 and its analogs 14a; and shark tetrameric nano-antibody 15 and 15a. The notation "Rn" represents all or portion of the hinge region of shark single domain antibodies. CHX represents segment of human IgG or CHI domain of shark antibody. "S" stands for a spacer or linker. "L" is a ligand.

Figure 4 shows the steps involved in the chemical synthesis of exemplary analogs represented by structures 1a and 4a, respectively, of camelid mini-antibody 1 and micro-antibody 4. The notation "Rn" represents all or portion of the hinge region of camelid or shark single domain antibodies.

Figure 5 shows the steps involved in the chemical transformation of exemplary sub-nano-antibody 5 into its analogs represented by structure 5a, and the synthesis of dimeric camelid nano-antibody 7 and its analogs represented by generic structure 7a.

Figure 6 shows the steps involved in the transformation of exemplary camelid dimeric nano-antibody 7 into trimeric and tetrameric nano-antibodies. The notation "Rn" represents all or portion of the hinge region of camelid or shark single domain antibodies.

Figure 7 shows the steps involved in the cloning and expression of exemplary shark IgNAR 2, exemplary shark micro-antibody 12, exemplary shark sub-nano-antibody 13 and shark-nano-antibody 30. The notation "Rn" represents all or portion of the hinge region of camelid or shark single domain antibodies.

Figure 8 shows the steps involved in the chemical synthesis of exemplary analogs of shark antibodies without the light chains: Shark IgNAR analogs represented by structure 2a; shark mini-antibody analogs represented by structure 11a; shark micro antibody analogs represented by structure 12a; shark sub-nano-antibody analogs represented by 13a; shark dimeric nano-antibody analogs represented by 14a; and shark tetrameric -nano-antibody analogs represented by 15a. The notation "Rn" represents all or portion of the hinge region of camelid or shark single domain antibodies.

Figure 9 shows the steps involved in the chemical synthesis of exemplary shark dimeric nano-antibody 14 and its conversion into exemplary shark trimeric and tetrameric nano-antibodies 32 and 31.

Figure 10 shows the steps involved in the immobilization of exemplary single-domain camelid and shark antibodies

deprived of light chains having the structures 1, 2, 4, 5, 6, 7, 8, 9, 11, 12,13, 14, 15, 19, 20, 31, and 32.

FIG. 11 shows an exemplary scheme of capturing and detecting antigens / biomarkers associated with a disease using camelid and shark heavy chain only antibodies and their analogs.

FIG. 12 shows an exemplary scheme of capturing and detecting antigens/biomarkers associated with a disease using immobilized shark single-domain IgNAR and their analogs.

FIG. 13 shows an exemplary scheme of capturing and detecting < 200 copies of antigens/biomarkers associated with a disease using camelid and shark heavy chain only antibodies and their analogs using immuno-PCR.

Figure 14 shows an exemplary scheme of capturing and detecting circulating tumor cells from bodily fluid using camelid and shark antibodies.

FIG. 15 shows an exemplary scheme of detecting prenatal genetic disorder using captured circulating fetal cells using camelid and shark heavy chain only antibodies and their analogs.

FIG. 16 shows an exemplary scheme of detecting chromosomal translocation using captured circulating tumor cells using camelid and shark heavy chain only antibodies and their analogs.

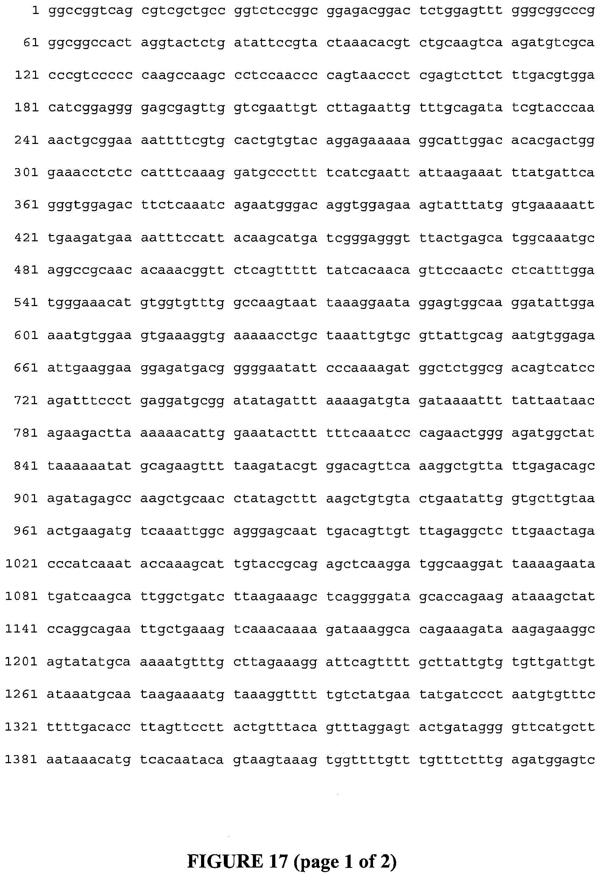

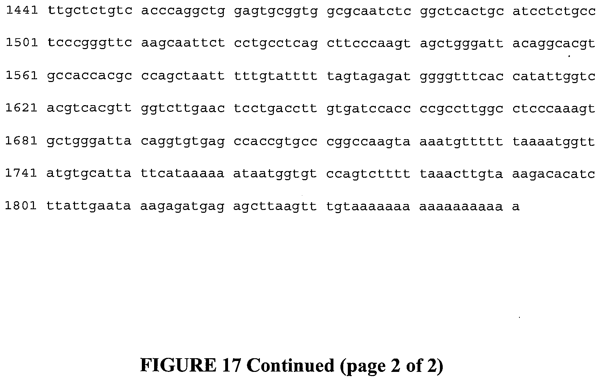

FIG. 17 shows an exemplary nucleic acid sequence encoding human Cyclophilin D.

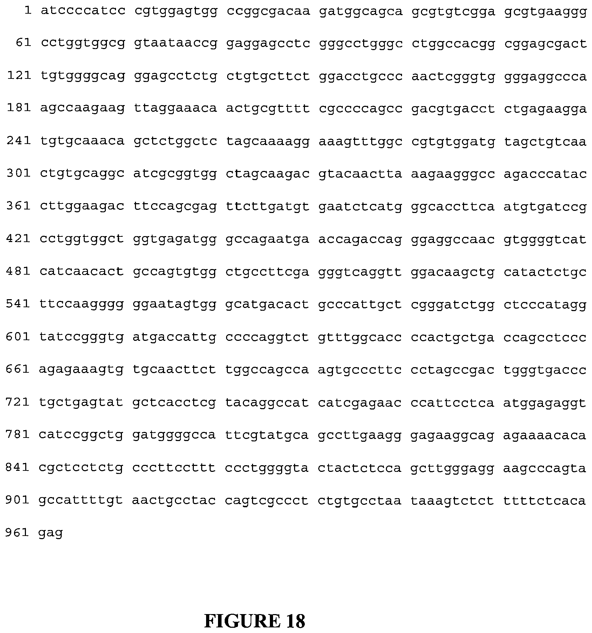

FIG. 18 shows an exemplary nucleic acid sequence encoding alpha beta binding Mitochondrial Alcohol Dehydrogenase (ABAD).

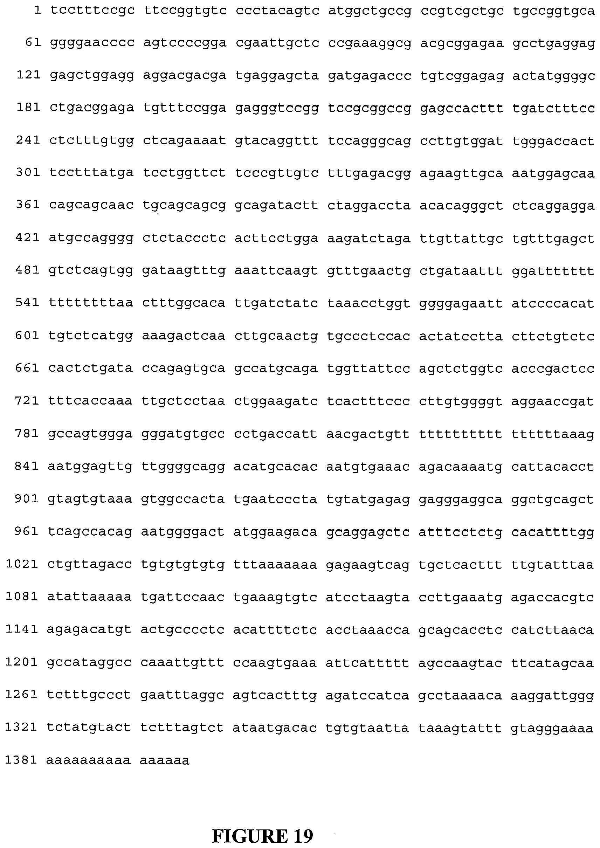

FIG. 19 shows an exemplary nucleic acid sequence encoding Translocase of the Outer Membrane (TOM).

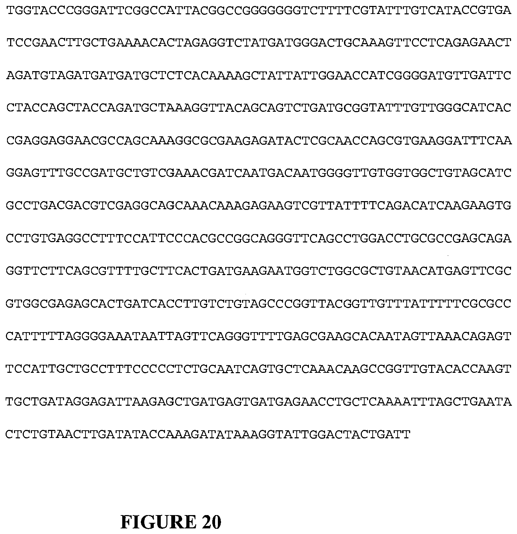

FIG. 20 shows an exemplary nucleic acid sequence encoding Prosequence Protease (hPreP).

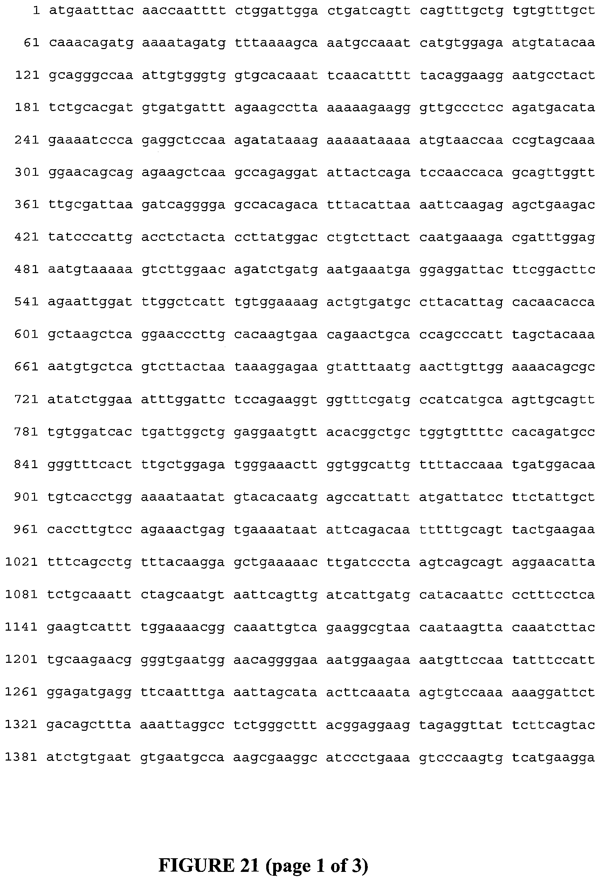

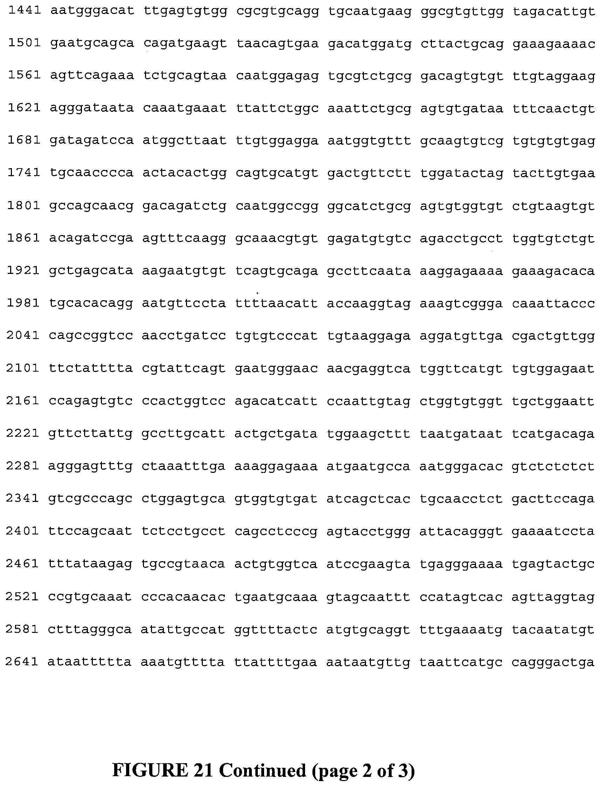

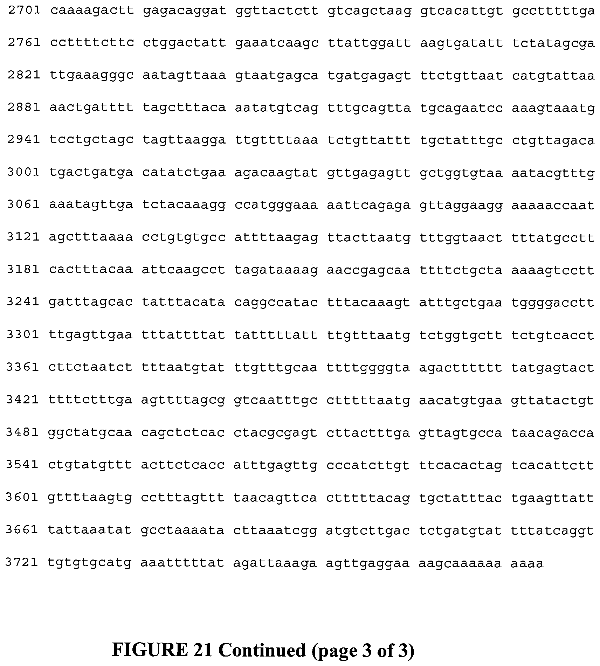

FIG. 21 shows an exemplary nucleic acid sequence encoding Homo sapiens integrin beta 1.

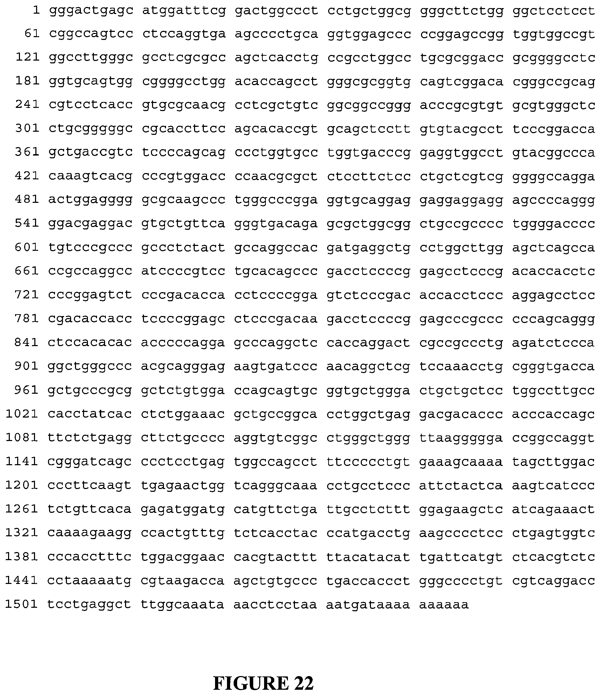

FIG. 22 shows an exemplary nucleic acid sequence encoding Homo sapiens mucosal vascular addressin cell adhesion molecule 1 (MADCAM1).

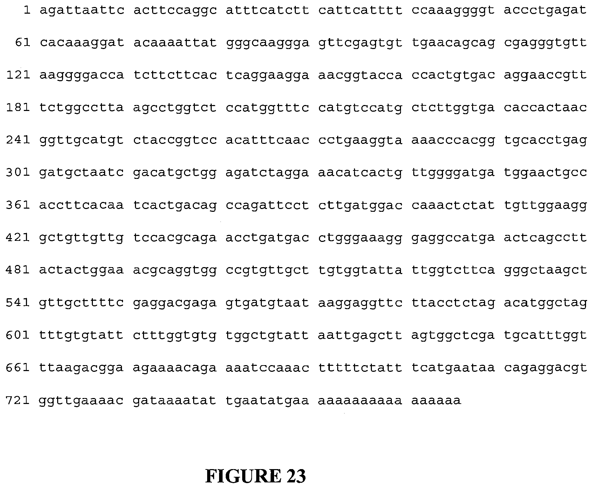

FIG. 23 shows an exemplary nucleic acid sequence encoding Cu/Zn-superoxide dismutase (mSOD1).

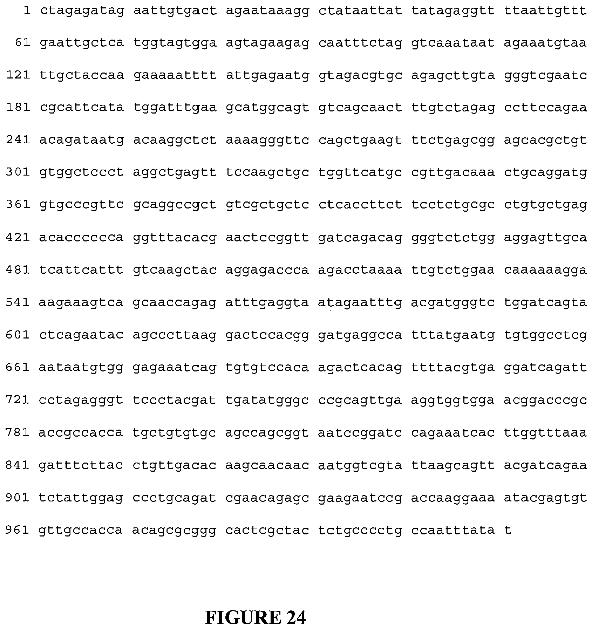

FIG. 24 shows an exemplary nucleic acid sequence encoding Mus musculus mRNA for MPTPdelta.

FIG. 25 shows an exemplary nucleic acid sequence encoding Homo sapiens huntingtin (HTT).

FIG. 26 shows an exemplary nucleic acid sequence encoding N-Methyl-D-Aspartate Receptor (NMDAR).

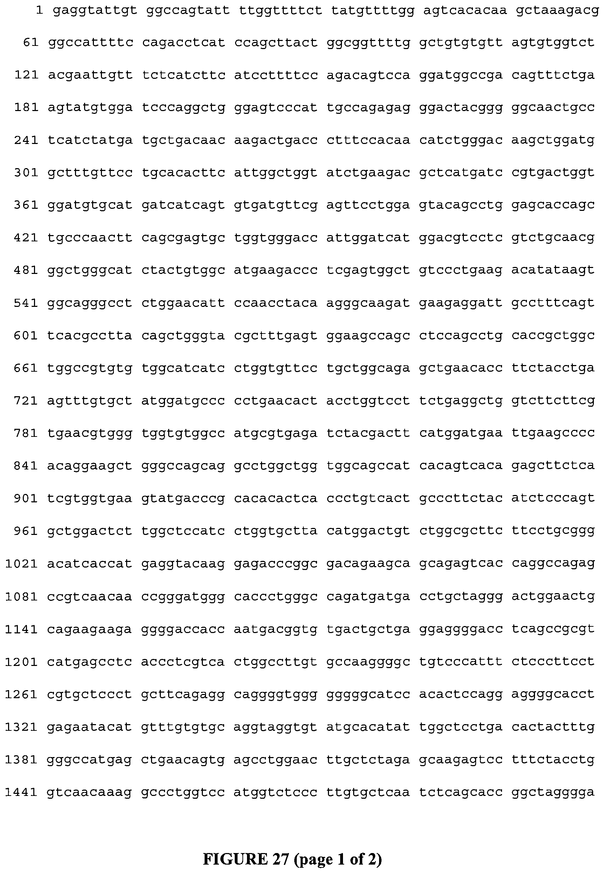

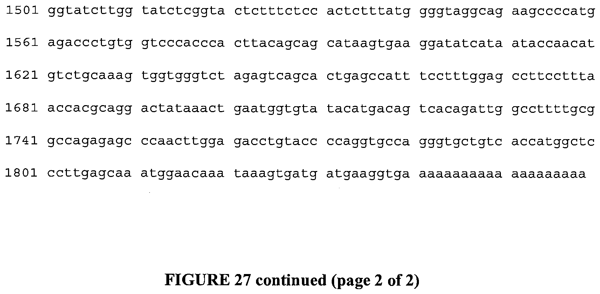

FIG. 27 shows an exemplary nucleic acid sequence encoding Phosphatidylserine Synthase (PTDS).

## DETAILED DESCRIPTION OF THE INVENTION

The present description discloses the use of camelid and/or shark single-domain heavy-chain only antibodies and their analogs for ultrasensitive detection of antigens. The method is useful for diagnosing human diseases at an early stage of their manifestation, when the concentration of antigens associated with such diseases is very low for example, 200 or fewer molecules in 0.1 ml of the bodily fluid. Also disclosed are methods for the development of nano-biomedical technology platforms utilizing camelid and/or shark heavy-chain only antibodies and their analogs for in-vitro diagnosis of human and animal diseases with such antibodies.

### Camelid **and Shark Antibodies**

The hetero-tetrameric structure of antibodies exists in humans and most animals but the single-domain heavy-chain only dimeric structure, without the light-chains, is considered characteristic of camelids and sharks [ Nature Biotechnology, 23, 1126 (2005)]. These antibodies are relatively simple molecules but with unique characteristics. Their

size is about 2/3rd the size of traditional antibodies, hence a lower molecular weight (about 90 KDa), with similar antigen binding affinity, but with water solubility 100 to 1000 folds higher than the conventional antibodies. Because of the lower molecular weight, the authors of this application call these antibodies as "Single-domain Mini-antibodies" (sdMnAbs) or simply "Mini-Antibodies" (MnAbs).

Another characteristic of the single-domain antibodies derived from sharks and camelids is that they have very high thermal stability compared to the conventional mAbs. For example, camel antibodies can maintain their antigen binding ability even at 90°C [Biochim. Biophys. Acta., 141, 7 (1999)]. Furthermore, complementary determining region 3 (CDR3) of camel Vab region is longer, comprising of 16-21 amino acids, than the CDR3 of mouse VH region comprising only of 9 amino acids [Protein Engineering, 7, 1129 (1994)]. The larger length of CDR3 of camel Vab region is responsible for higher diversity of antibody repertoire of camel antibodies.

In addition to being devoid of light chains, the camel heavy-chain antibodies also lack the first domain of the constant region called CHI, though the shark antibodies do have CH1 domain and two additional constant domains CH4 and CH5 [ Nature Biotech. 23, 1126 (2005)]. Furthermore, the hinge regions of camel and shark antibodies have an amino acid sequence different from that of normal heterotetrameric conventional antibodies [(S. Muyldermans, Reviews in Mol. Biotech., 74, 277 (2001)]. Without the light chain, these antibodies bind to their antigens by the variable antigen-binding domain of the heavy-chain immunoglobulin, which is referred to as Vab by the authors of this application (VHH in the literature), to distinguish it from the variable domain VH of the conventional antibodies. The single -domain Vab is amazingly stable by itself without having to be attached to the parent antibody. This smallest intact and independently functional antigen-binding fragment Vab, with a molecular weight of ~12-15 KDa, is referred to as nano-antibody by the authors of this application. In the literature, it is known as nanobody [(S. Muyldermans, Reviews in Mol. Biotech., 74, 277 (2001)].

The genes encoding these full length single-domain heavy-chain antibodies and antibody-antigen binding fragment Vab (camel and shark) can be cloned in phage display vectors, and selection of antigen binders by panning and expression of selected VHH in bacteria offer a very good alternative procedure to produce these antibodies on a large scale. Also, only one domain has to be cloned and expressed to produce in vivo an intact, matured antigen-binding fragment.

There are structural differences between the variable regions of single domain antibodies and conventional antibodies. Conventional antibodies have three constant domains while camel has two and shark has five constant domains. The largest structural difference is, however, found between a VH (conventional antibodies) and Vab (heavy-chain only antibodies of camel and shark) (see below) at the hypervariable regions. Camelid Vab and shark V-NAR domains each display surface loops which are larger than for conventional murine and human IgGs, and are able to penetrate cavities in target antigens, such as enzyme active sites and canyons in viral and infectious disease biomarkers [PNAS USA., 101, 12444 (2004); Proteins, 55, 187 (2005)]. In human and mouse, the VH loops are folded in a limited number of canonical structures. In contrast, the antigen binding loop of Vab possess many deviations of these canonical structures that specifically bind into such active sites, therefore, represent powerful tool to modulate biological activities [( K. Decanniere et al., Structure, 7, 361 (2000)]. The high incidence of amino acid insertions or deletions, in or adjacent to first and second antigen-binding loops of Vab will undoubtedly diversify, even further, the possible antigen-binding loop conformations.

Though there are structural differences between camel and shark parent heavy-chain antibodies (FIG. 1), the antigen-antibody binding domains, Vab and V-NAR, respectively, have similar binding characteristics. The chemical and/or protease digestion of camel and shark antibodies results in Vab and V-NAR domains, with similar binding affinities to the target antigens [Nature Biotechnology, 23, 1126 (2005)].

Other structural differences are due to the hydrophilic amino acid residues which are scattered throughout the primary structure of Vab domain. These amino acid substitutions are, for example, Leu45 to R (arginine) or Leu45 to C (cysteine); Val37 to Y (Tyr); G44 to E( Glu), and W47(Trp) to G (Gly). Therefore, the solubility of Vab is much higher than the Fab fragment of conventional mouse and human antibodies.

Another characteristic feature of the structure of camelid Vab and shark V-NAR is that it often contains a cysteine residue in the CDR3 in addition to cysteines that normally exist at positions 22 and 92 of the variable region. The cysteine residues in CDR3 form S-S bonds with other cysteines in the vicinity of CDR1 or CDR2 [Protein Engineering, 7 1129 (1994)]. CDR1 and CDR2 are determined by the germline V gene. They play important roles together with CDR3 in antigenic binding [Nature Structural Biol, 9, 803 (1996); J. Mol. Biol, 311, 123 (2001)]. Like camel CDR3, shark also has elongated CDR3 regions comprising of 16-27 amino acids residues [Eur. J. Immunol, 35, 936 (2005)].

The germlines of dromedaries and llamas are classified according to the length of CDR2 and cysteine positions in the V region [Nguyen et al, EMBO J., 19, 921 (2000); Harmsen et al, Mol. Immun., 37, 579 (2000)].

Immunization of camels with enzymes generates heavy-chain antibodies (HCAb) significant proportions of which are known to act as competitive enzyme inhibitors that interact with the cavity of the active site [(M. Lauwereys et al., EMBO, J. 17, 3512 (1998)]. In contrast, the conventional antibodies that are competitive enzyme inhibitors cannot bind into large cavities on the antigen surface. Camel antibodies, therefore, recognize unique epitopes that are out of

reach for conventional antibodies.

Production of inhibitory recombinant Vab that bind specifically into cavities on the surface of variety of enzymes, namely, lysozyme, carbonic anhydrase, alfa-amylase, and beta- lactamase has been achieved [M. Lauwereys, et al., EMBO, J. 17, 3512 (1998)]. Hepatitis C protease inhibitor from the camelised human VH has been isolated against an 11 amino acid sequence of the viral protease [F. Martin et al, Prot. Eng., 10, 607 (1997)].

**Novel Analogs of Single-Domain Heavy-Chain Camelid and Shark Antibodies:**

Figures 2 and 3 outlines the analogs of new generation of camelid and shark antibodies and their analogs, respectively, which will assist us to develop ultrasensitive and ultraspecific diagnostic assays for the detection /identification of the pathological proteins and antigens.

**Production of Parent Single-Domain Heavy-Chain Mini-Antibodies (sdmnAbs) of Structure 1**

Host animals such as camel, llama, or alpaca will be immunized with the desired antigen(s), for example HER-2 protein, a biomarker for breast cancer or A$\beta$42 antigenic peptide for detecting amyloid plaque, following the procedures described by Murphy et al, in 1989 [Am. J. Vet. Res., 50, 1279 (1989)], but with slight modification. Immunization of camels will be done with 250 ug antigenic peptide per injection will be used, followed by 4 booster shots every two weeks 4 weeks after the initial injection. For baby sharks, 10 ug antigen/injection will be used. One antigen per animal for immunization will be used, though it may be feasible to immunize an animal simultaneously with multiple antigens to raise an immune response to each antigen separately, which can make the production cost effective [EMBO, J., 17, 3512 (1998); J. Immunol. Methods, 240,185 (2000)].

After immunization, 100 ml camel blood (or 5 ml from shark) will be withdrawn from the animal and the total IgGs will be precipitated out using ammonium sulfate precipitation procedure. Using size exclusion chromatography over Sephadex G 25, the conventional IgGs, MW 150 KDa, will be removed from the single-domain mini-IgG, I, with MW of 90 to 100 K Da. Affinity purification to obtain high affinity sdmnAb, I, will be done by magnetic beads coated with the antigenic peptide.

**Recombinant Production of Camelid and Shark Single-Domain Antibodies:**

Recombinant production of single-domain heavy-chain parent camelid antibodies 1, 4, 5, 6, 7, 8 ,9 (figure 2) and shark antibodies 2, 11, 12, 13, 14, and 15 will be done according to protocols and procedures described in pending Patent Application published as US 2010/0092470.

**Chemical Synthesis of Analogs of Single-Domain Camel Antibodies**

**Derivatization and Immobilization of Camelid Mini-antibody 1:**

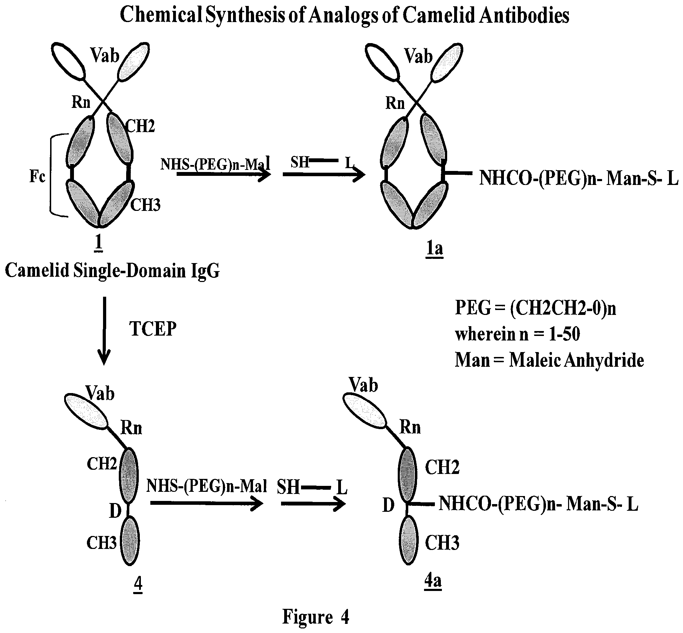

Schematics for derivatization and immobilization of mini-antibodies 1 are shown in figure 4. Mini-antibody 1(1 mg, 11 nmols) will be treated with commercial HS-$(PEG)_n$-Mal (11 ul of 10 mM stock= 110 nanomoles) wherein n= 1-50, in 50 mM MOPS/150 mM NaCl, pH 6.8, at RT for 1 hour to obtain the pegylated conjugate of figure 4 (structure not shown) which will be desalted by dialysis on C-3 Amicon filters to remove excess NHS-PEG-Mal reagent.

While the pegylation is underway, the ligand will be treated with 10X folds of Traut's Reagent in MOPS buffer, pH 6.8, containing 5% EDTA, at RT for 1 -2 hours. The thiolated ligand will then be purified either by dialysis (if ligands is chemical or biochemical entity) or by washing with MOPS buffer if ligand is a solid matrix.

The pegylated intermediate will be immediately conjugated with 10-20 folds excess of thiolated ligand: "SH- L" in MOPS buffer, pH 6.8 buffer containing 5 mM EDTA for 2-3 hours at room temperature (RT); where "L" may be enzyme (HRP, AP, Luciferase, galactosidase), protein, peptide, biotin, fluorophore, DNA, RNA, and solid matrix such as, magnetic beads, glass slides, gold nanoparticles, microchannels, microfluidic device.

When the ligand is a chemical or biochemical entity, for example, fluorophore, biotin, enzyme, protein, etc, the purification of the conjugate will be done by reverse-phase C8 HPLC.

Nucleic acid conjugates of mini- and nano-antibodies 1-15a will also be prepared using their pegylated conjugates followed by treatment with the thiolated-DNA/RNA molecules of interest (Fig. 4).

When the ligand is a solid matrix, such as, magnetic beads, glass slide, microchannels, etc., which we will use to immobilize the camelid antibodies, all we need to do is to wash the excess reagent with the appropriate buffer.

The activity and the amount of camelid antibody loaded onto the solid matrix will be determined by ELISA and commercially available protein assay kits.

**Single-domain Heavy-Chain Camelid Micro-Antibody 4 and its Analogs 4a:**

Micro-antibody, 4 , will be prepared by treating mini-antibody 1 (2 mg) with 1.0 ml of 10 mM TCEP (tris-carboxyethyl-phosphine) in 20 mM Phosphate/150 mM NaCl, pH7.4 at room temperature (RT) for one hour. The resulting micro-antibody 4 will be desalted on centricon-3 to remove the excess reagent and the buffer and stored at 4°C in 1X PBS.
Derivatization of 4 into 4a will be accomplished by the method described above for conversion of 1 into 1a.

**Single-Domain Heavy-Chain Camelid Sub-nano-antibody 5 and its Analogs of Structure 5a**

Micro-antibody 4 will be treated with trypsin or pepsin under controlled conditions at RT to cleave the CH2-CH3 domains from the antibody. After deactivation of the proteolytic enzyme with fetal calf serum, the subnano-antibody 5 will be isolated using size exclusion chromatography.
Derivatization of 5 into 5a will be accomplished by the method described above for conversion of 1 into 1a.

**Single-Domain Heavy-Chain Camelid Nano-antibody 6 and its Analogs of Structure 6a:**

Sub-nano-antibody 5 will be treated with pepsin at a low pH of 4.5 in 2M sodium acetate buffer under mild conditions for 1-8 hours to cleave the CH2-CH3 domains from the antibody. After deactivation of the proteolytic enzyme with fetal calf serum, the nano-antibody 6 will be isolated using size exclusion chromatography.
Derivatization of 6 into 6a will be accomplished by the method described above for conversion of 1 into 1a.

**Single-Domain Heavy-Chains Bivalent Nano-antibody 7 and its Analogs of Structure 7a:**

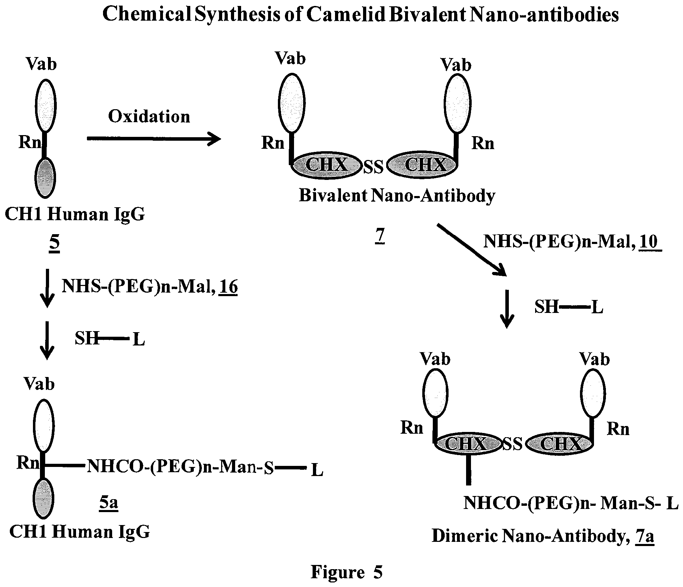

Schematics for the chemical synthesis of dimeric nano-antibodies and heir analogs are displayed in figure 5. Nano-antibody 5 will first be oxidized with 1% iodine in 20% tetrahydrofuran/70% water /10% pyridine for 5-10 minutes to transform into the dimeric nano-antibody 7. Treatment of 7 will be done with commercial NHS-$(PEG)_n$-Mal, wherein n= 1-50, in 50 mM MOPS/150 mM NaCl, pH 6.8, at RT for 1 hour to obtain the pegylated intermediate with maleimido group (structure not shown in figure 5) which, after purification by dialysis on C-3 Amicon filters, will be immediately conjugated with thiolated-ligand in MOP buffer containing 5% EDTA at pH6.8 for 2-3 hours at RT to obtain, after purification, 7a. The dimeric conjugate 7a will then be characterized by ELISA and Western blot assays.

**Trivalent and Tetravalent Camelid Nano-antibodies and Analogs:**

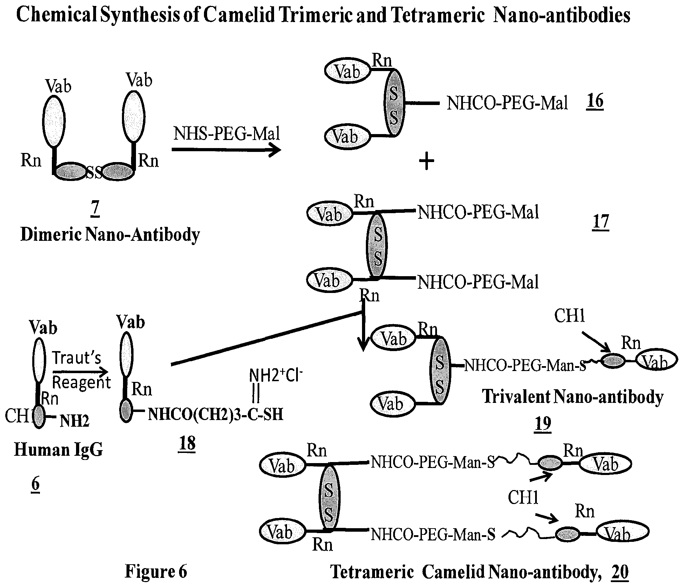

Schematics for the chemical synthesis of trivalent and tetravalent camelid nano-antibodies without the light-chains, and their analogs are shown in figure 6.

**Protocol for Developing Trivalent 8 and Tetravalent 9 Camelid Nano-antibodies:**

Bivalent nano-antibody, 7, prepared by oxidative dimerization or chemical ligation, will be conjugated with NHS-(PEG)3-Mal (10 folds excess) in MOPS buffer at pH 7.0 for 1 hour at RT. Chemical ligation of the resulting monomeric and dimeric pegylated products 16 and 17 with the thiolated nano-antibody 18 (figure 6) will be carried out by combining the two at pH 6.8 buffer containing 5 mM EDTA and allowing the reaction to occur at RT for at least 2 hours. The so formed trivalent, 19, and tetravalent nano-antibody 20 will be purified by size exclusion chromatography and stored at 4°C in PBS containing 0.02% NaN3.
The attachment of a ligand to 19 and 20 can be readily done by making use of the lysine(s) of the hinge region to conjugate with the NHS-L.
Pentavalent and higher analogs of nano-antibodies (Vab domains of camel antibodies) can be similarly prepared.

**Production of Single-Domain Heavy-Chain Shark IgNAR (Structure 2):**

Immunization of Sharks and Isolation of Shark IgNAR: Baby sharks will be immunized with the desired antigen(s), for example ALZAS, Tau, A|342 peptide which are the potential biomarkers for Alzheimer's disease, following the protocol described by Suran et al [J. Immunology, 99, 679 (1967)]. Briefly, the antigen (20ug per kg animal weight), dissolved in 20 mg/ml keyhole limpet hemocyanin (KLH) supplemented with 4 mg /ml complete Freund's adjuvant, will be injected intramuscularly. Four booster shots every two weeks four weeks after the initial injection will be administered.
After immunization, 3-5 ml shark blood will be withdrawn from the animal and the total IgGs will be precipitated out using 50% ammonium sulfate, followed by centrifugation at 2000 RPM for 10 minutes. After discarding supernatant, the precipitate will be dissolved in 20 mM PBS/150 mM NaCl containing 0.02% sodium azide and size fractionated on

Sephadex G200. The conventional IgGs, MW ~230 KDa, will be separated out from the shark IgNAR with MW of ~180 K Da. Alternatively, the conventional IgG fraction will first be depleted with protein G bound to magnetic beads, followed by isolation of V-NAR protein with magnetic beads coated with protein-A. Affinity purification to obtain high affinity shark Ig-NAR, 2, will be done by magnetic beads coated with antigenic peptide.

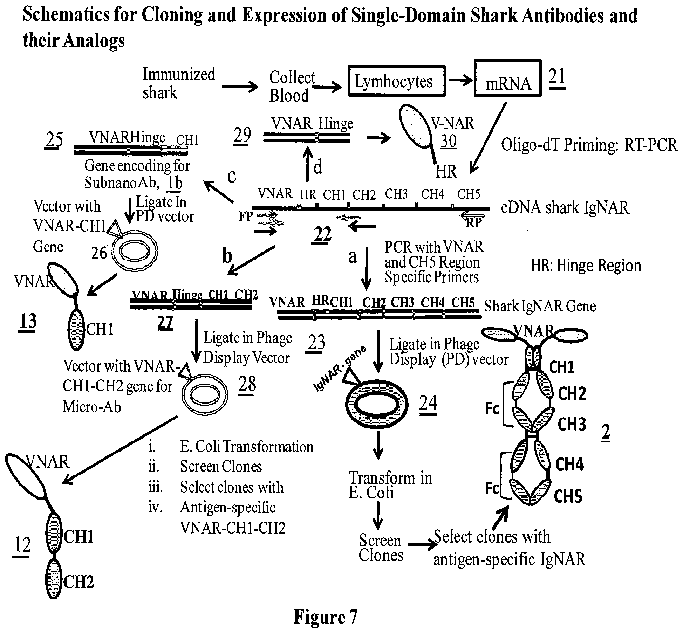

After determining the amino acid sequence of IgNAR, 2, nucleic acid sequence will be derived based from the amino acid sequence and recombinant DNA protocols will be established to produce the shark single-domain antibody 2 on a large scale. Schematics for cloning and expression of IgNAR are shown in figure 7.

**Isolation of RNA from Immunized Shark's Lymphocytes and Cloning:**

Isolation of total RNA, 21, from immunized sharks will be done from 3-5 ml of shark blood using commercially available RNA extraction kits such as Bio-Rad's AquaPure® RNA Isolation kit. Reverse transcription using oligo-dT primer will be achieved by PCR using high fidelity DNA polymerase to obtain the IgNAR cDNA, 22, shown in Fig. 7.

**Recombinant Production of Shark Heavy Chain Only Antibodies and Their Analogs**

An exemplary cloning strategy is shown in Fig. 7. Amplicons for IgNAR cDNA, 22, and its analogs will be performed using the following protocol:

IgNAR cDNA= 1.0 ug

Primers Mix =10 pmol (forward and reverse primers)

1 mM dTNPs = 10 ul

10mMMgCl2 - 5ul

10X PCR Buffer = 5 ul

Taq DNA Polymerase = 0.6 ul

Water to = 50 ul

After first denaturation round of 94°C for 10 minutes, 35 to 36 cycles of amplification will be performed under conditions as described below:

Denaturation: 20 seconds at 94°C

Annealing : 30 Seconds at 56°C

Extension: 50 seconds at 72°C Final

Extension: 10 min, 72°C

All or portions of IgNAR cDNA using different combinations of the following forward and reverse primers.

Forward primers
5'-gcatgggtag accaaacaccaag-3' (SEQ ID NO: 1)
5'-gcgtcctcagagagagtcccta-3' (SEQ ID NO: 2)
5'-gagacggacgaatcactgaccatc-3' (SEQ ID NO: 3)
5'- gggtagaccaaacaccaagaacagc-3' (SEQ ID NO: 4)
Reverse primers
5'-gttctagccaataggaacgtatag-3' (SEQ ID NO: 5)
5'-gtttgcacaagagagtagtctttac-3' (SEQ ID NO: 6)
5'-cctaaattgtcacagcgaatcatg-3' (SEQ ID NO: 7)
5'- gtgcagttccctagaagtcttg-3' (SEQ ID NO: 8)

After amplification, the amplicon will be purified on 1.5% agarose. The amplicon will be extracted from the gel

and its 5'-end kinased with gamma-ATP for blunt-end ligation with the phage-display vector using T4 DNA-ligase following standard ligation protocols.

Library or Plasmid Construction: Prior to cloning, the PCR amplicon encoding IgNAR gene will be digested with SfiI and NotI (Roche) following the cocktail:

V-NAR-CH1-CH2-CH3 -CH4-CH5 DNA= 5 ug

10X Restriction Buffer 5 ul

SfiI (10U/ul) 8ul

Water to 50 ul

Incubate 50°C for 8 hour

NotI 35 U

Reaction Buffer 4.5

Water to 60 ul

Incubate at 37°C for 4-5 hours.

Ethanol Precipitate at -70°C Pellet

Water to 50 ul

Agarose gel (1.5%) purification

Pure DNA Encoding Shark IgNAR Antibody

**Vector Ligation:**

IgNAR DNA = 200 ng

Vector DNA = 1000 ng

10X Ligase Buffer = 5 ul

T4 DNA Ligase = 10 U

Water to 50 ul

Incubate 15 hours at 4°C.

Ethanol Precipitate at -70°C

Suspend pellet in 10 ul.

**Electroporation:**

250 ul of E. Coli TGI cells will be made electrocompetent with BRL Cell-Porator® following vendor protocol.

Panning of Phage-Displayed IgNAR-Antibody 2 Library: Electroporated TGI cells will be transfected with the phagemid-IgNAR DNA insert. Approximately, 1010 cells will be grown to mid-logarithmic phase before injection with M13K07 helper phages. Virions will be prepared as described in the literature [Andris-Widhopf J., et al, J. Immunology Methods, 242, 159 (2002)] and used for panning at a titer of 1013/ml. Specific IgNAR antibody against the antigenic

peptide will be enriched by five consecutive rounds of panning using magnetic beads conjugated with antigenic peptide. Bound phage particles will be eluted with 100 mM TEA (pH 10.00), and immediately neutralized with 1M Tris.HCl (pH 7.2) and will be used to reinfect exponentially growing E. Coli TGI cells.

The enrichment of phage particles carrying antigen-specific IgNAR antibody will be assessed by ELISA before and after five rounds of panning. After the fifth panning, individual colonies will be picked up to analyze the presence of the virion binding by anti-M13-HRP conjugate.

**Expression and Purification of the Single -Domain IgNAR 2:**

The selected positive clones will be used to infect a new bacterial strain, HB 2151, a non-suppressor strain that recognizes the amber codon as a stop codon for soluble protein production. The HB2151 cell harboring the recombinant phagemids will be grown at 28°C in 250 ml 2X YT-ampicillin, 1% glucose in culture flasks until OD600 0.7. The cells will be washed and resuspended in 250 ml 2X YT-ampicillin, supplemented with ImM isopropyl beta D-thiogalactopyranoside (IPTG), and incubated over night at 22°C to induce protein expression.

Before adding IPTG to the cultures, a portion will be spotted on an LB/ampicillin plate for future analysis of the clones. The culture will be then be centrifuged at 4000 RPM for 15 minutes to pellet the bacterial cells. The culture supernatant will then be screened by ELISA for antigen-specific IgNAR protein 2.

**Chemical Synthesis of Single-Domain Heavy-Chain Shark Only Antibodies and Their Analogs 2a, 11,11a, 12,12a, 13,13a,14,14a, 15,15a:**

**Derivatization of Shark IgNAR 2 to Obtain Analogs of Structure 2a:**

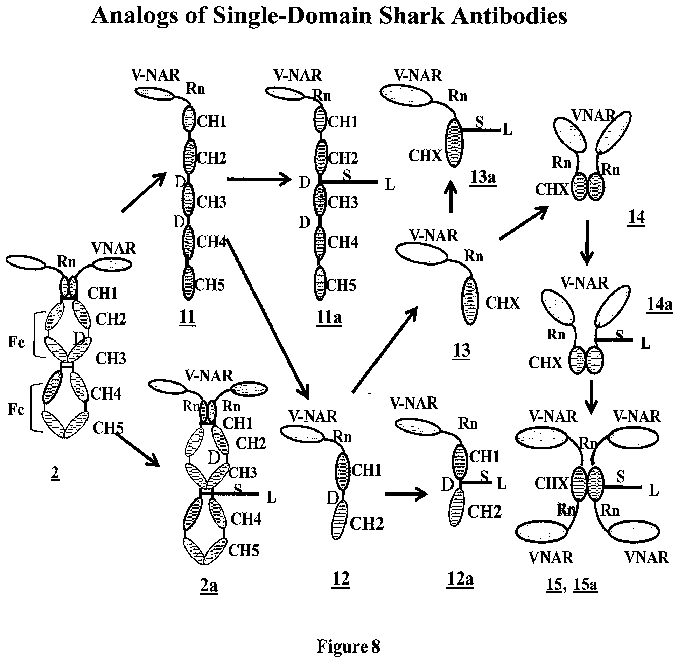

Schematics for derivatization of shark IgNAR 2 are shown in figure 8. Shark antibody 2 (1 mg, 6 nmols) will be treated with commercial NHS-$(PEG)_n$-Mal (6 ul of 10 mM stock= 60 nanomoles) wherein n= 1-50, in 50 mM MOPS/150 mM NaCl, pH 6.8, at RT for 1 hour to obtain the pegylated conjugate (structure not shown) which will be desalted by dialysis on C-3 Amicon filters to remove excess NHS-PEG-Mal reagent.

While the pegylation is underway, the ligand will be treated with 10X folds of Traut's Reagent in MOPS buffer, pH 6.8, containing 5% EDTA, at RT for 1 -2 hours. The thiolated ligand will then be purified either by dialysis (if ligands is a chemical or biochemical entity) or by washing with MOPS buffer if ligand is a solid matrix.

The pegylated intermediate will be immediately conjugated with 4-5 folds excess of thiolated ligand: "SH- L" in MOPS buffer, pH 6.8 buffer containing 5 mM EDTA for 2-3 hours at room temperature (RT); where "L" may be enzyme (HRP, AP, Luciferase, galactosidase), protein, peptide, biotin, fluorophore, DNA, RNA, and solid matrix such as, magnetic beads, glass slides, gold nanoparticles, microchannels, microfluidic device.

When the ligand is a chemical or biochemical entity, for example, fluorophore, biotin, enzyme, protein, etc, the purification of the conjugate 2a will be done by reverse-phase C8 HPLC.

Nucleic acid conjugates of shark IgNAR 2 and analogs 11,12,13,14, and 15 will also be prepared using their pegylated conjugates followed by treatment with the thiolated-DNA/RNA molecules of interest.

When the ligand is a solid matrix, such as, magnetic beads, glass slide, microchannels, etc., which we will use to immobilize the camelid antibodies, all we need to do is to wash the excess reagent with the appropriate buffer.

The activity and the amount of single-domain shark antibody loaded onto the solid matrix will be determined by ELISA and commercially available protein assay kits.

**Single-domain Heavy-Chain Shark Mini-Antibody 11 and its Analogs 11a:**

Mini-antibody, 11, will be prepared by treating the IgNAR 2 (2 mg) with 1.0 ml of 10 mM TCEP (tris-carboxyethylphosphine) in 20 mM Phosphate/150 mM NaCl, pH7.4 at room temperature (RT) for one hour. The resulting micro-antibody 11 will be desalted on centricon-3 to remove the excess reagent and the buffer and stored at 4°C in 1X PBS.

Derivatization of 11 into 11a will be accomplished by the method described above for conversion of 2 into 2a.

**Single-Domain Heavy-Chain Shark Micro-antibody 12 and its Analogs of Structure 12a:**

Mini-antibody 11 will be treated with trypsin or pepsin under controlled conditions at RT to cleave the CH3-CH4-CH5 domains from the antibody. After deactivation of the proteolytic enzyme with fetal calf serum, the shark micro-antibody 12 will be isolated using size exclusion chromatography.

Derivatization of 12 into 12a will be accomplished by the method described above for conversion of 2 into 2a.

**Single-Domain Heavy-Chain Shark Sub-nano-antibody 13 and its Analogs of Structure 13a:**

Micro-antibody 12 will be treated with trypsin or pepsin under controlled conditions at RT to cleave the CH2 domain from the antibody. After deactivation of the proteolytic enzyme with fetal calf serum, the shark sub-nano-antibody 13 will be isolated using size exclusion chromatography.

Derivatization of 13 into 13a will be accomplished by the method described above for conversion of 2 into 2a.

**Single-Domain Heavy-Chain Shark Dimeric -Nano-antibody 14 and its Analogs of Structure 14a:**

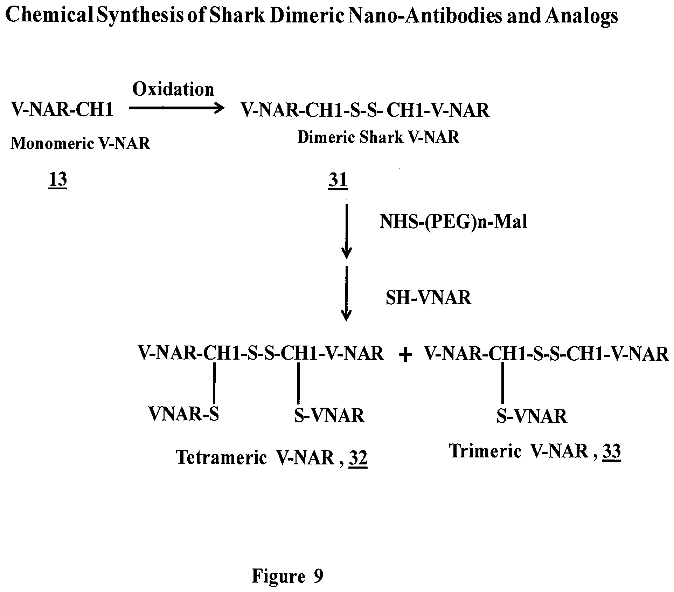

Dimeric V-NAR will be prepared by the oxidation of monomeric V-NAR, 13^ to obtain 14 as described in figure 9. These protocols are general and do not need detailed explanation.

Likewise, the transformation of 14 into its analogs of structure 14a will be accomplished as described above for the preparation of 2a from 2.

**Tetrameric and Trimeric V-NAR Nano-antibodies 31 and 32:**

Dimeric V-NAR nano-antibody 14 will be treated with 4-5 molar equivalent of NH5-PEG-Mal to obtain a mixture oftri- and tetra-pegylated derivatives of dimeric V-NAR nano-antibody (figure 9).

After purification, the tri- and tetrameric pegylated products will be treated with thiolated V-NAR to obtain, after purification by HPLC or just by dialysis, tetrameric and trimeric V-NAR nano-antibodies 31 and 32.

**Immobilization of Single-Domain Camelid Mini-Antibody and Shark IgNAR Antibody and Analogs onto Solid Matrixes:**

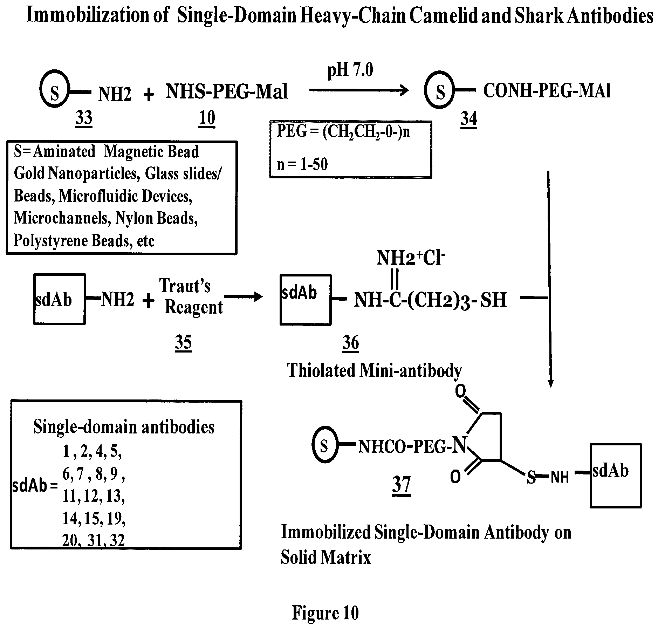

Immobilization of single-domain heavy-chain only shark and camelid native antibodies and their analogs onto solid matrixes, such as gold particles, magnetic particles, microchannels, glass particles and other solid surfaces will be accomplished using the steps outlined in Fig. 10. Aminated solid matrix 33 will first be derivatized with NHS-(PEG)n-Mal, 10, where n= 20 (20 fold molar excess) at pH 7.0 for 1 hour at RT. The solid matrix will then be washed thoroughly with the same buffer (50 mM MOPS/150 mM NaCl, pH 7.0). Any unconjugated amine groups will be masked with sulfoNHS-Acetate (Pierce) by incubated the solid matrix with 40 fold excess of the reagent at pH 7.0 for 60 minutes. After washing off the excess masking reagent, the pegylated matrix 34 will then be conjugated with thiolated single-domain heavy-chain antibody, 36, (10X excess) over the starting amine concentration. The conjugation will be performed at pH 6.5 for 2 hours at RT with gentle shaking of the matrix. The unused antibody will be recovered, and the matrix very well washed with IX PBS /0.5% Tween-20 to obtain complex 37 in which nano-antibody is covalently bound to a solid matrix. The activity of the bound heavy-chain antibody will be measured using ELISA.

**Biomarkers for various diseases**

Single-domain heavy-chain only shark and camelid native antibodies and their analogs can be used to detect the presence or absence of one or more antigens or can be used for diagnosis of one or more diseases. Single-domain heavy-chain only shark and camelid native antibodies and their analogs can bind specifically to the antigens or one or more biomarkers for various diseases. Exemplary sequences of various biomarkers or antigens are disclosed in application published as US 2010/0092470 filed September 21, 2009. Exemplary sequences of nucleic acids encoding additional biomarkers for Alzheimer's Disease are disclosed in Fig. 1727.

**Reference Example 1:**

**Capture and Detection of Pathogenic Antigens/Proteins Using Shark and Camel Single-Domain Antibodies (sdAbs)**

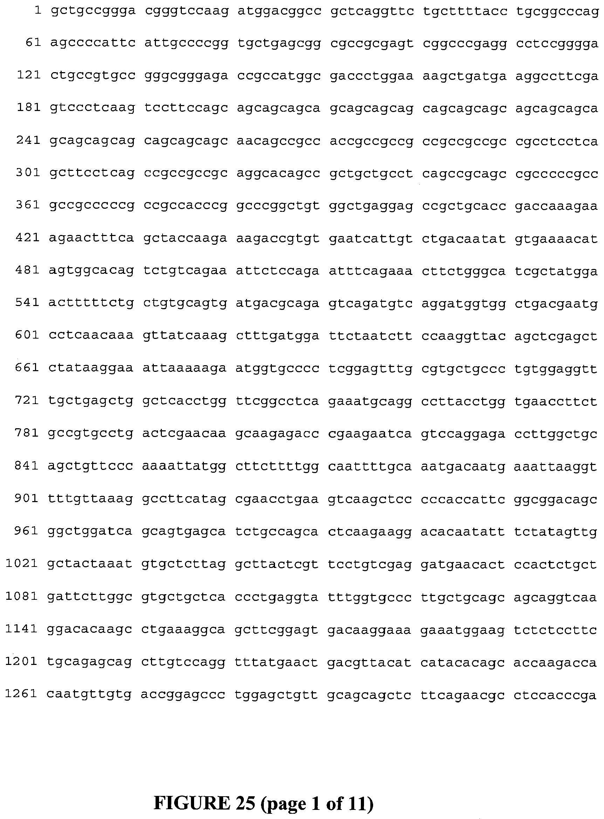

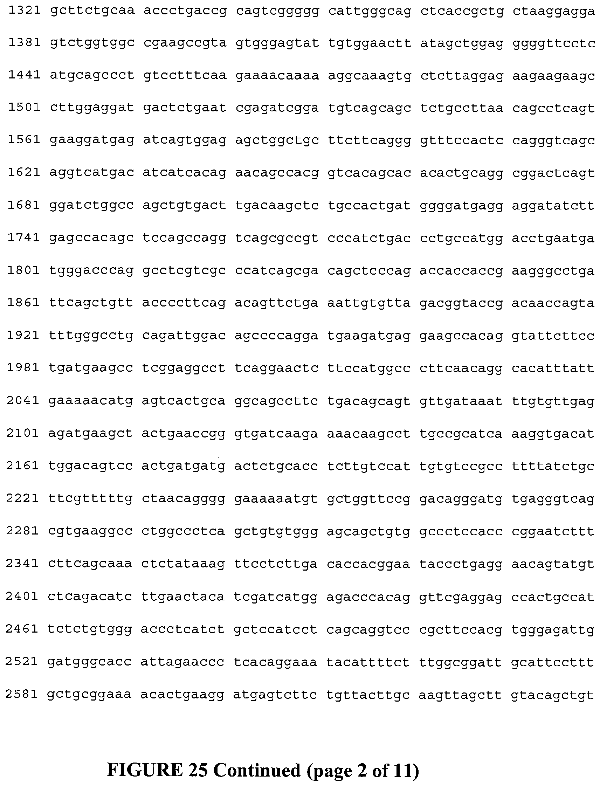

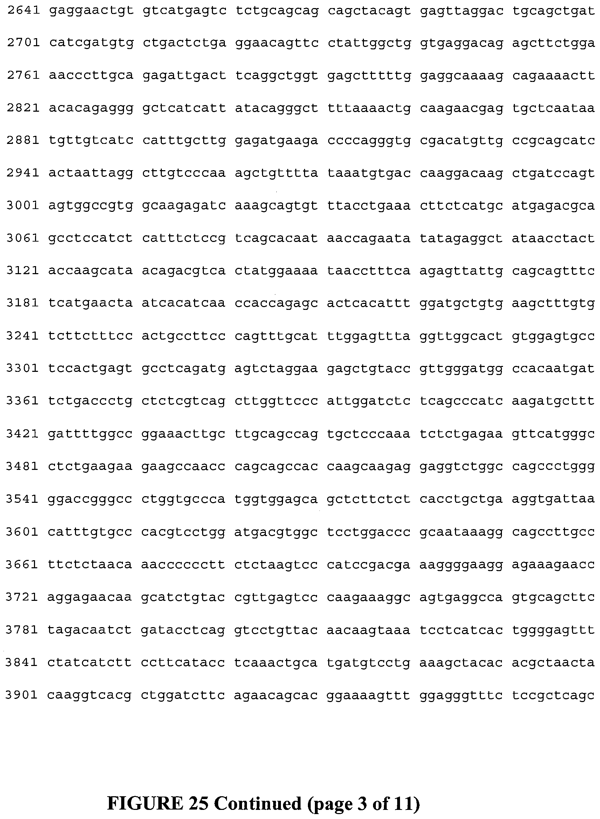

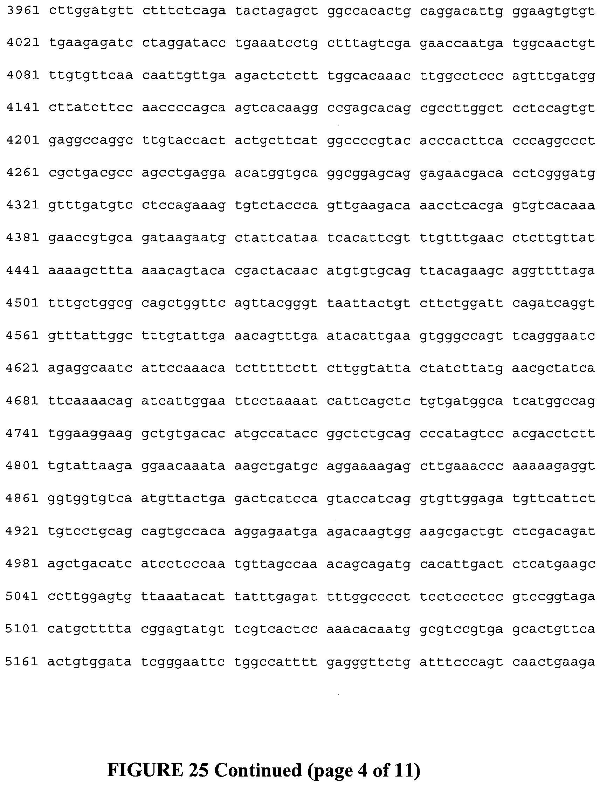

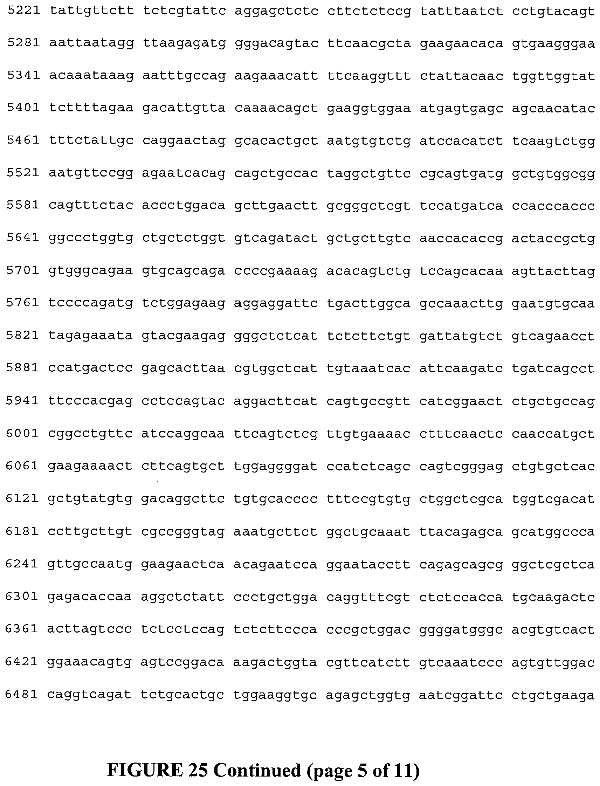

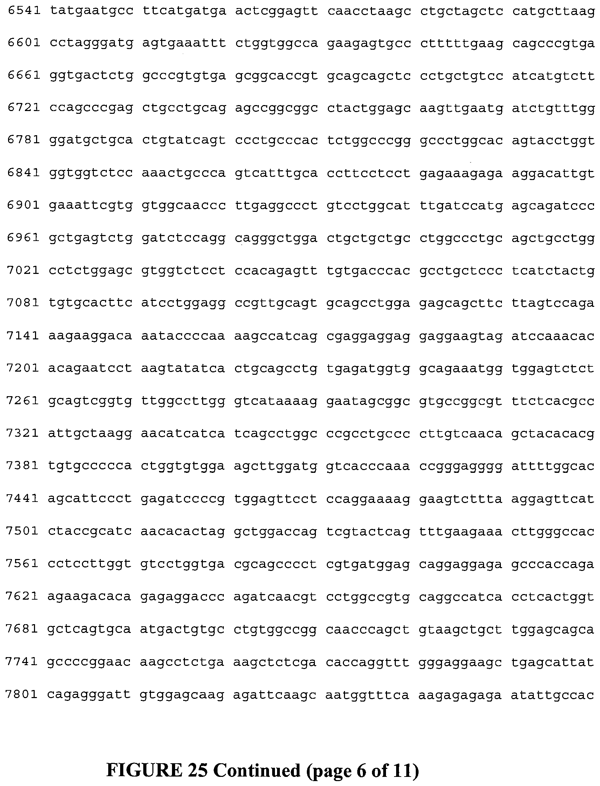

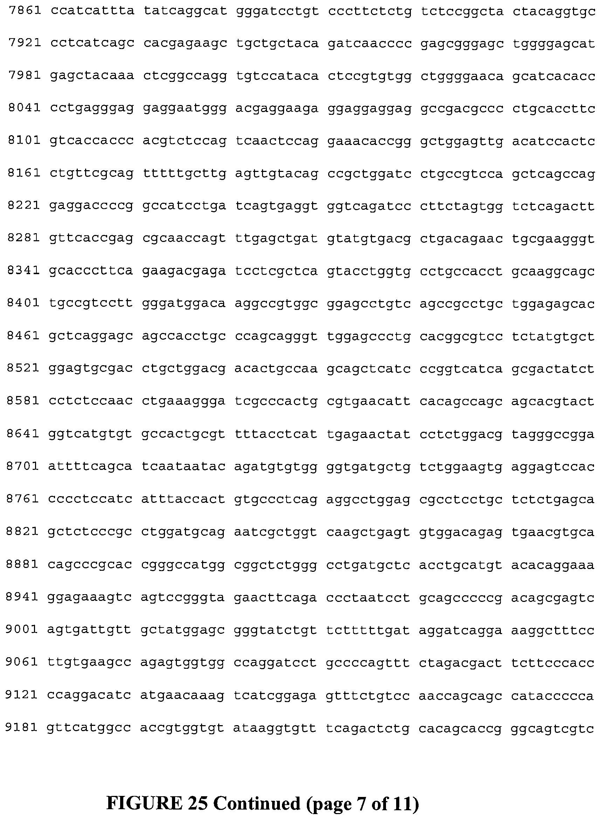

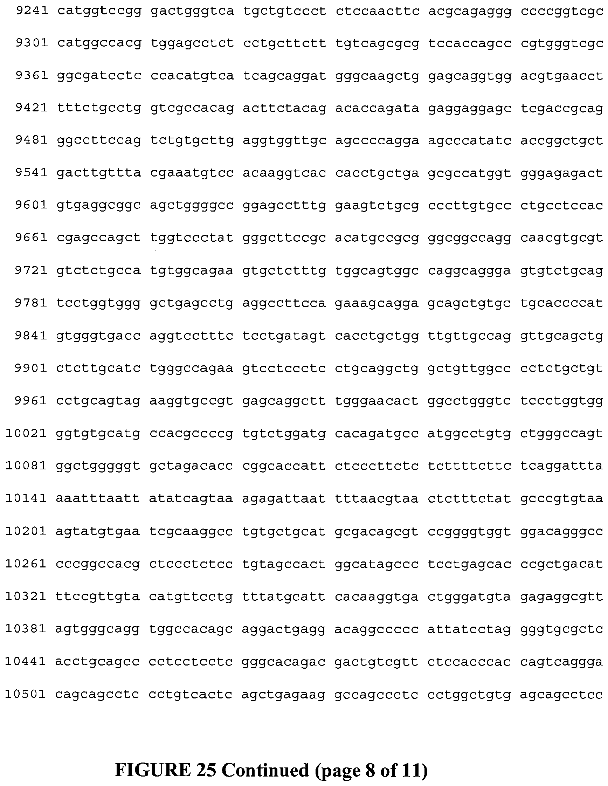

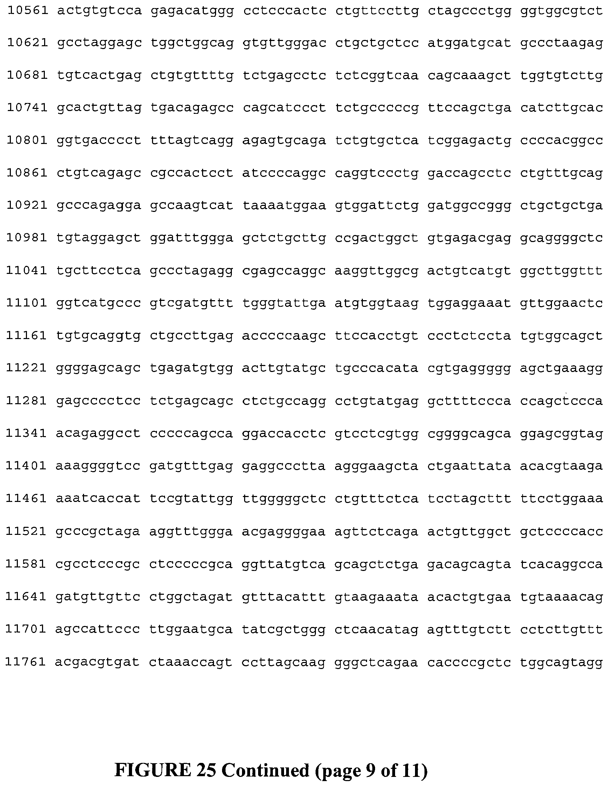

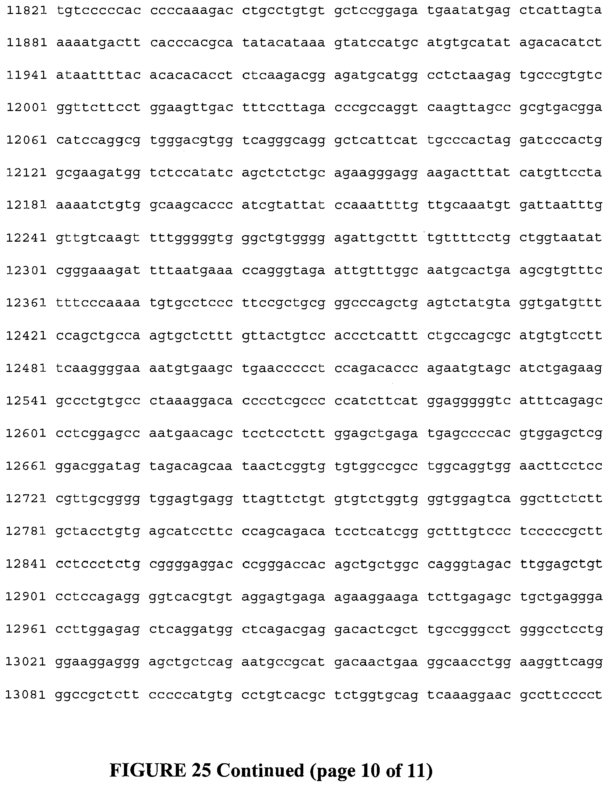

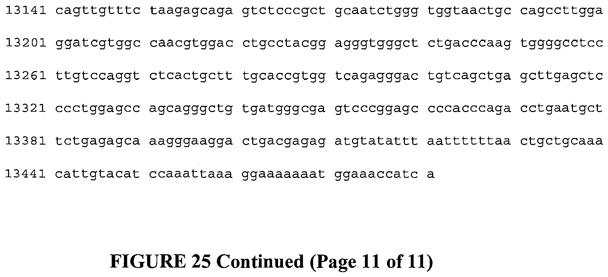

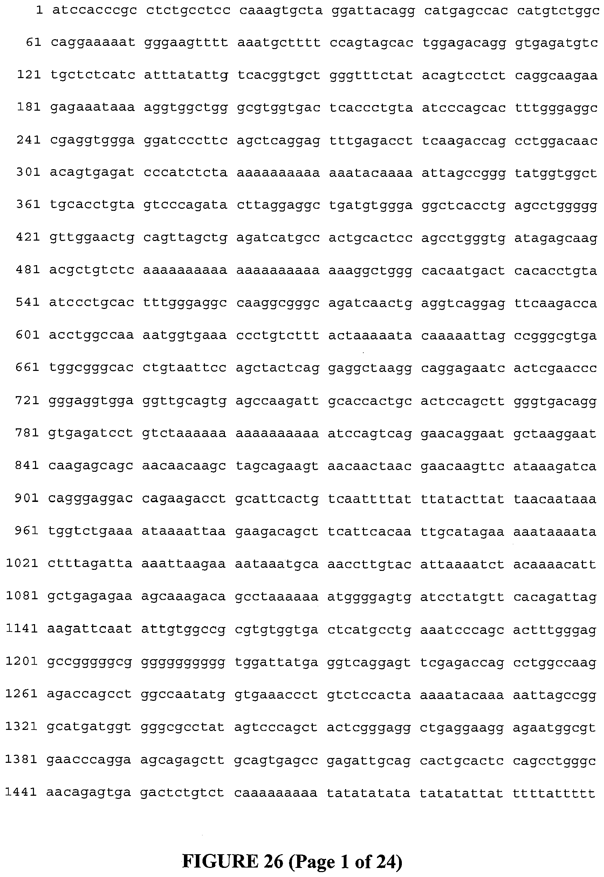

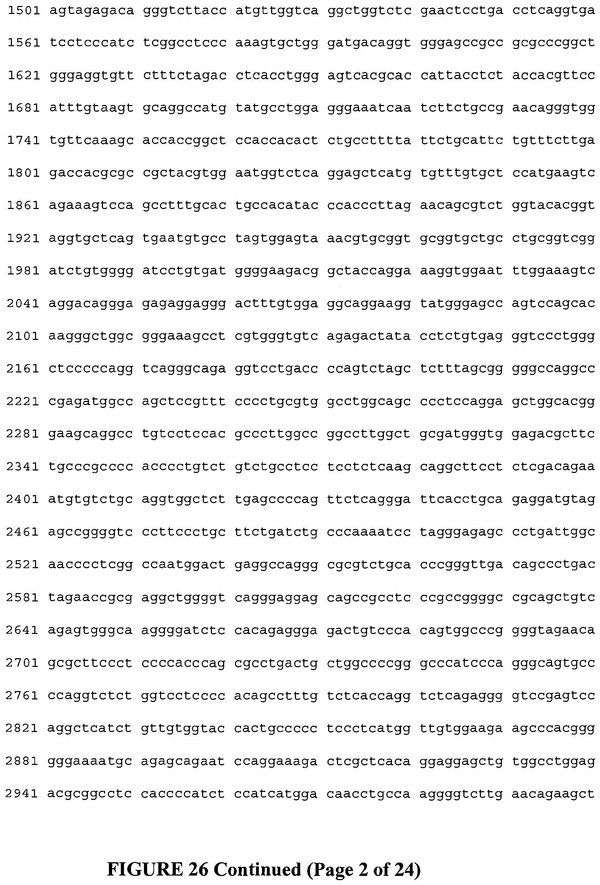

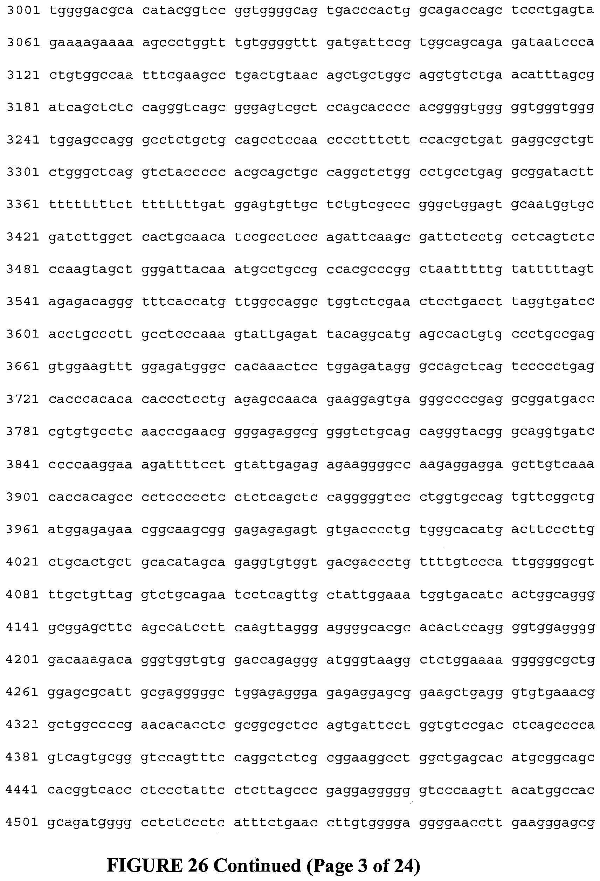

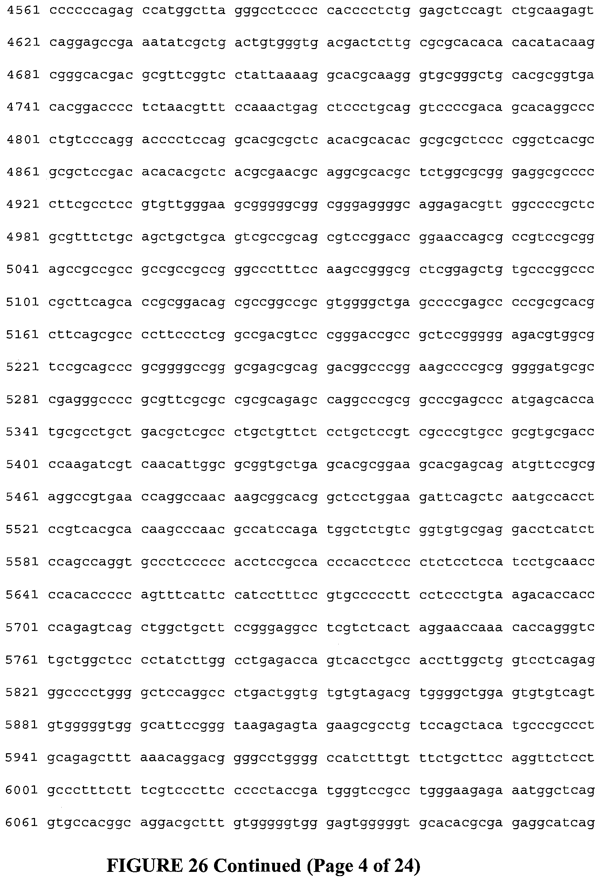

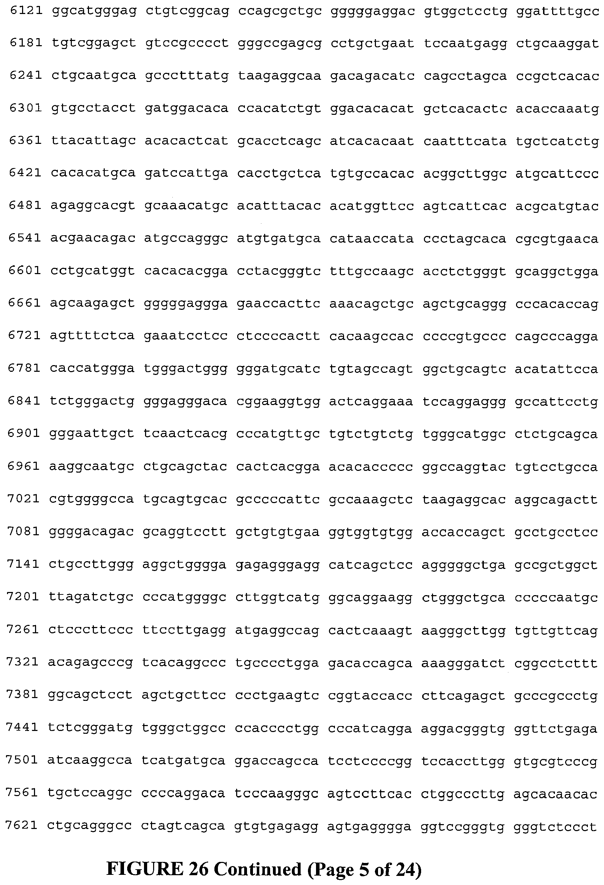

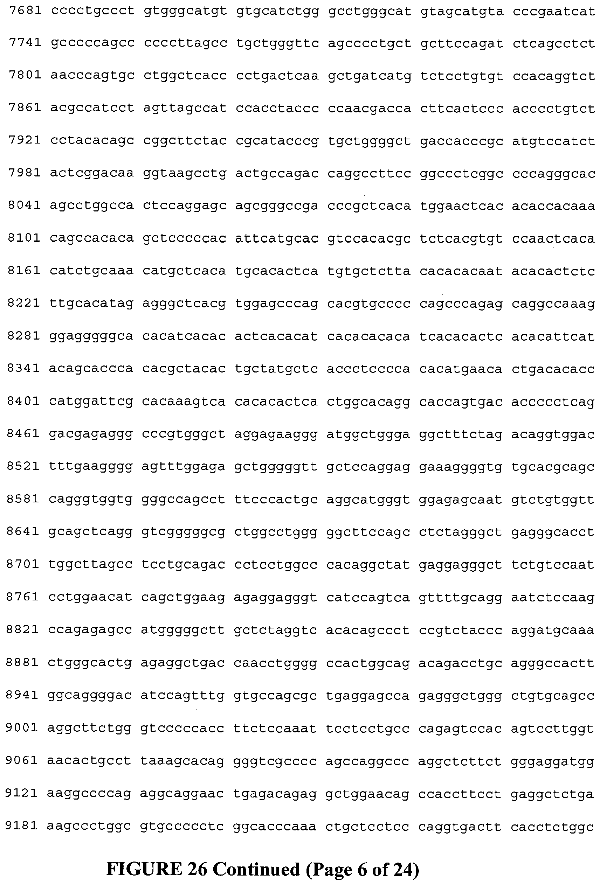

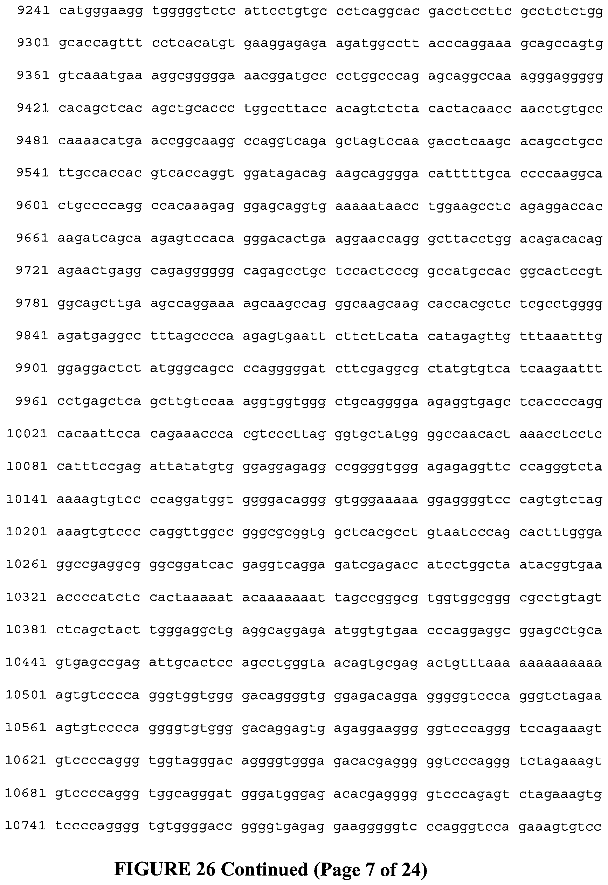

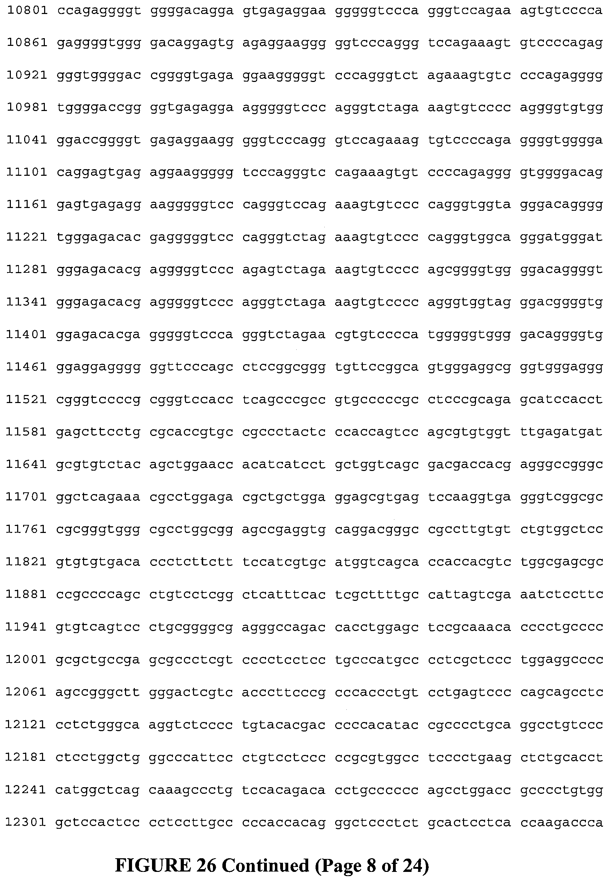

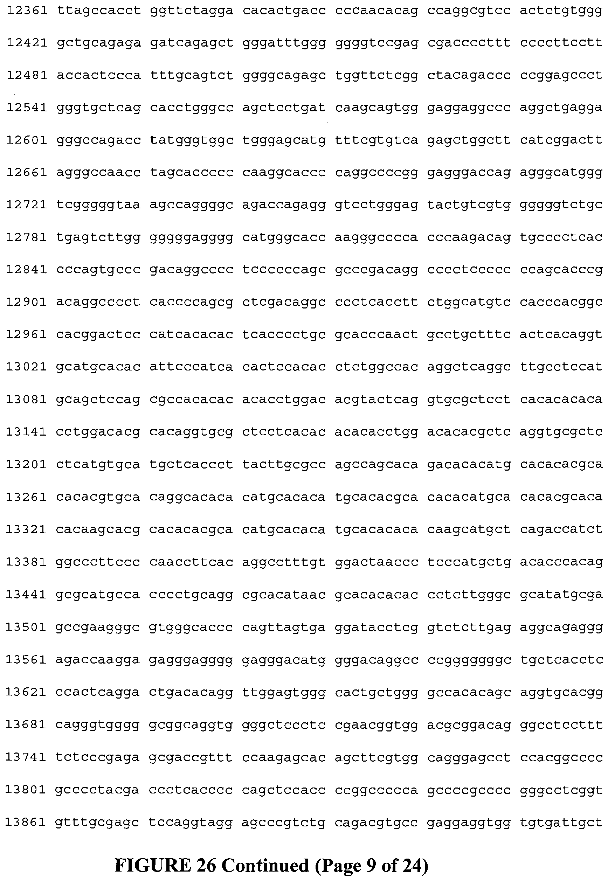

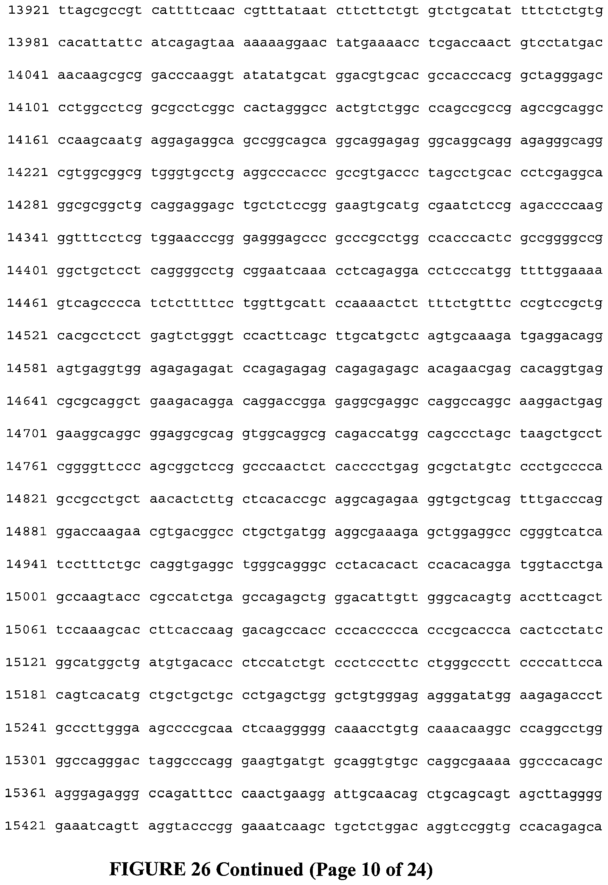

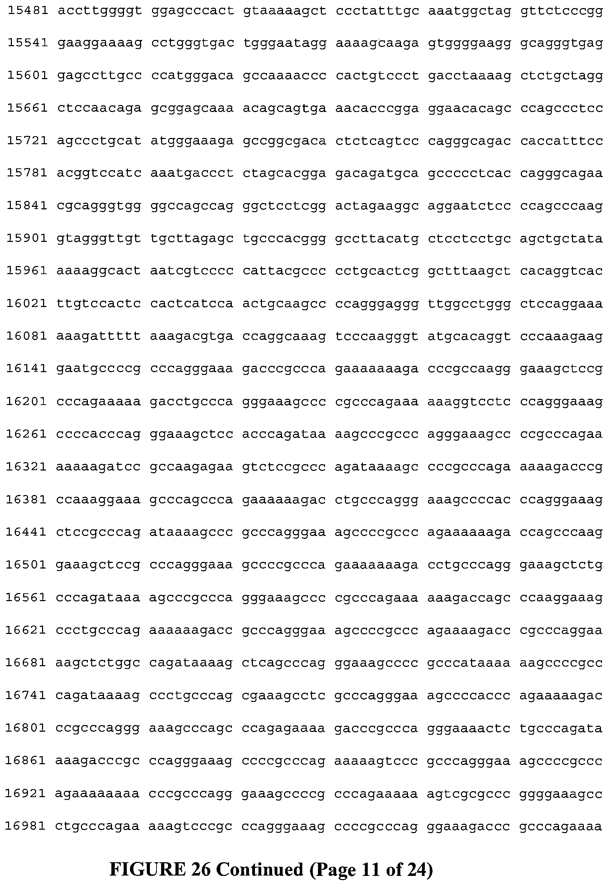

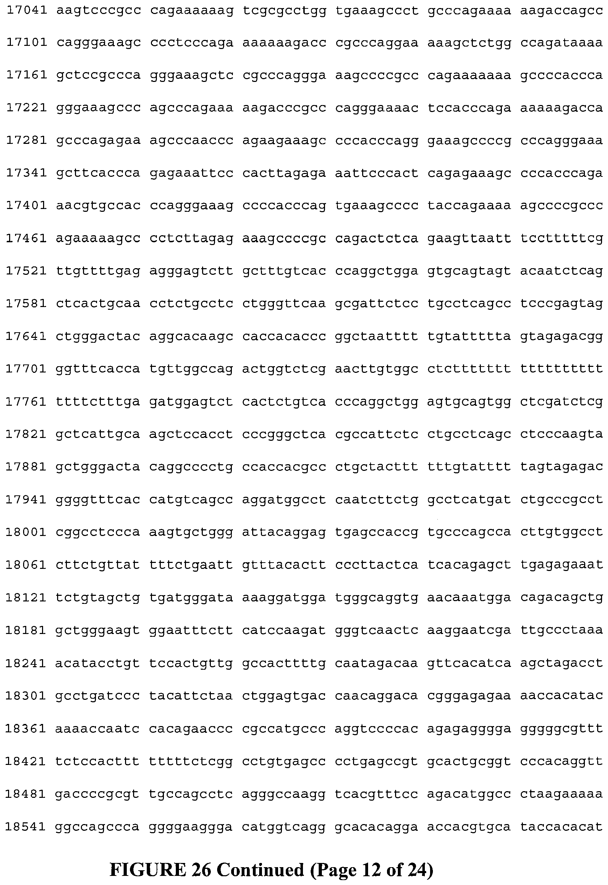

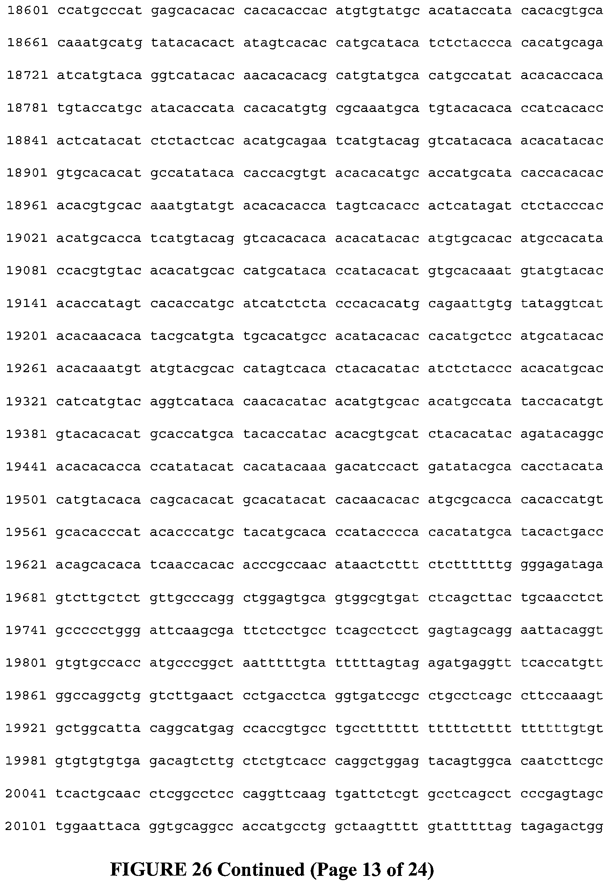

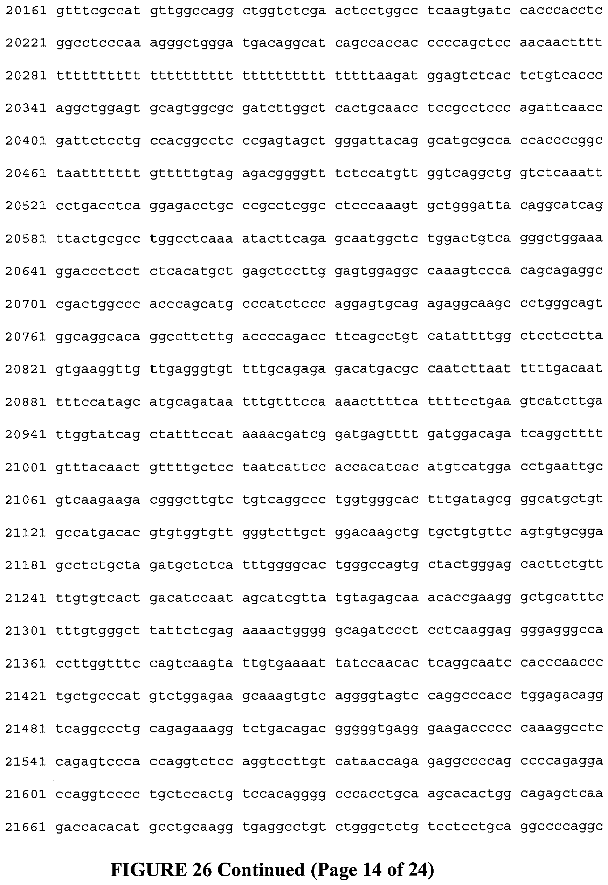

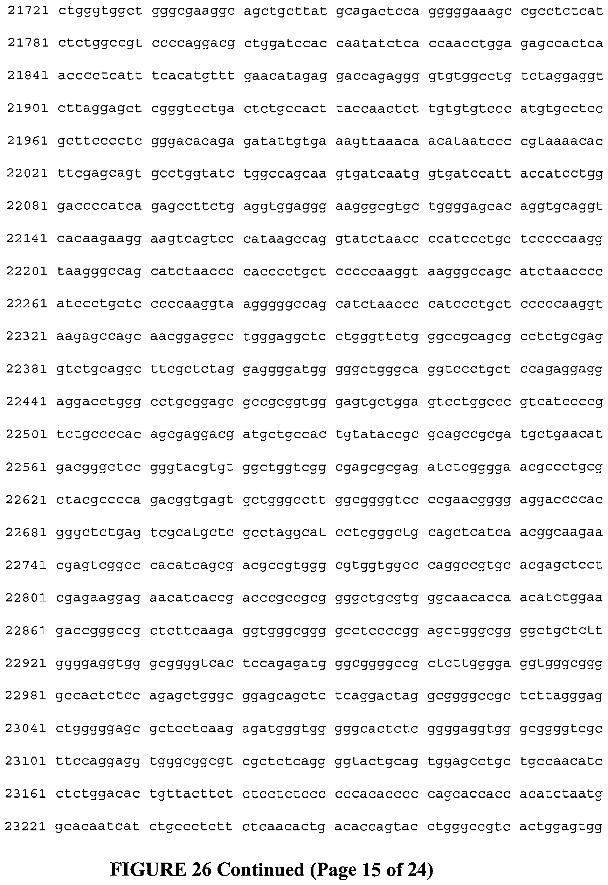

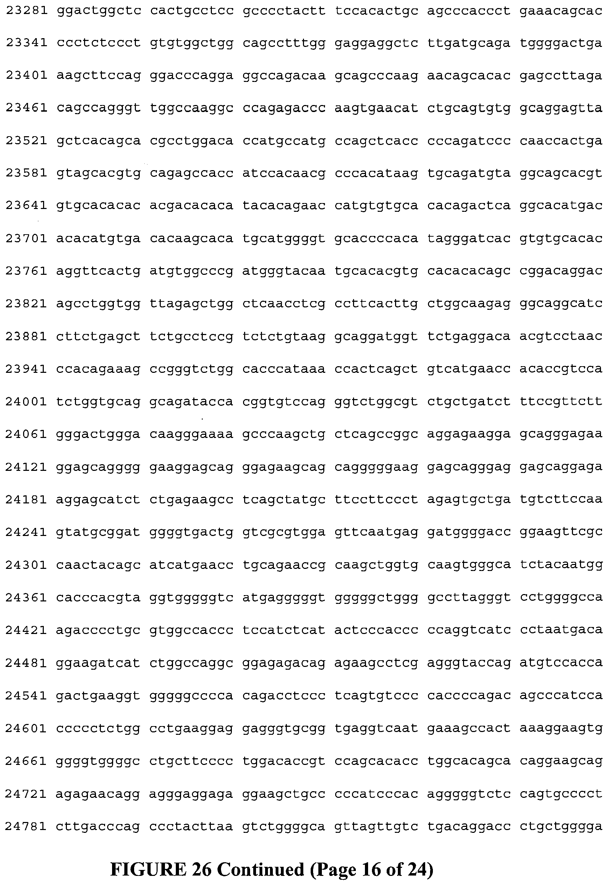

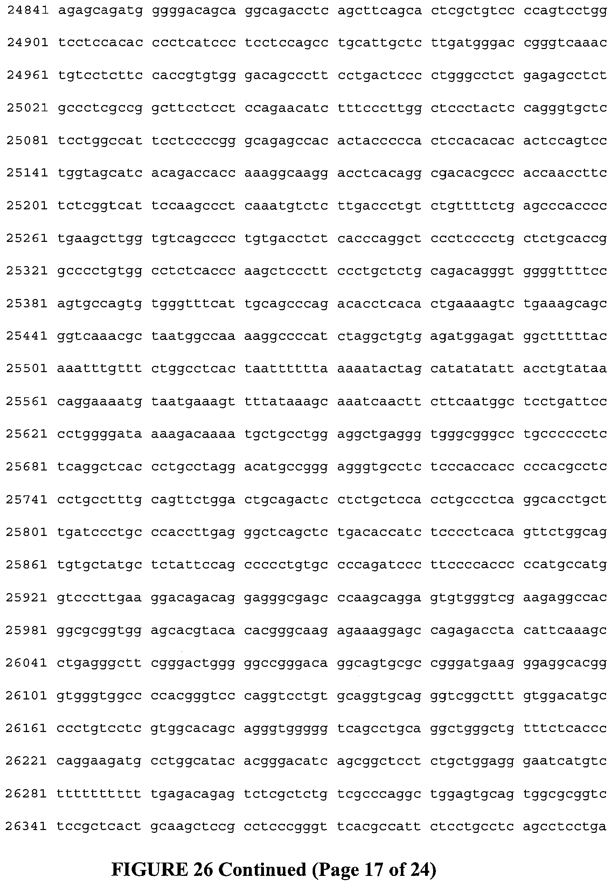

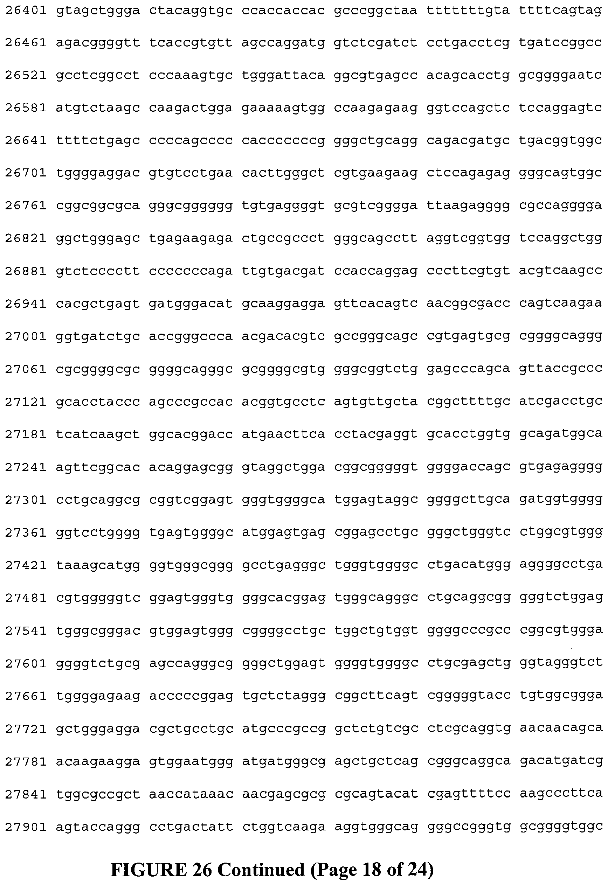

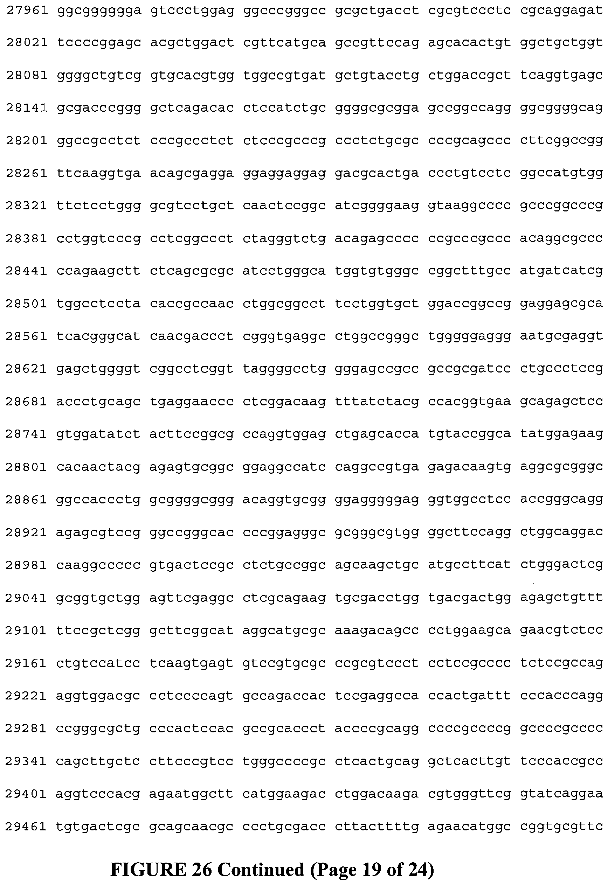

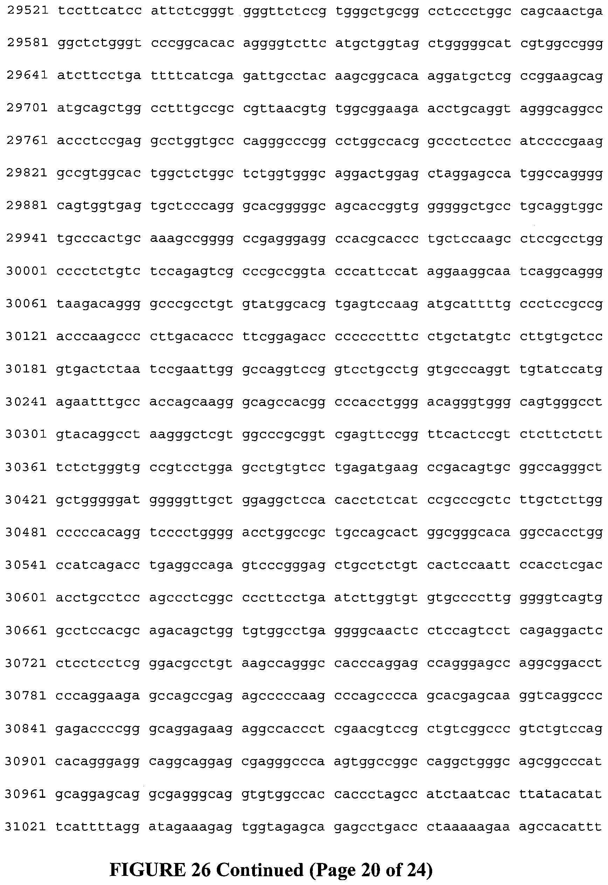

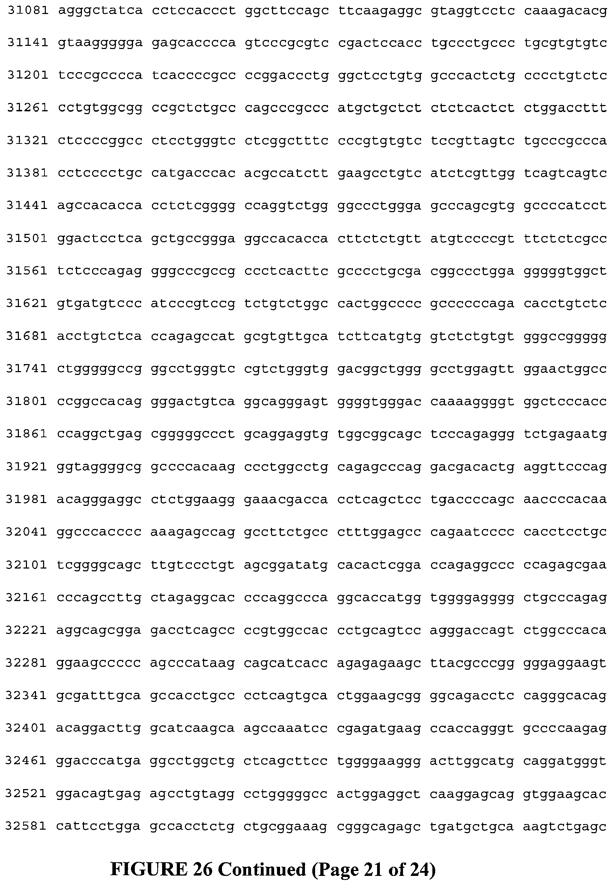

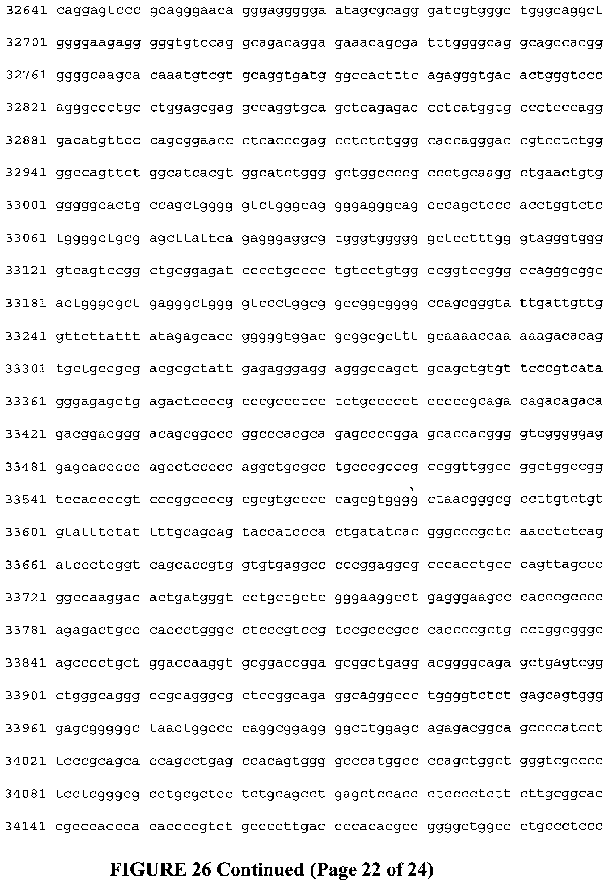

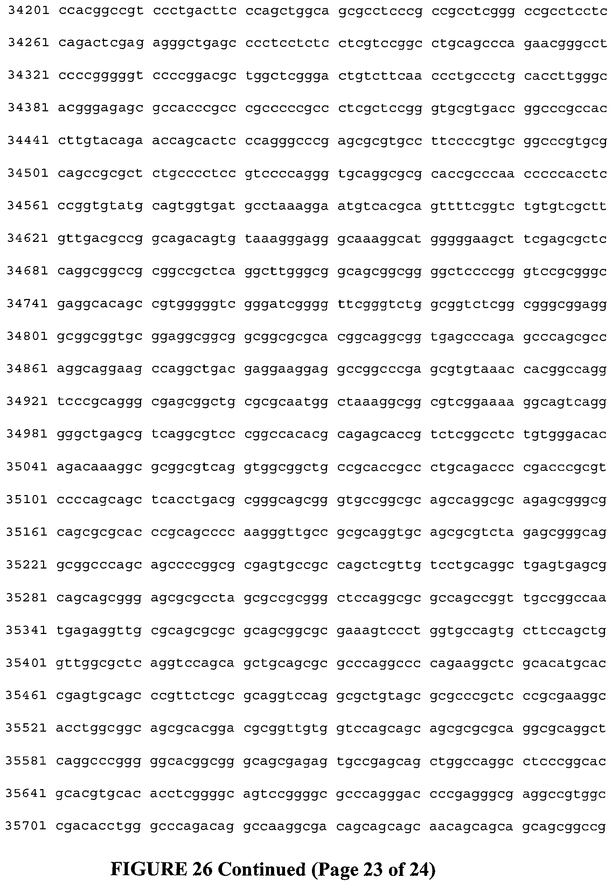

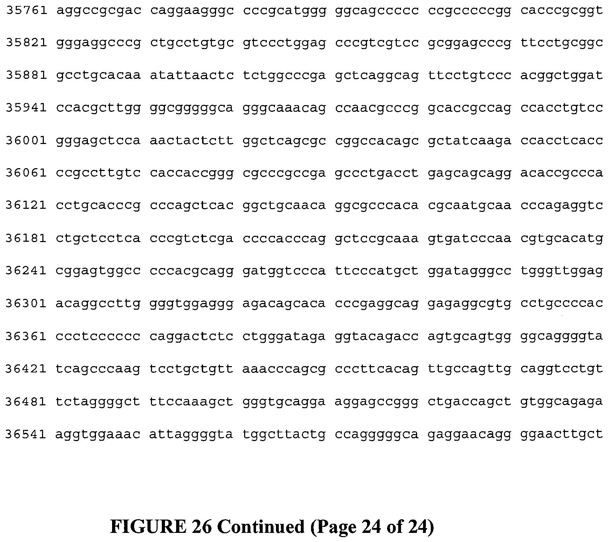

Serum from patient blood (10 ml), collected in EDTA tubes will be treated with shark and camelid heavy chain only antibodies and their analogs coated magnetic beads for 1-2 hours on a rotator with gentle rotation to bind the antigen. The beads will be separated using a magnetic rack and subsequently washed very well with PBS/1% BSA. The antigen-microantibody complex so formed will be treated with complex, detection antibody bound to an enzyme (AP, HRP, Luciferase, beta-galactosidase, gold particles) or DNA to sandwich the antigen between the shark and camelid heavy chain only antibodies and their analogs and the detection antibody forming the complex which will be detected either using an enzyme substrate or AgN03 if the detection antibody is conjugated to gold particles. Exemplary schematics of the process is shown in Fig. 25. Alternatively, the detection antibody could be conjugated to DNA molecules which

can then be amplified by PCR to obtain detection sensitivity equivalent to the detection of DNA by PCR as shown in Fig. 26.

**Reference Example 2:**

**Non-Invasive Detection of Prenatal Genetic Disorders from Captured Circulating Fetal Cells (CFCs) Using Heavy-Chain Antibodies**

Blood (10 ml) from a pregnant woman will be treated at RT for 1 hour with sdAb conjugated to magnetic beads, with gentle shaking. The beads will be allowed to settle down in a magnetic rack and then subsequently washed with a wash buffer containing 20 mM PO4 -2/ 150 mM NaCl/0.1%Triton X-100 (3 x 2 ml) to ensure complete removal of blood and serum. The beads will then be washed with 1X PBS to remove triton. The bound DNA will then be eluted by hot 10 mM Tris.HCl, pH7.0 or by protease digestion.

This fetal DNA will then be analyzed by real-time PCR using Y-chromosome primers to test the gender and by chromosome 21 primers to test for Down syndrome.

**Reference Example 3**

**In-Vitro Capture of Pathological Proteins with Single-Domain Camelid and/or Shark antibodies and Detection by Enzymatic Signal A**mplification:

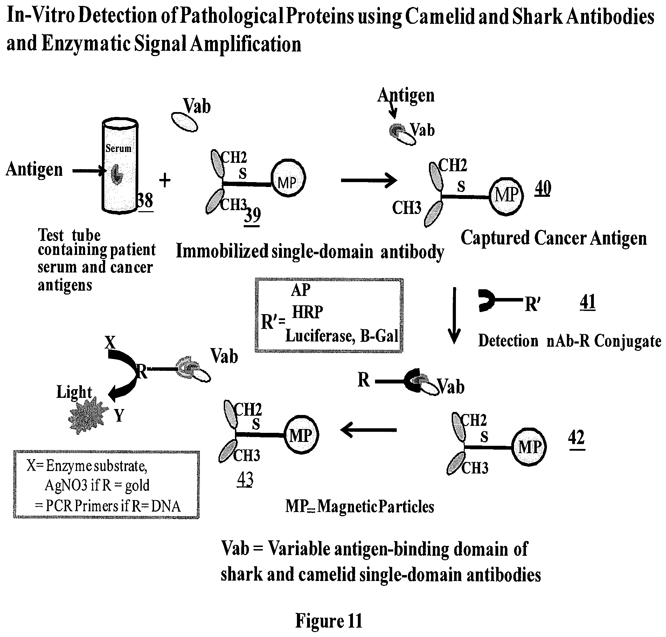

The high specificity of camelid and shark antibodies can be exploited to detect proteins at a much lower concentrations than what is currently possible. These antibodies are stable and functional at higher temperatures (80 to 90°C). Also, they are stable in the presence detergents and denaturing agents. This allows us to capture the pathological antigens under stringent conditions such as performing the capture reaction at elevated temperatures and using detergents (say for an example the use of up to 10% TritonX-100-), followed by high temperature stringent washings containing detergents to minimize non-specific capture. Such use of stringent conditions is only possible in immunoassays utilizing camelid and shark antibodies. When combined with enzymatic signal as shown in figure 11, the camelid and shark antibodies should be able to detect 0.1 to 1.0 attomoles of target molecules in 25 ul reaction volume, which itself is a much improvement over the existing proteomic detection technologies.

In the representative example shown in figure 11, the patient serum was incubated with magnetic beads coated with camel micro-antibody 39 (0.5 ml beads containing at least 1.0 ug camelid micro-antibody) with gentle shaking of the reaction contents at RT for 45 minutes. After 45 minutes, the beads were allowed to settle down and washed with 2XSSC buffer containing 1.0 % Tween-20. Detection of beads bound pathological antigen from 40 was accomplished by incubating the beads with a AP conjugate 41 of detection antibody for 1 hour at RT on a rocker. The beads were then thoroughly washed ( 5 x 2 ml) with preheated 2XSSC buffer (60 °C) containing 0.5% NP-40 to remove any non-specifically bound complex 41 (other commercially available detergents such as Triton X-100, Tween-20, SDS, LiDS, IGEPAL, Luviquat, DTPO, Antifoam 204, etc. can also be used.). The washings of the beads can also be done at temperature above 60 °C all the way up to 85 °C to remove any contaminants from the complex 42. The detection of complex 42 was then accomplished with Attophase (100 ul of 1.0 micromolar solution, 37 °C for 30 minutes), a fluorescence substrate for AP. The liberated green fluorescence was measured using a 96 microwell plate fluorimeter. 0.1 attomole of serum PSA antigen could be readily detected with 3:1 signal to background ratio.

**Reference Example 4**

**In-Vitro Capture of Pathogens by Single-Domain Shark IgNAR in Solution Phase and Detection by Enzymatic Signal Amplification:**

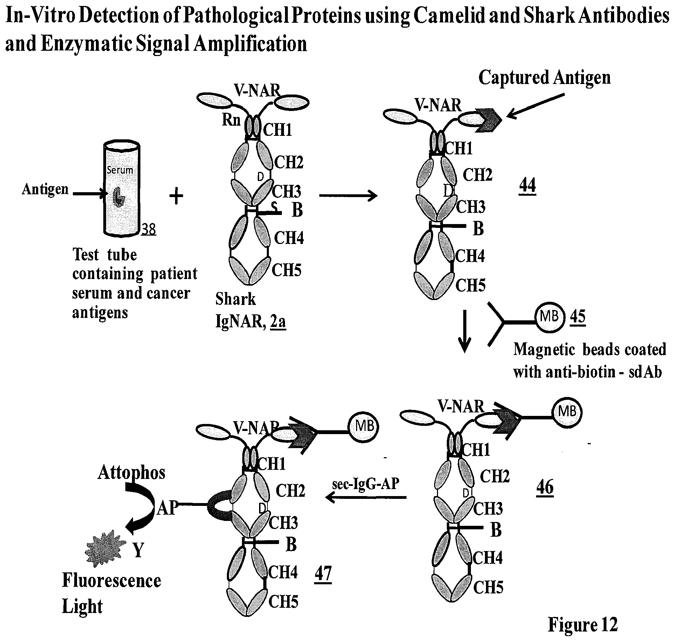

In this technology format, the biotinylated shark IgNAR, 2a (figure 12), can be added to the patient serum and allowed to react with the pathogen at 37°C for about one hour while the reactants are gently stirred or rotated on a orbital shaker. Anti-biotin camelid antibody (or shark antibody) bound to magnetic beads 45 can be added to reaction mixture to capture the so formed shark-IgNAR-Antigen complex 44 forming a complex of structure 46. The magnetic beads will be allowed to settle down in a magnetic rack and washed very well with a preheated (60 °C) wash buffer containing at least 1% NP-40. The complex 46 can then be detected by incubating with AP-IgG (sec) camelid complex using Attophos as a substrate as described above.

Other camelid and /or shark antibodies and their analogs can also be used the same way.

**Reference Example 5**

**Ultra-sensitive Signal Amplification Using Single-Domain Heavy-Chain Only Camelid and Shark Antibodies for In-Vitro Detection of Pathogens by Immuno-PCR:**

The two vital components of the method disclosed are: #1) Ultra-specific capture of pathological proteins by the new generation of single-domain camelid and shark antibodies lacking the light-chains (heavy-chain only antibodies), and #2) an ultrasensitive signal amplification technology to detect fewer than 200 molecules of protein biomarkers to diagnose diseases at a very early stage of the their manifestation. Therefore, in its preferred embodiment, this invention incorporates inherently highly specific heavy-chain antibodies for capturing the pathological proteins / antigens with high specificity with low to zero cross-reactivity, followed by detection of the captured antigen by enzymatic signal amplification, preferably immuno-PCR, to develop an ultra sensitive, highly specific and reliable diagnostic assay to detect fewer than 200 copies of the pathological proteins from biological samples.

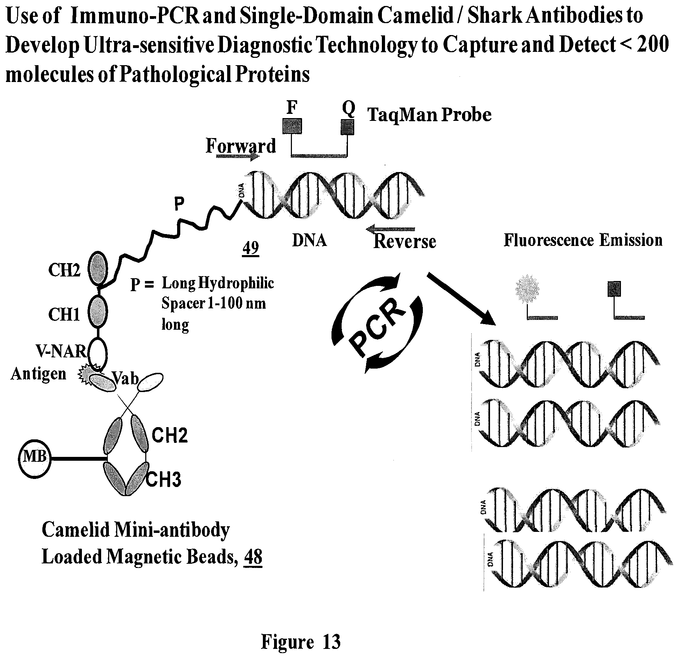

Figures 13 outlines the steps of the process involved. The protocol involves capturing the antigen from bodily fluid utilizing camelid and/or shark antibody coated magnetic beads by bringing in contact the said sample containing antigen with the beads. In the example shown in figure 13, camelid mini-antibody coated magnetic beads 48 are mixed with the serum for 1-2 hours with reactants constantly but slowly mixing all the time. The beads are then allowed to settle down in a magnetic rack and very well washed to ensure complete removal of the serum.

The detection of the captured antigen will be done by adding conjugate, 49, of secondary antibody that is conjugated to 100-120 bases long DNA via a hydrophilic linker that is at least 5 nanometer long to diminish and/or remove any stearic affects in the subsequent enzymatic amplification. The reaction between the captured antigen and the conjugate 49 will be allowed to take place for 2-3 hours after which the beads will be thoroughly washed to remove any unreacted conjugate 49.

The subsequent amplification of the attached DNA molecule by PCR using PCR kit from Applied Biosystems will allow for the indirect detection of the antigen with sensitivity almost equal to the sensitivity of detection of DNA by PCR.

There are many possible permutations and combinations of this technology. For instance, the antigen can be detected in solution phase by biotinylated or digoxigenin labeled camelid or shark antibody as described above following the steps of figure outlined in figure 12. The antigen-antibody complex so formed can be immobilized onto some solid matrix using camelid or shark anti-biotin or anti-digoxigenin antibody. Detection can be done by Immuno-PCR by forming a complex of the immobilized antigen-camelid-antibody with the secondary antibody bound to DNA which can be amplified by PCR.

Alternatively, the detection antibody in figure 13 can be conjugated with camelid or shark anti-biotin Mini- or nano-antibody. Biotinylated DNA can be used as a detection agent which will be amplified by PCR as outlined in figure 13.

**Reference Example 6**

**In-Vitro Capture and Detection of Rare Cells Using Shark and Camelid Heavy Chain Only Antibodies and Their Analogs**

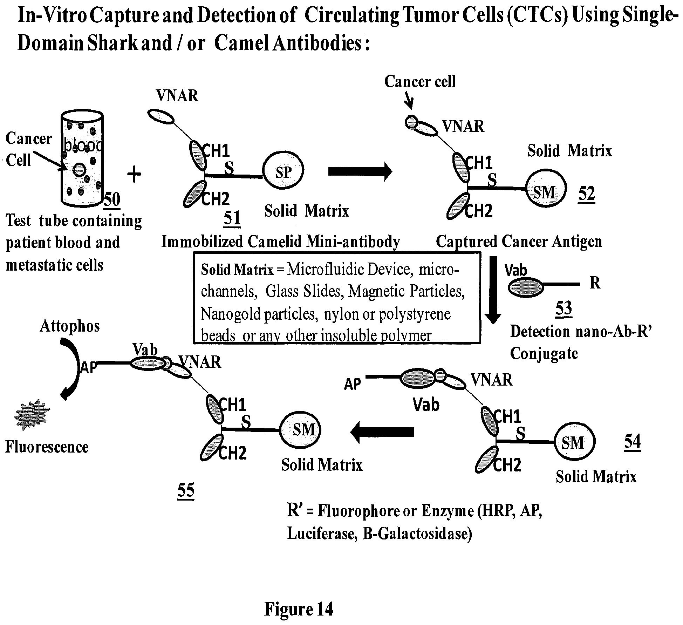

Fresh 5 ml patient blood will be diluted with 20 ml 1XPBS/1%BSA to 25 ml. To capture circulating tumor cells (CTCs), this sample will then be passed through a micro-fluidic device coated with an appropriate shark and camelid heavy chain only antibodies and their analogs, such as, anti-EpCAM-micro-antibody (camelid) 51 following flow rate recommended by the manufacturer of microfluidic device. To ensure that antibodies or its analogs do not lose any activity upon conjugation, all solid matrixes will first be coated with a hydrophilic polymer, such as, NHS-PEG-Mal (MW ~5000). The conjugation of the thiolated shark and camelid heavy chain only antibodies and their analogs with maleimido-group of the polymer can be achieved at pH 6.8 in a buffer containing 5% EDTA. Exemplary schematics of the process are shown in Fig. 14.

Alternatively, magnetic beads coated with EpCAM can be used. EpCAM (epithelial cell adhesion molecules) is frequently over expressed by carcinomas of lung, colorectal, breast, prostate, head and neck, liver, and is absent from hematological cells. The captured cells can be washed with 1% PBS (no BSA). The cell can be fixed with methanol, and then DAPI stained following CK8 or CK18 and CD45. Identification and enumeration will be done by fluorescence microscopy based upon the morphological characteristics, cell size, shape, and nuclear size. DAPI+, CK+, and CD45- cells will be classified as CTCs.

**Alternative Strategies to Capture Circulating Tumor Cells (CTCs):**

Patient’s blood (2-3 ml) (or urine 15-20 ml after centrifugation to pellet down the cells and suspending them in 1-2 ml HBSS media) will be incubated with an appropriate biotinylated mini-sdAb (1.5 ug/ml blood sample) at RT for

one hour. For example, to capture epithelial cancer cells, such as from breast, prostate, and ovarian cancers, biotinylated-anti-EpCAM-mini-antibody (camel antibody against EpCAM antigens) will be used to label the circulating cancer cells in the blood. After diluting with HBSS or RPMI-1640 media or 1XPBS/2.5%BSA to lower the sample viscosity, the diluted blood is then passed through a microfluidic device coated with antibiotin-mini-camelid or shark antibody at a flow rate allowing maximum cell capture. The captured CTCs can then be fixed by fixing with methanol, followed by fixing with 1% PFA using any standard cell fixing procedures. Enumeration will then be done by DAPI staining followed by immunohistochemical staining with commonly used mouse mHCAb such as CK-7 but more preferably mini-CK-7 for higher specificity. CTCs have to be CD45 negative.

Alternatively, most of the RBCs from the blood sample can be first lysed using ammonium chloride solution (155 mM NH4C1 / 10 mM NaHC03). After pelleting, the washed cells will be suspended in HBSS media (1-2 ml) and passed through the microfluidic device coated with heavy-chain antibody specific for the cell type one needs to capture and analyze.

Alternatively, the diluted blood sample after incubation with the biotinylated-antiEpCAM-mini-antibody or micro-antibody will be treated with the anti-biotin-mini-camelid antibody coated magnetic particles (Miltenyl) for 30 minutes while the sample is being gently rotated on a rotating wheel. After pulling down the magnetic particles with a magnet, the CTCs bound to the particles will be washed with PBS/1% BSA. The CTCs can then be enumerated by spreading them in a unilayer on a glass slide, drying them for one to two hours, followed by fixing with methanol, 1% PFA and staining the CTCs with CK-7.

Furthermore, these captured CTCs can be analyzed for the gene expression. For example, in case of prostate cancer patient, one can look for TMPRSS2-ERG translocation using PCR primers. TMPRSS2-ERG transcript is present in about 50% of the prostate cancer patients. Similarly, one can look for HER-2 expression in case of breast cancer.

**Reference Example 7**

**Capture and Analysis of Fetal Cells**:

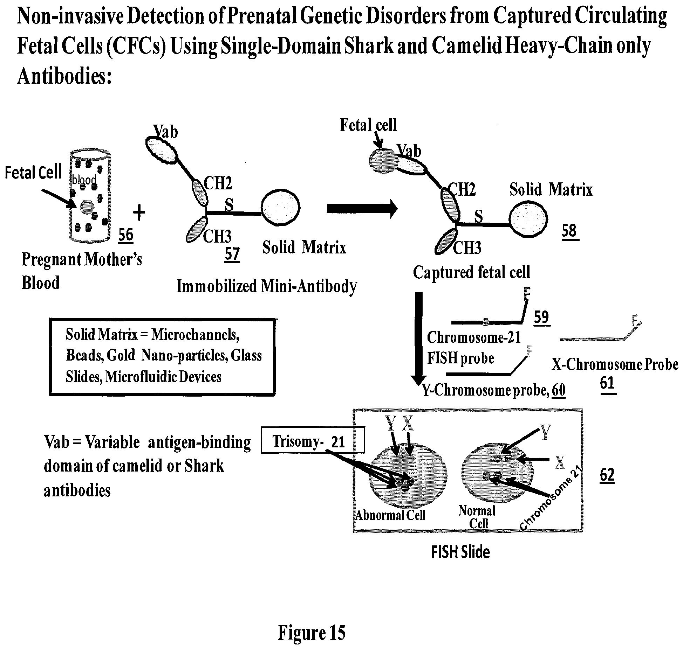

To capture fetal cells from the blood of pregnant mothers, 5 ml blood from pregnant mothers can be diluted to 15 ml with HAM-F 12 media containing 1% BSA and passed through the microfluidic device coated with the camelid and/or shark antibodies against the fetal cell surface antigens, CD71, glycophorin-A (GPA), CD133, and CD34. The captured fetal cells be analyzed for fetal gender, and genetic abnormalities using either PCR but preferably FISH probes for chromosomes X, Y, 13, 18 and 21 as shown in figure 15. For FISH analysis, the captured cells will be fixed with methanol followed by fixation with 1% PFA. After staining with epsilon-hemoglobulin, the cells be hybridized with Vysis FISH Fetal male gender can be readily detected by the appearance of XY fluorescence signal under the fluorescence microscope. Cells stained with epsilon-hemoglobulin showing XX signal will be identified as female fetal cells. Trisomies can be readily identified also based upon whether two or three chromosomes are giving the fluorescence signals.

Alternatively, most of the RBCs can either be carefully lysed using a mild treatment with ammonium chloride lysis reagent (155 mM NH4C1 /10 mM NaHC03) to enrich for fetal nucleated red blood cells (fhRBCs) before incubating the sample with a mixture of biotinylated antibodies.

Still another option will be the use of a density gradient such as Ficol 1.073 or Percol 1.073. The buffy coat can then be processed as above to yield fetal nRBCs.

**Reference Example 8**

**Detection of Chromosomal Translocations from Captured Circulating Tumor Cells (CTCs) Using Shark and Camelid Heavy Chain Only Antibodies and Their Analogs**

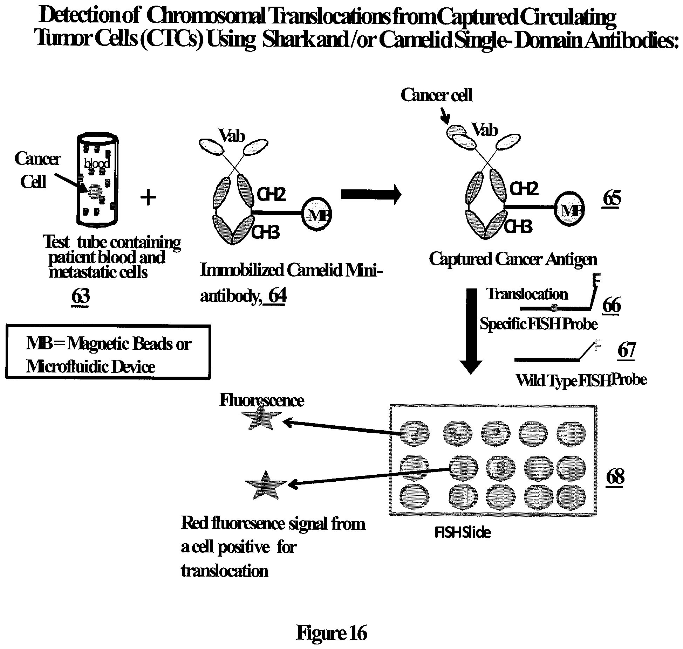

Cells will be captured as described above and also shown in Fig. 16. Enumeration of can be done using an appropriate FISH probes. For example, to test for the presence of TMPRESS2/ERG translocation in case of prostate cancer, FISH probes designed to hybridize with the junction region will be used. Similarly, in case of CML, bcr-Abl FISH probe will be used. An exemplary schematics of the process is shown in Fig. 16.

Unless otherwise defined, all technical and scientific terms used herein have the same meaning as commonly understood by one of ordinary skill in the art to which this invention belongs. All nucleotide sequences provided herein are presented in the 5' to 3' direction.

**Claims**

**1.** A sub-nano-antibody analog having the structure:

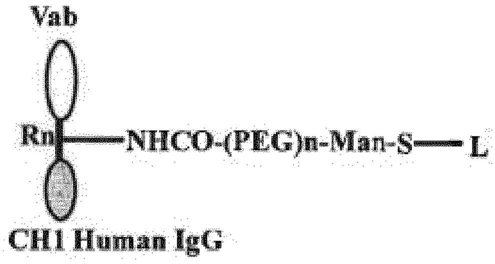

wherein:

(i) Vab is one camelid variable region of a single-domain heavy chain antibody lacking light chains,
(ii) Rn represents all of the hinge region of camelid single domain heavy chain antibodies,
(iii) n is 1-50,
(iv) Man is Maleic Anhydride and
(v) **S-L** is a thiolated ligand; wherein L is a solid matrix.

**2.** The sub-nano-antibody analog of claim 1, wherein the solid matrix of (v) is selected from the group consisting of magnetic beads, glass slides, gold nanoparticles, microchannels and microfluidic device.

**3.** A diagnostic composition comprising the sub-nano antibody analog of claims 1 or 2.

## Patentansprüche

**1.** Subnano-Antikörper-Analogon mit der Struktur:

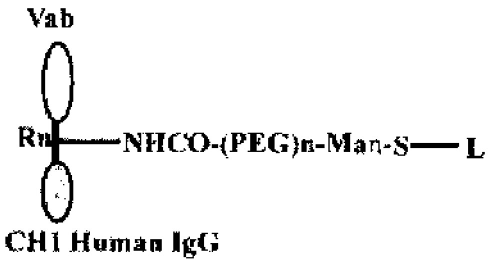

wobei:

(i) Vab eine Kamelide Variable Region eines schwerkettigen Antikörpers mit einer einzelnen Domäne ohne leichte Ketten ist,
(ii) Rn die gesamte Hinge Region von schwerkettigen Kameliden-Antikörpern mit einer einzelnen Domäne darstellt,
(iii) n 1 bis 50 ist,
(iv) Man Maleinsäureanhydrid ist und
(v) S-L ein thiolierter Ligand ist; wobei L eine feste Matrix ist.

**2.** Subnano-Antikörper-Analogon nach Anspruch 1, wobei die feste Matrix von (v) aus der Gruppe bestehend aus magnetischem Granulat, Glasobjektträgern, Gold-Nanopartikeln, Mikrokanälen und mikrofluidischer Vorrichtung ausgewählt wird.

**3.** Diagnostische Zusammensetzung bestehend aus dem Subnano-Antikörper-Analogon der Ansprüche 1 oder 2.

## Revendications

**1.** Un analogue d'anticorps sub-nanométrique possédant la structure :

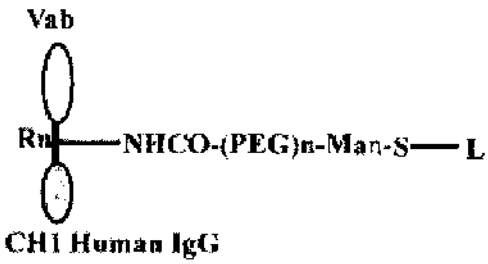


dans lequel :

(i) Vab est une région variable d'un anticorps à chaîne lourde à domaine unique de camélidé n'ayant pas de chaînes légères,
(ii) Rn représente toute la région charnière des anticorps à chaîne lourde à domaine unique de camélidé
(iii) n est entre 1 et 50,
(iv) Man est l'anhydride maléique et
(v) S-L est un ligand thiolé ; L étant une matrice solide.

**2.** L'analogue d'anticorps sub-nanométrique selon la revendication 1, dans lequel la matrice solide de (v) est choisie parmi le groupe constitué de billes magnétiques, de lames de verre, de nanoparticules d'or, de microcanaux et d'un dispositif microfluidique.

**3.** Une composition de diagnostic comprenant l'analogue d'anticorps sub-nanométrique selon les revendications 1 ou 2.

# Comparison of Structures of Camelid, Shark and Mouse Antibodies

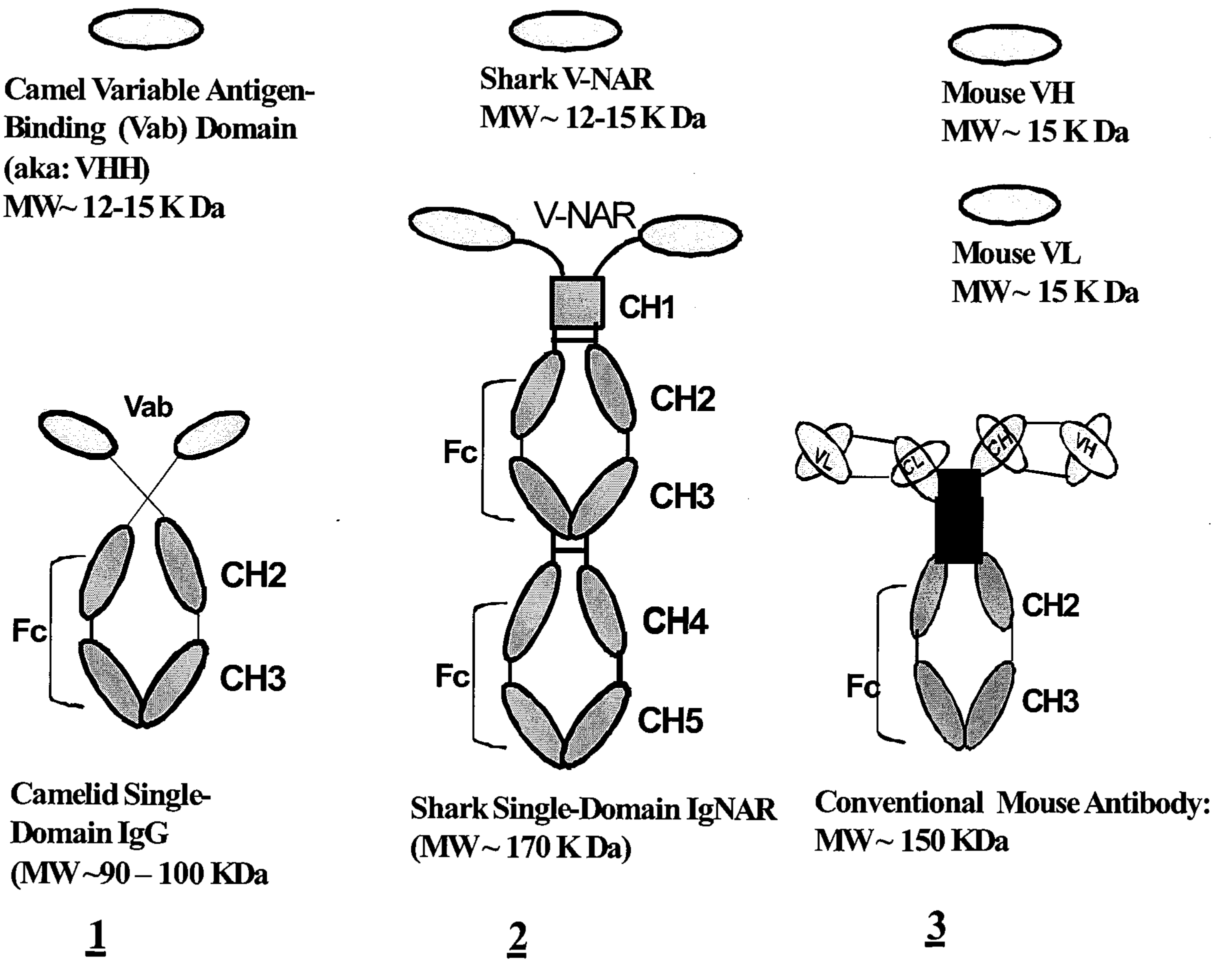


Figure 1

# Novel Analogs of Heavy-chain antibodies

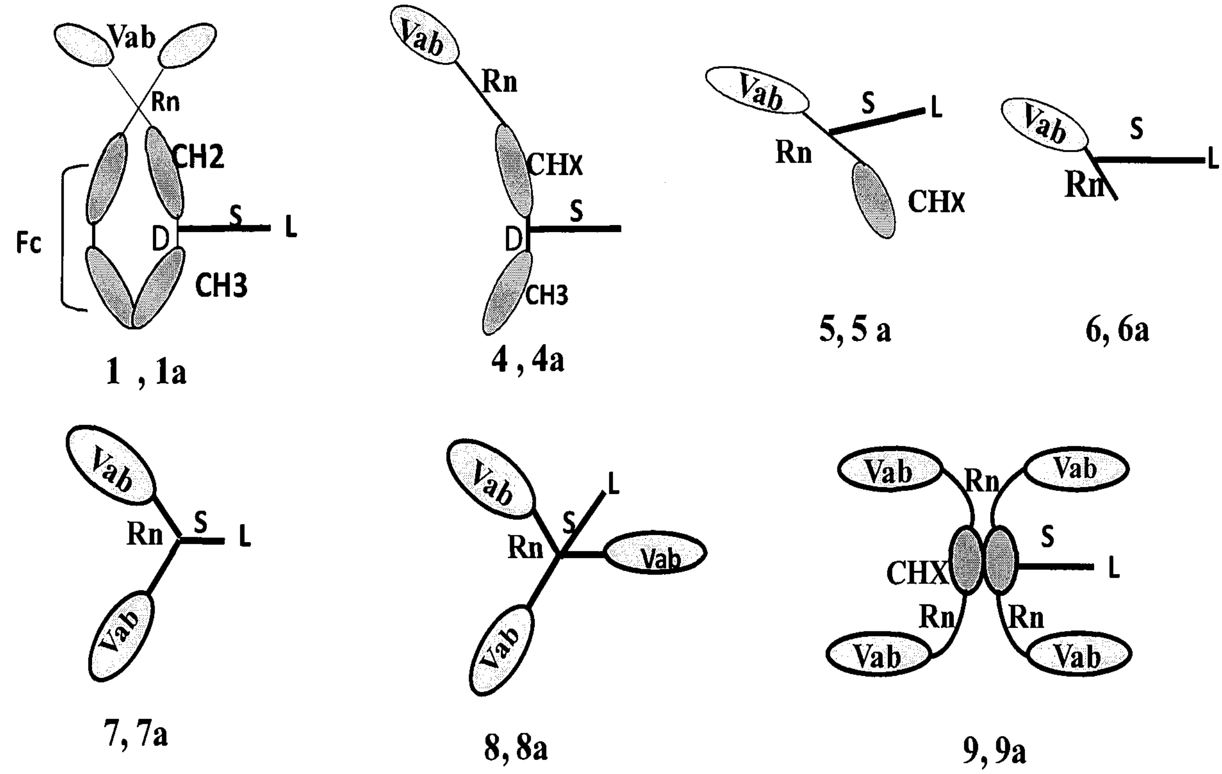


Figure 2

# Novel Analogs of Single-Domain Heavy-Chain Shark Antibodies

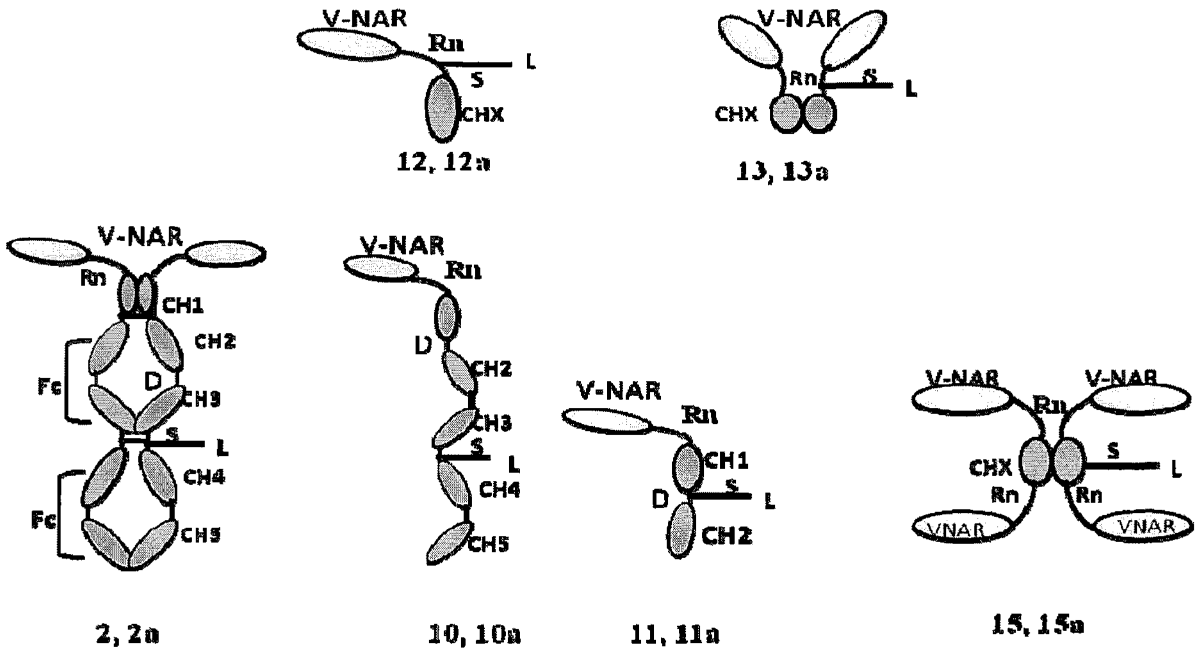


**Wherein**

"a" after the digit/number indicates modified analog of the numbered native camelid and/or shark antibody. For example structure "1a" is the analog derived from native, unmodified antibody "1".

**S** = Heterobifunctional linker with one end being capable of conjugating with heavy-chain antibodies, and the other end with haptens, enzymes, and solid matrixes.

Generic composition of "S" is shown below:

$$S = X \overset{P}{\text{————————}} Y$$

**X** = NHS, Maleimide, CHO, COOH, CN, SCN, Epoxide, $NH_2$, Phosphate, thiophosphate, etc

**"Y"** Forms covalent bond with NH2, SH, CHO, NHS groups of fluorophores, haptens, enzymes, proteins, gold, magnetic particles, and solid matrixes

# Figure 3 (page 1 of 2)

**Y** = Maleimide, CHO, COOH, SH, SCN, Epoxide, Phosphate, Thiophosphate

NHS (N-hydroxy-succinimide) with or without a sulfonate group.

$(CH_2CH_2O)n$ where n= 1-500

$(CH_2)n$ where n = 1-15

**P** = $(CH_2\text{-R-NHCO})n$ and n = 1-100

R= methyl, ethyl, isopropyl, phenyl, alkylphenyl, etc.

= Nucleic acids, albumins, proteins and peptides, hydrophilic polymers

**L**= Biotin, Digoxegenin, Fluorescein, Cy3, Cy5, Cy7, Bodipy dyes, TAMRA, BHQ, MGBNQ, Texas Red, Alexa Fluores 350-750, Rhodamine, Oregon Green Dyes, SYTO dyes, Cascade Blue, Starbright Orange.

= Anti-camelid/shark-biotin-antibody, Streptavidin (SA), Avidin (AV)

= Horseradish Peroxidase (HRP), Alkaline Phosphatase (AP), Luciferase, □-galactosidase,

= Streptavidin-HRP, Streptavidin-AP, Streptavidin-Luciferase, Streptavidin- Galactosidase.

= Solid matrixes, such as, gold nanoparticles, magnetic particles, glass particles, glass slides, microarrays, microchannels, microfluidic devises and their appropriately modified analogs that generates reactive groups for covalent conjugation with "Y".

= Nucleic acids, 10 bases to 1000 bases long, chimeric or not, PNA, LNA, phosphorothioates, methylphosphonates, Si-RNA, micro-RNA, RNA, RNA -Analogues

= radioisotope

# Figure 3 Continued (page 2 of 2)

## Chemical Synthesis of Analogs of Camelid Antibodies

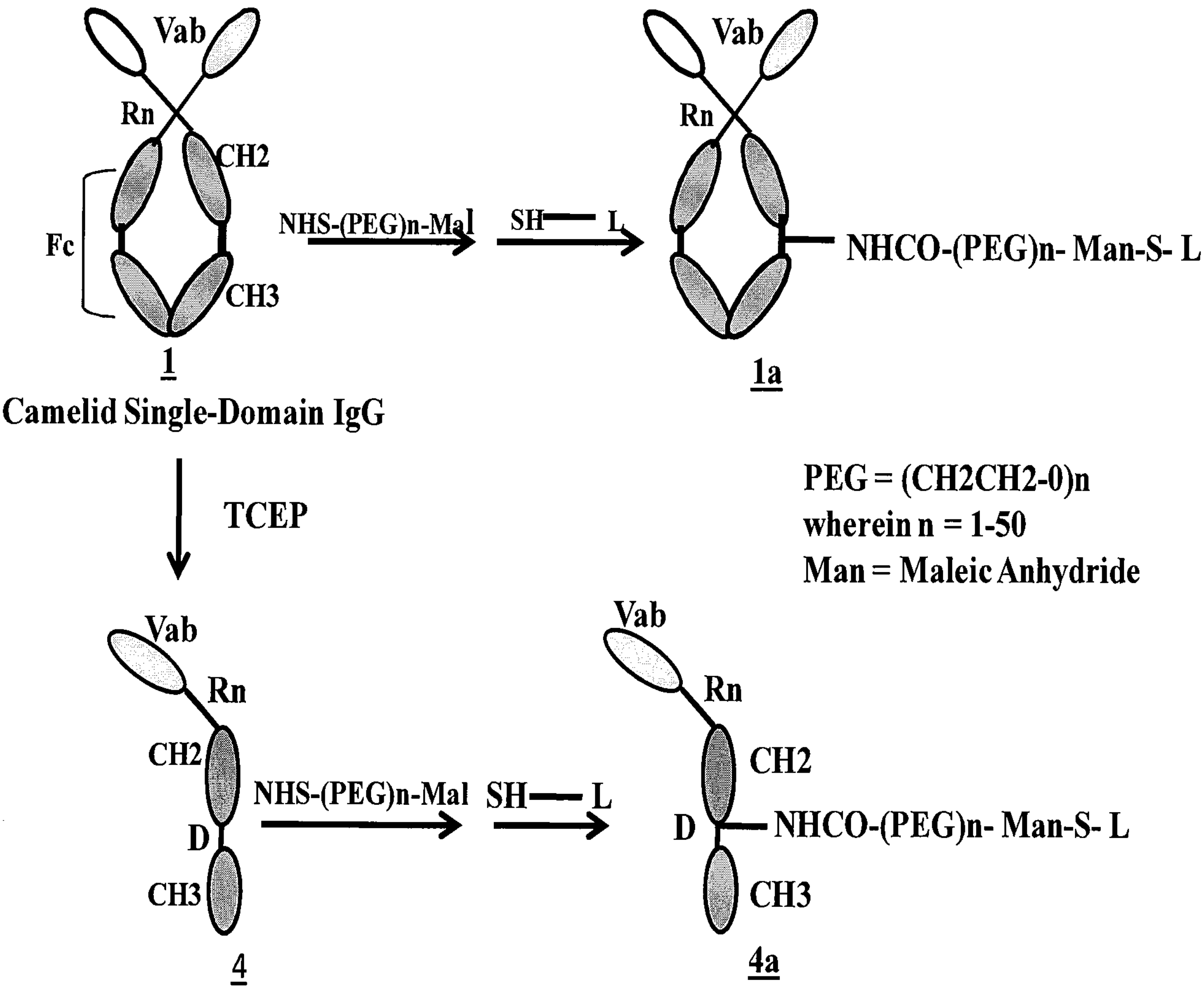


Figure 4

## Chemical Synthesis of Camelid Bivalent Nano-antibodies

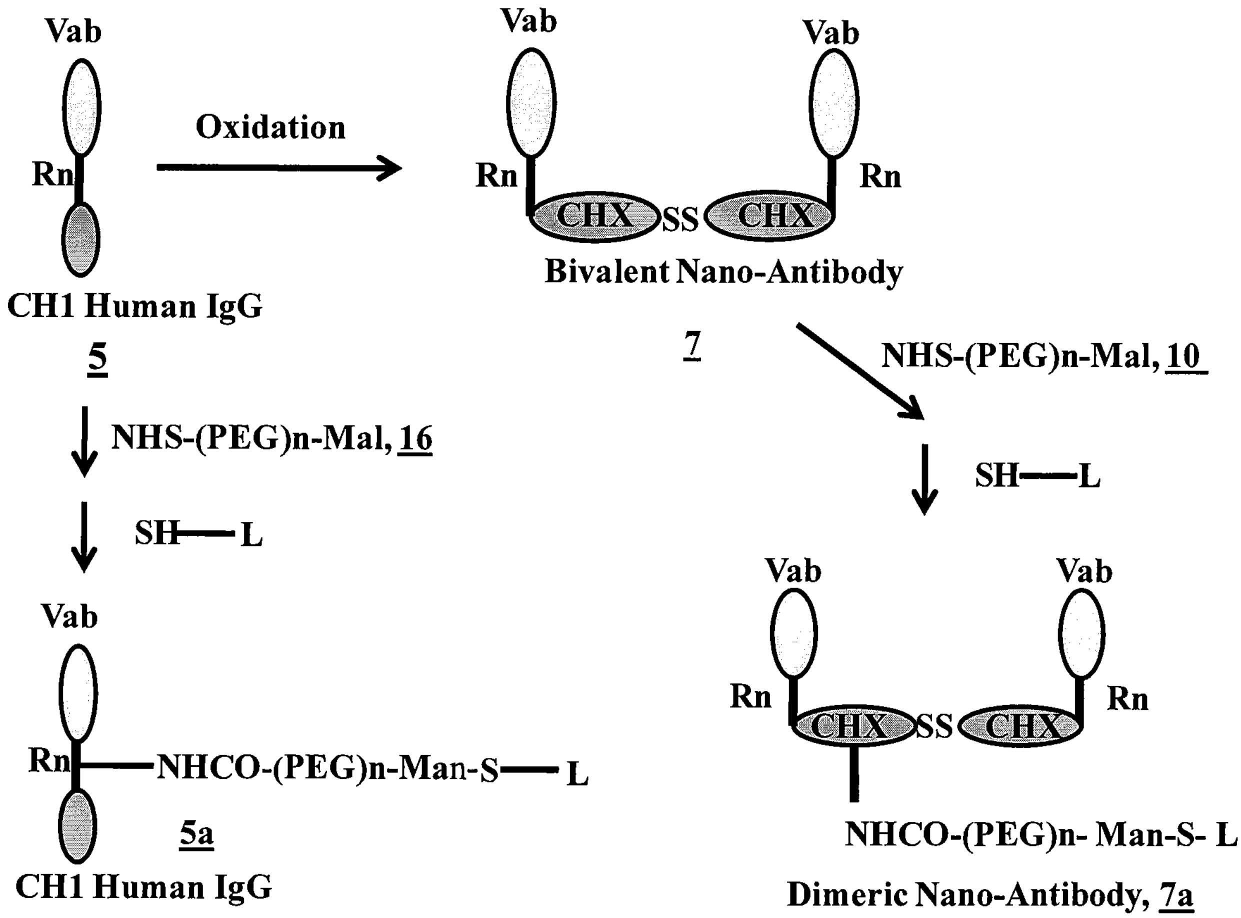


Figure 5

## Chemical Synthesis of Camelid Trimeric and Tetrameric Nano-antibodies

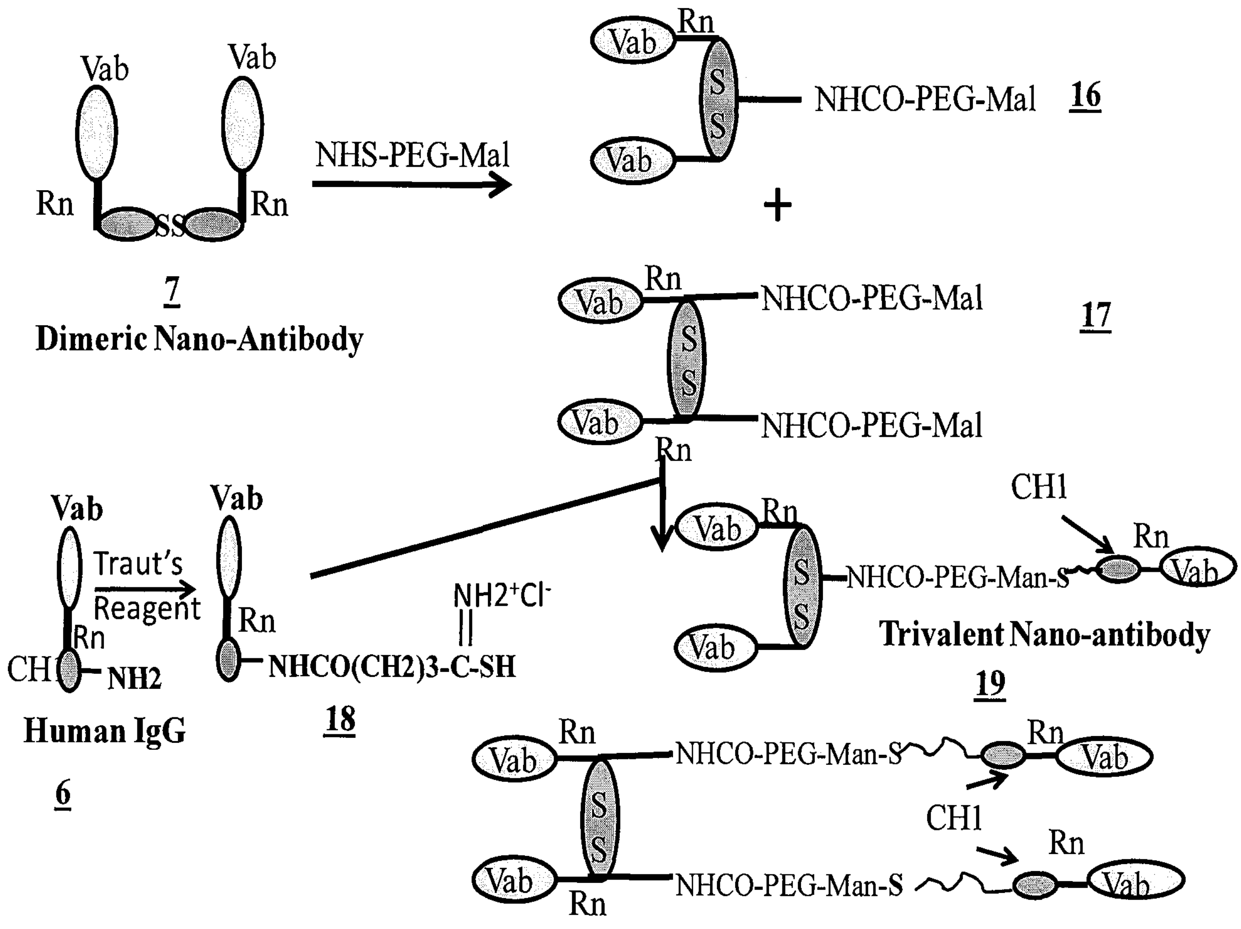


Figure 6 Tetrameric Camelid Nano-antibody, 20

**Schematics for Cloning and Expression of Single-Domain Shark Antibodies and their Analogs**

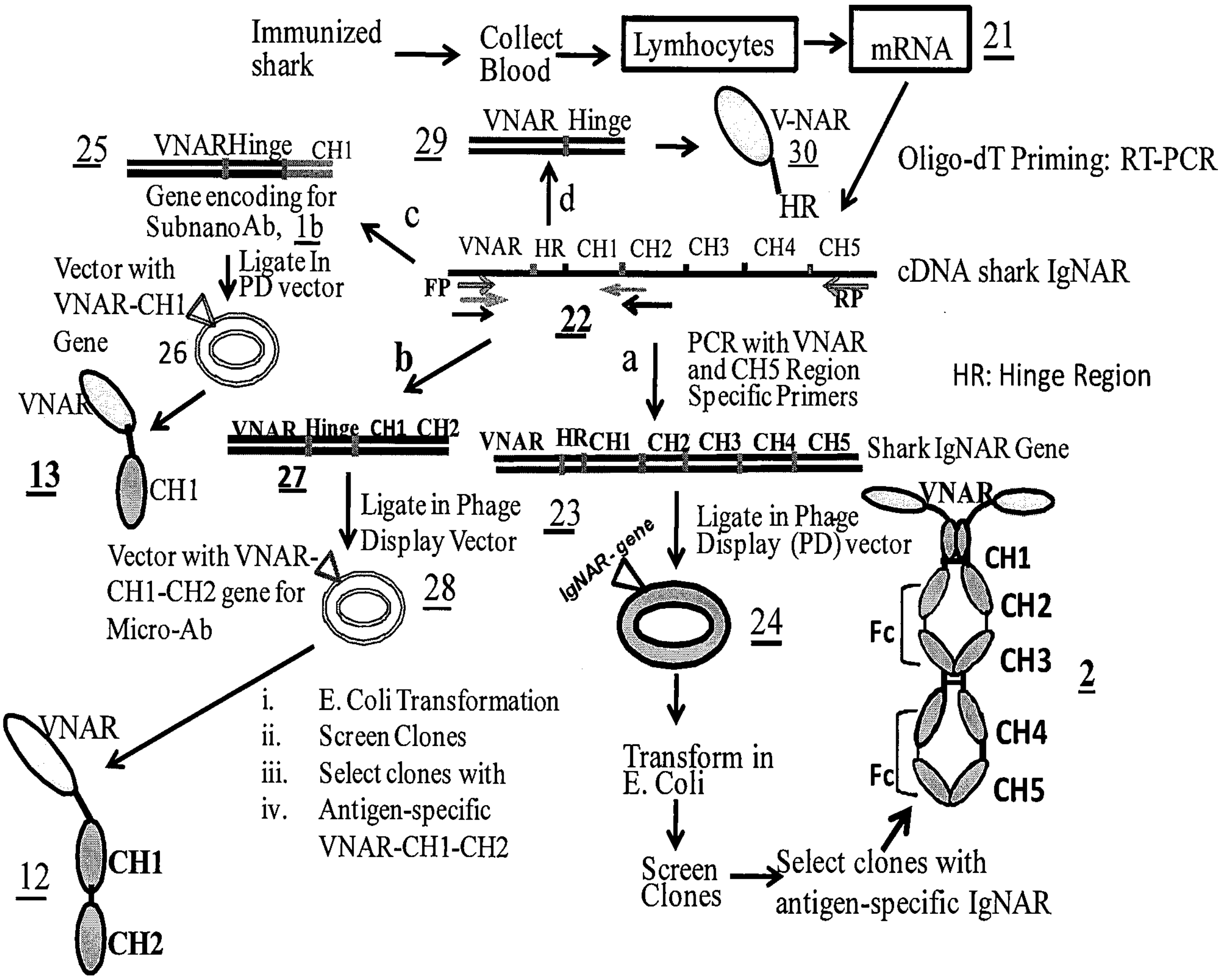


Figure 7

# Analogs of Single-Domain Shark Antibodies

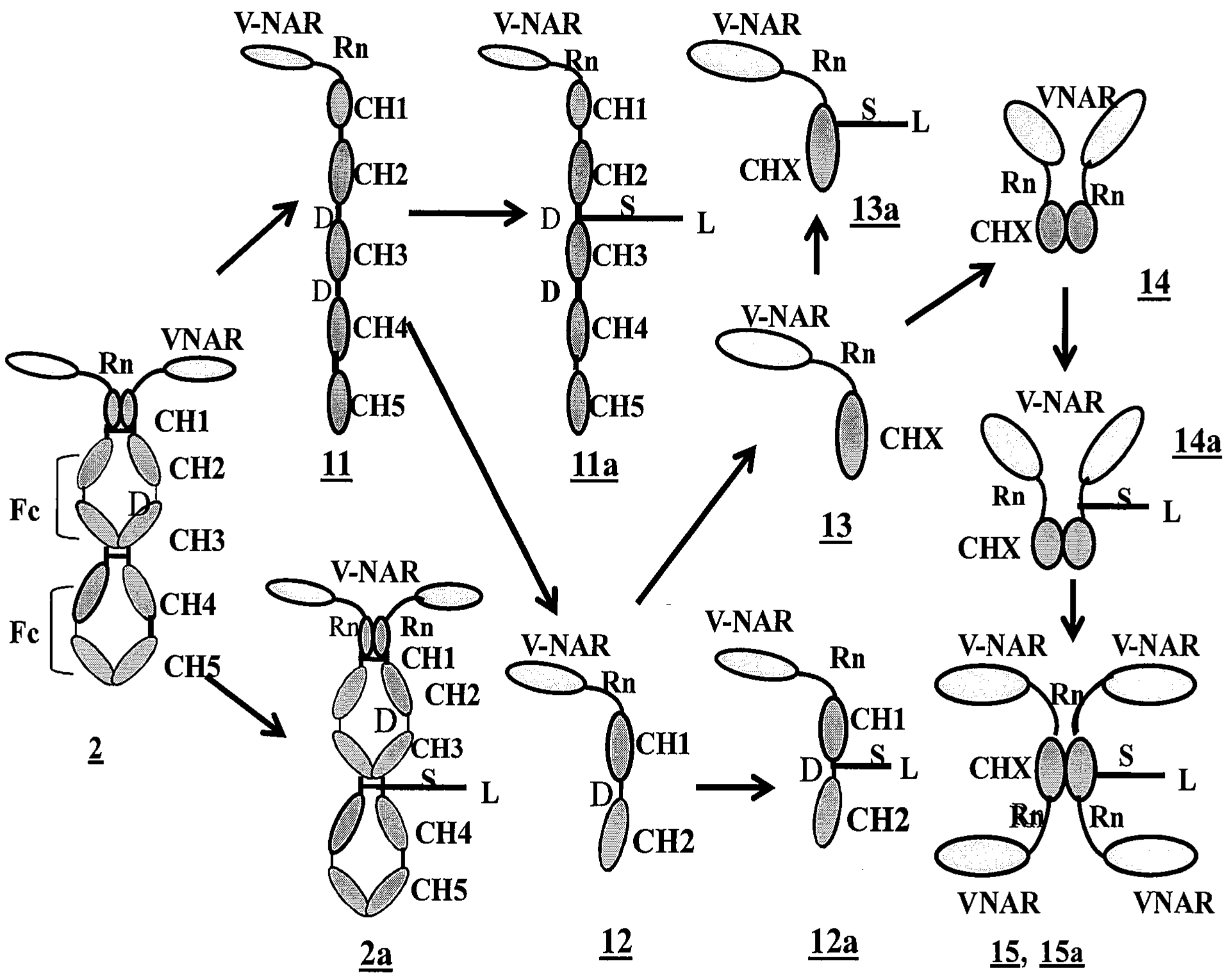


Figure 8

## Chemical Synthesis of Shark Dimeric Nano-Antibodies and Analogs

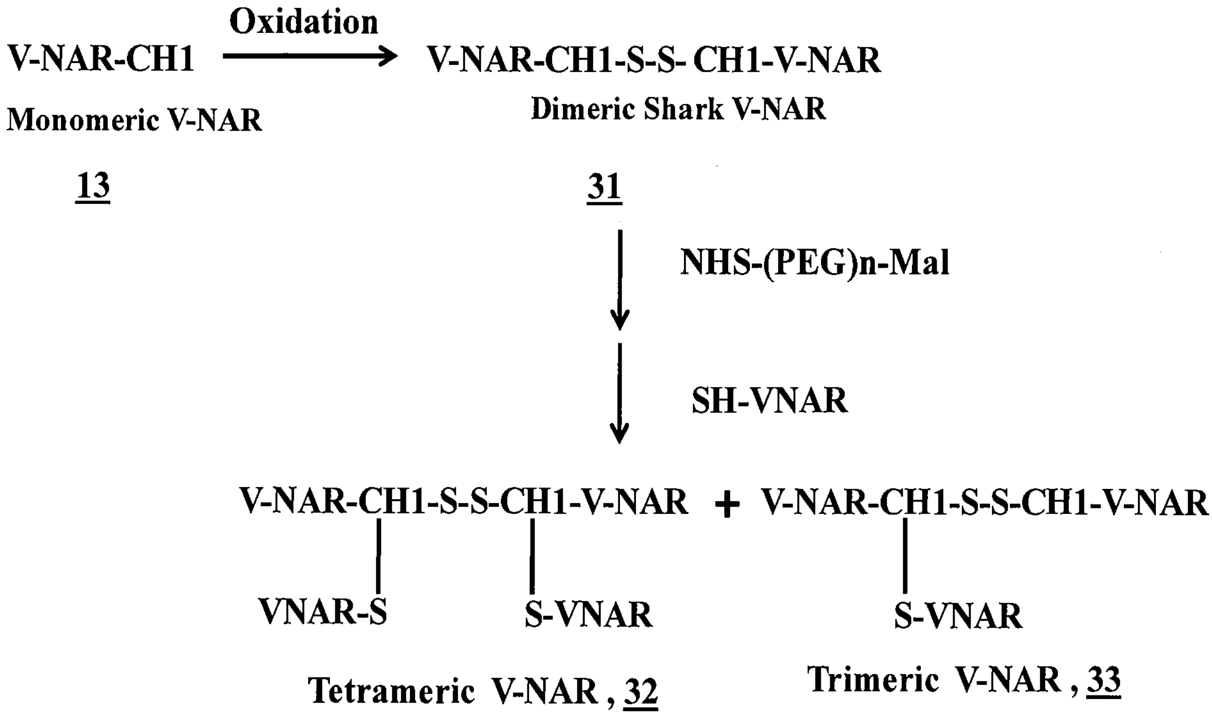


Figure 9

## Immobilization of Single-Domain Heavy-Chain Camelid and Shark Antibodies

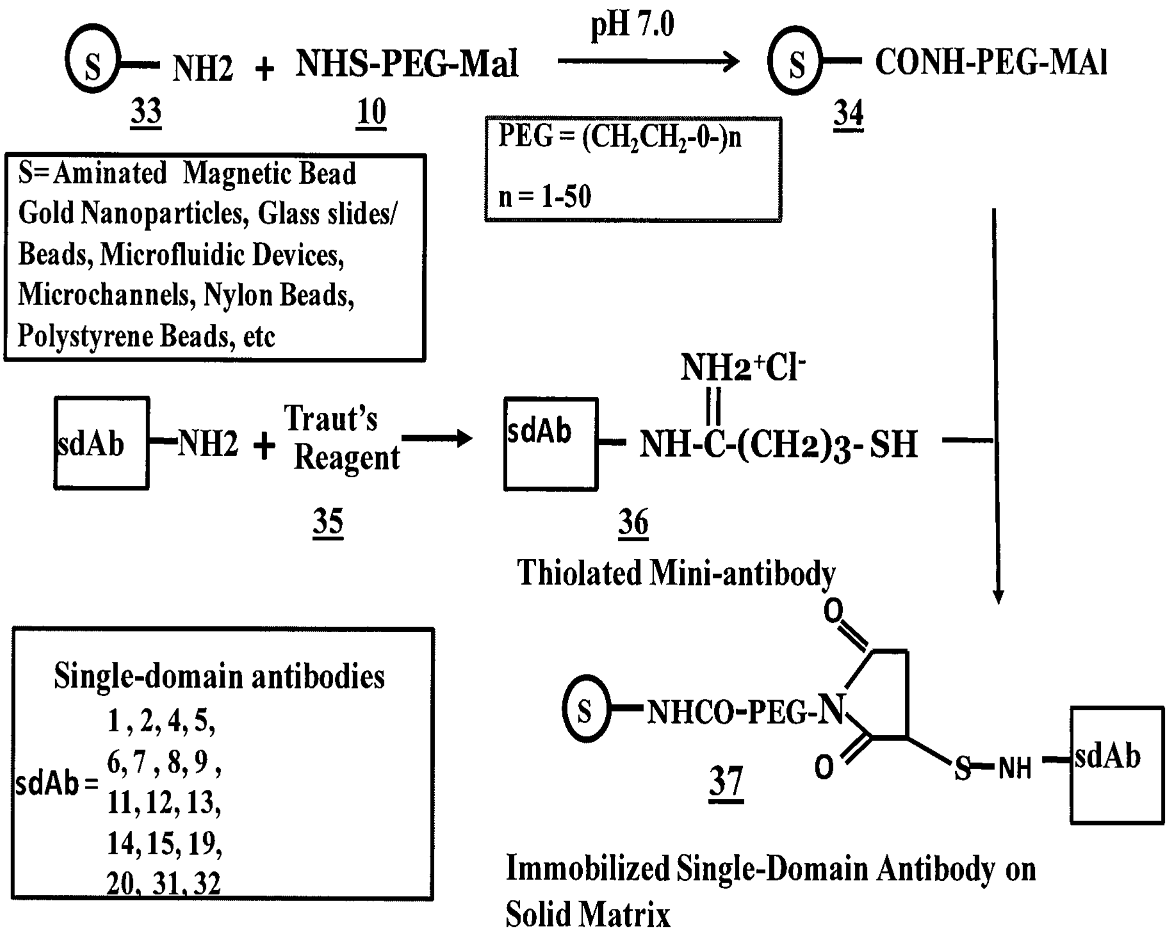


Figure 10

In-Vitro Detection of Pathological Proteins using Camelid and Shark Antibodies and Enzymatic Signal Amplification

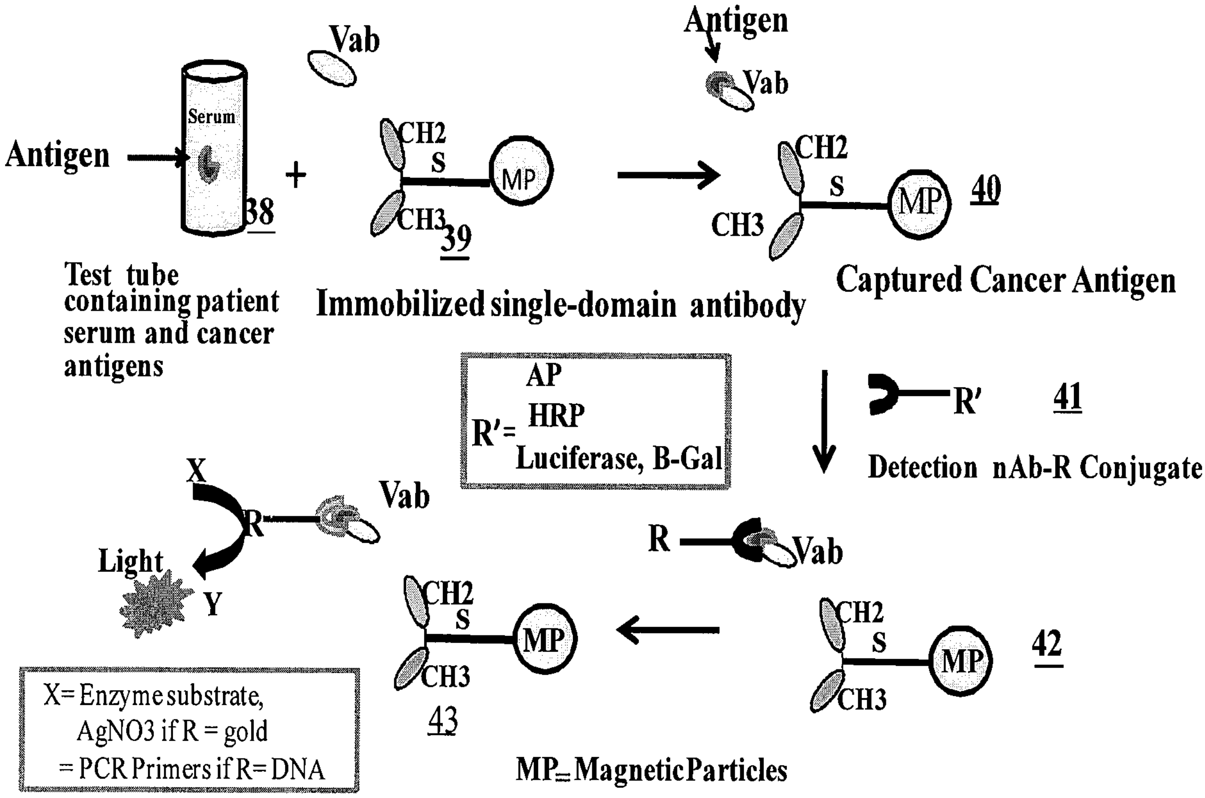


Vab = Variable antigen-binding domain of shark and camelid single-domain antibodies

Figure 11

## In-Vitro Detection of Pathological Proteins using Camelid and Shark Antibodies and Enzymatic Signal Amplification

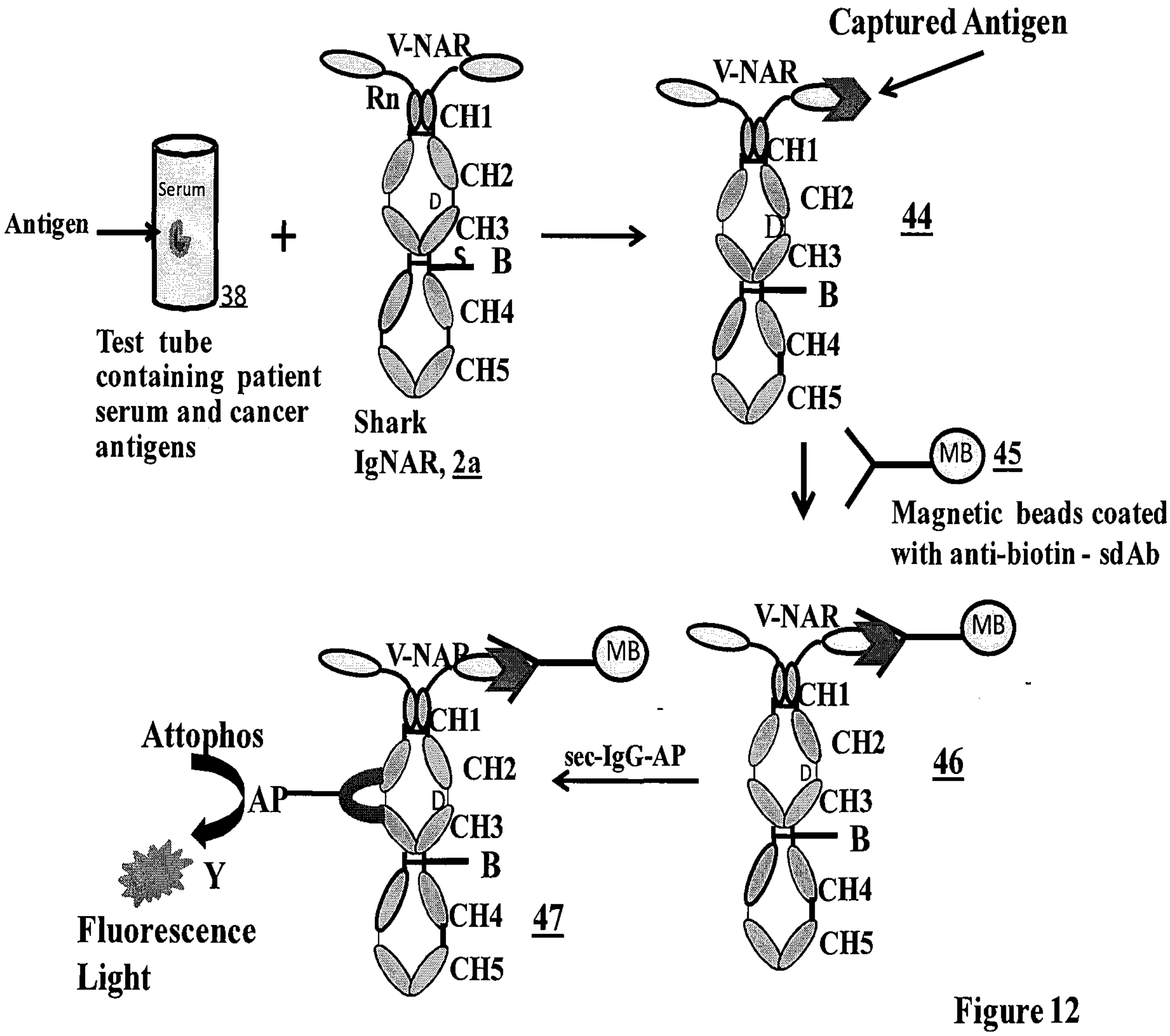


Figure 12

## Use of Immuno-PCR and Single-Domain Camelid / Shark Antibodies to Develop Ultra-sensitive Diagnostic Technology to Capture and Detect < 200 molecules of Pathological Proteins

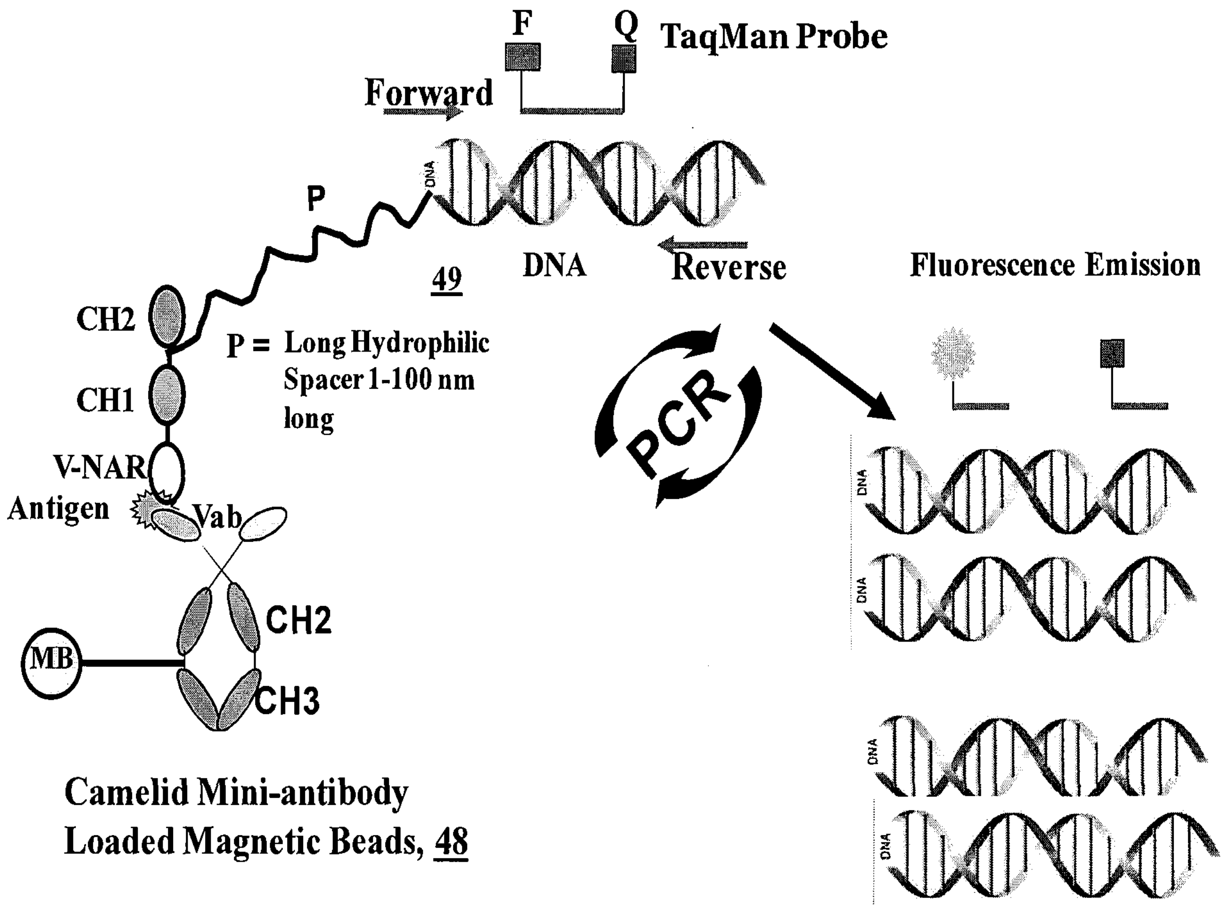


Figure 13

**In-Vitro Capture and Detection of Circulating Tumor Cells (CTCs) Using Single-Domain Shark and / or Camel Antibodies:**

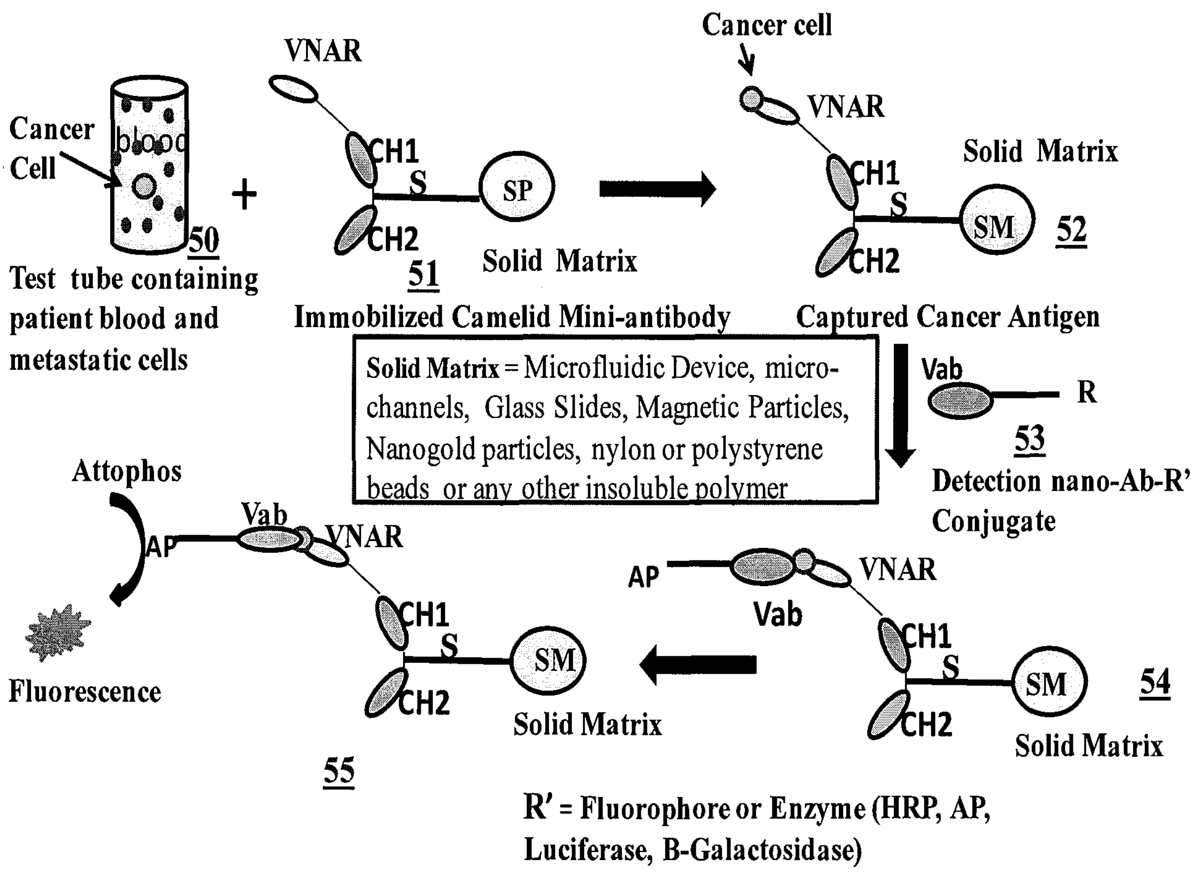


Figure 14

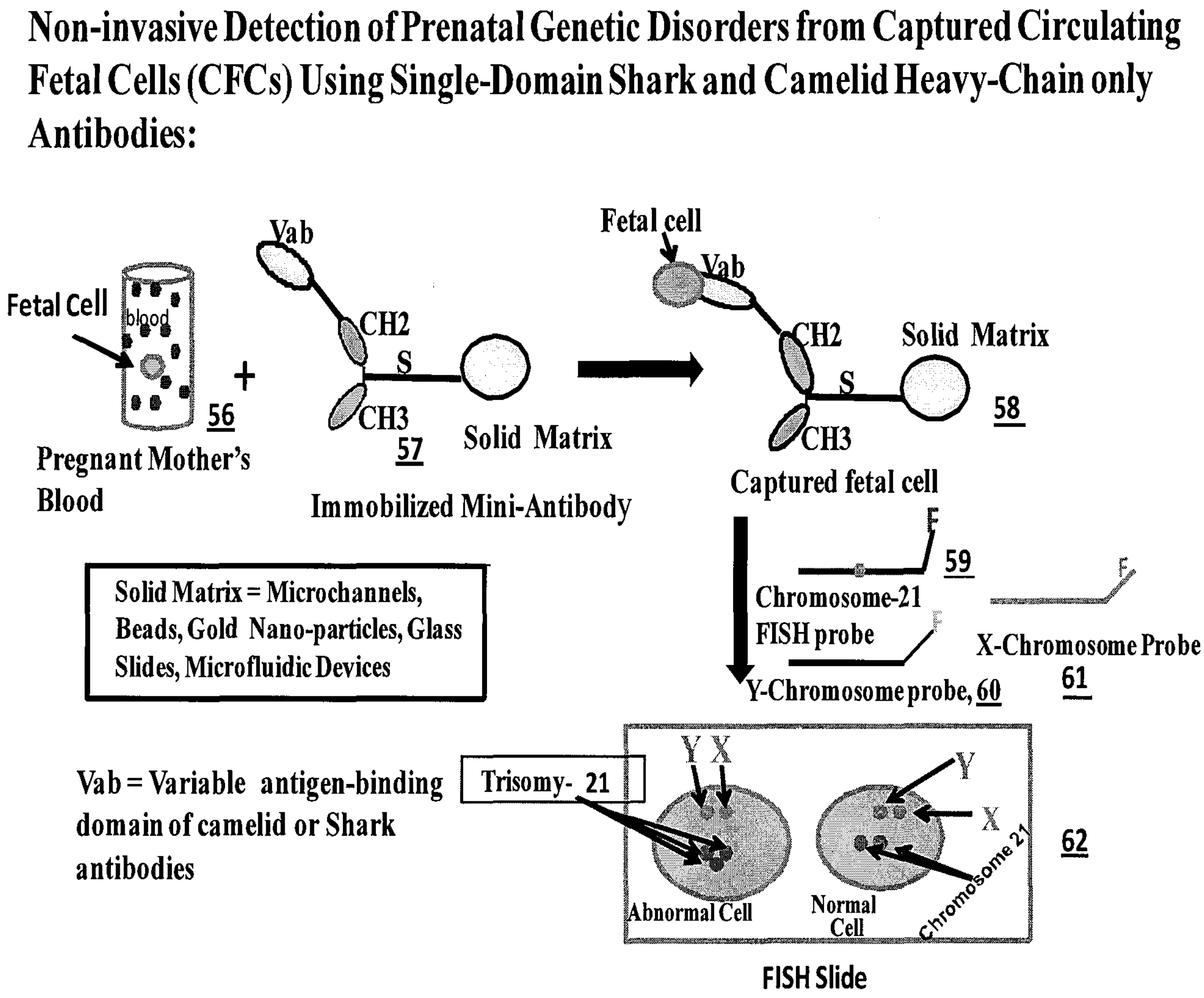
Non-invasive Detection of Prenatal Genetic Disorders from Captured Circulating Fetal Cells (CFCs) Using Single-Domain Shark and Camelid Heavy-Chain only Antibodies:
Fetal Cell
blood
56
Pregnant Mother's Blood
Vab
CH2
S
CH3
57
Solid Matrix
Immobilized Mini-Antibody
Fetal cell
Vab
CH2
Solid Matrix
S
58
CH3
Captured fetal cell
Solid Matrix = Microchannels, Beads, Gold Nano-particles, Glass Slides, Microfluidic Devices
F
59
Chromosome-21 FISH probe
X-Chromosome Probe
61
Y-Chromosome probe, 60
Vab = Variable antigen-binding domain of camelid or Shark antibodies
Trisomy- 21
Y X
Y
X
Chromosome 21
Abnormal Cell
Normal Cell
62
FISH Slide

Figure 15

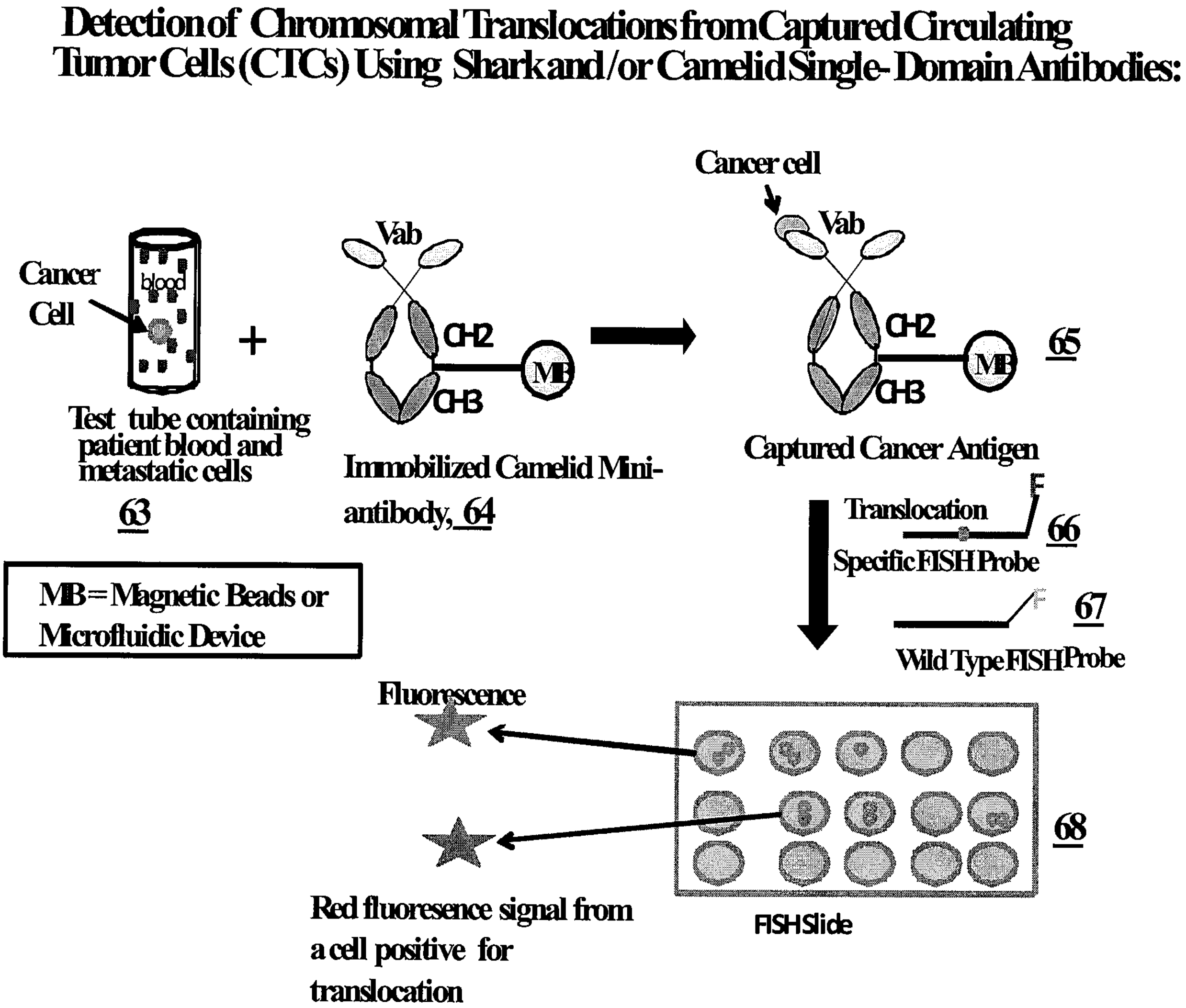
Detection of Chromosomal Translocations from Captured Circulating Tumor Cells (CTCs) Using Shark and/or Camelid Single-Domain Antibodies:
Cancer Cell
blood
+
Test tube containing patient blood and metastatic cells
63
Vab
CH2
CH3
MB
Immobilized Camelid Mini-antibody, 64
Cancer cell
Vab
CH2
CH3
MB
65
Captured Cancer Antigen
Translocation Specific FISH Probe
F
66
F
67
Wild Type FISH Probe
MB = Magnetic Beads or Microfluidic Device
Fluorescence
Red fluoresence signal from a cell positive for translocation
FISH Slide
68

Figure 16

1 ggccggtcag cgtcgctgcc ggtctccggc ggagacggac tctggagttt gggcggcccg
61 ggcggccact aggtactctg atattccgta ctaaacacgt ctgcaagtca agatgtcgca
121 cccgtccccc caagccaagc cctccaaccc cagtaaccct cgagtcttct ttgacgtgga
181 catcggaggg gagcgagttg gtcgaattgt cttagaattg tttgcagata tcgtacccaa
241 aactgcggaa aattttcgtg cactgtgtac aggagaaaaa ggcattggac acacgactgg
301 gaaacctctc catttcaaag gatgcccttt tcatcgaatt attaagaaat ttatgattca
361 gggtggagac ttctcaaatc agaatgggac aggtggagaa agtatttatg gtgaaaaatt
421 tgaagatgaa aatttccatt acaagcatga tcgggagggt ttactgagca tggcaaatgc
481 aggccgcaac acaaacggtt ctcagttttt tatcacaaca gttccaactc ctcatttgga
541 tgggaaacat gtggtgtttg gccaagtaat taaaggaata ggagtggcaa ggatattgga
601 aaatgtggaa gtgaaaggtg aaaaacctgc taaattgtgc gttattgcag aatgtggaga
661 attgaaggaa ggagatgacg gggaatatt cccaaaagat ggctctggcg acagtcatcc
721 agatttccct gaggatgcgg atatagattt aaaagatgta gataaaattt tattaataac
781 agaagactta aaaaacattg gaaatacttt tttcaaatcc cagaactggg agatggctat
841 taaaaaatat gcagaagttt taagatacgt ggacagttca aaggctgtta ttgagacagc
901 agatagagcc aagctgcaac ctatagcttt aagctgtgta ctgaatattg gtgcttgtaa
961 actgaagatg tcaaattggc agggagcaat tgacagttgt ttagaggctc ttgaactaga
1021 cccatcaaat accaaagcat tgtaccgcag agctcaagga tggcaaggat taaaagaata
1081 tgatcaagca ttggctgatc ttaagaaagc tcaggggata gcaccagaag ataaagctat
1141 ccaggcagaa ttgctgaaag tcaaacaaaa gataaaggca cagaaagata aagagaaggc
1201 agtatatgca aaaatgtttg cttagaaagg attcagtttt gcttattgtg tgttgattgt
1261 ataaatgcaa taagaaaatg taaaggtttt tgtctatgaa tatgatccct aatgtgtttc
1321 ttttgacacc ttagttcctt actgtttaca gtttaggagt actgataggg gttcatgctt
1381 aataaacatg tcacaataca gtaagtaaag tggttttgtt tgtttctttg agatggagtc

**FIGURE 17 (page 1 of 2)**

1441 ttgctctgtc acccaggctg gagtgcggtg gcgcaatctc ggctcactgc atcctctgcc

1501 tcccgggttc aagcaattct cctgcctcag cttcccaagt agctgggatt acaggcacgt

1561 gccaccacgc ccagctaatt tttgtatttt tagtagagat ggggtttcac catattggtc

1621 acgtcacgtt ggtcttgaac tcctgacctt gtgatccacc ccgccttggc ctcccaaagt

1681 gctgggatta caggtgtgag ccaccgtgcc cggccaagta aaatgttttt taaaatggtt

1741 atgtgcatta ttcataaaaa ataatggtgt ccagtctttt taaacttgta aagacacatc

1801 ttattgaata aagagatgag agcttaagtt tgtaaaaaaa aaaaaaaaaa a

**FIGURE 17 Continued (page 2 of 2)**

```
  1 atccccatcc cgtggagtgg ccggcgacaa gatggcagca gcgtgtcgga gcgtgaaggg
 61 cctggtggcg gtaataaccg gaggagcctc gggcctgggc ctggccacgg cggagcgact
121 tgtggggcag ggagcctctg ctgtgcttct ggacctgccc aactcgggtg gggaggccca
181 agccaagaag ttaggaaaca actgcgtttt cgccccagcc gacgtgacct ctgagaagga
241 tgtgcaaaca gctctggctc tagcaaaagg aaagtttggc cgtgtggatg tagctgtcaa
301 ctgtgcaggc atcgcggtgg ctagcaagac gtacaactta aagaagggcc agacccatac
361 cttggaagac ttccagcgag ttcttgatgt gaatctcatg ggcaccttca atgtgatccg
421 cctggtggct ggtgagatgg gccagaatga accagaccag ggaggccaac gtggggtcat
481 catcaacact gccagtgtgg ctgccttcga gggtcaggtt ggacaagctg catactctgc
541 ttccaagggg ggaatagtgg gcatgacact gcccattgct cgggatctgg ctcccatagg
601 tatccgggtg atgaccattg ccccaggtct gtttggcacc ccactgctga ccagcctccc
661 agagaaagtg tgcaacttct tggccagcca agtgcccttc cctagccgac tgggtgaccc
721 tgctgagtat gctcacctcg tacaggccat catcgagaac ccattcctca atggagaggt
781 catccggctg gatggggcca ttcgtatgca gccttgaagg gagaaggcag agaaaacaca
841 cgctcctctg cccttccttt ccctggggta ctactctcca gcttgggagg aagcccagta
901 gccattttgt aactgcctac cagtcgccct ctgtgcctaa taaagtctct tttctcaca
961 gag
```

# FIGURE 18

```
   1 tcctttccgc ttccggtgtc ccctacagtc atggctgccg ccgtcgctgc tgccggtgca
  61 ggggaacccc agtccccgga cgaattgctc ccgaaaggcg acgcggagaa gcctgaggag
 121 gagctggagg aggacgacga tgaggagcta gatgagaccc tgtcggagag actatggggc
 181 ctgacggaga tgtttccgga gagggtccgg tccgcggccg gagccacttt tgatctttcc
 241 ctctttgtgg ctcagaaaat gtacaggttt tccagggcag ccttgtggat tgggaccact
 301 tcctttatga tcctggttct tcccgttgtc tttgagacgg agaagttgca aatggagcaa
 361 cagcagcaac tgcagcagcg gcagatactt ctaggaccta acacagggct ctcaggagga
 421 atgccagggg ctctaccctc acttcctgga aagatctaga ttgttattgc tgtttgagct
 481 gtctcagtgg gataagtttg aaattcaagt gtttgaactg ctgataattt ggattttttt
 541 tttttttaa ctttggcaca ttgatctatc taaacctggt ggggagaatt atccccacat
 601 tgtctcatgg aaagactcaa cttgcaactg tgccctccac actatcctta cttctgtctc
 661 cactctgata ccagagtgca gccatgcaga tggttattcc agctctggtc acccgactcc
 721 tttcaccaaa ttgctcctaa ctggaagatc tcactttccc cttgtggggt aggaaccgat
 781 gccagtggga gggatgtgcc cctgaccatt aacgactgtt tttttttttt ttttttaaag
 841 aatggagttg ttggggcagg acatgcacac aatgtgaaac agacaaaatg cattacacct
 901 gtagtgtaaa gtggccacta tgaatcccta tgtatgagag gagggaggca ggctgcagct
 961 tcagccacag aatggggact atggaagaca gcaggagctc atttcctctg cacattttgg
1021 ctgttagacc tgtgtgtgtg tttaaaaaaa gagaagtcag tgctcacttt ttgtatttaa
1081 atattaaaaa tgattccaac tgaaagtgtc atcctaagta ccttgaaatg agaccacgtc
1141 agagacatgt actgcccctc acattttctc acctaaacca gcagcacctc catcttaaca
1201 gccataggcc caaattgttt ccaagtgaaa attcattttt agccaagtac ttcatagcaa
1261 tctttgccct gaatttaggc agtcactttg agatccatca gcctaaaaca aaggattggg
1321 tctatgtact tctttagtct ataatgacac tgtgtaatta taaagtattt gtagggaaaa
1381 aaaaaaaaaa aaaaaa
```

# FIGURE 19

TGGTACCCGGGATTCGGCCATTACGGCCGGGGGGGTCTTTTCGTATTTGTCATACCGTGA
TCCGAACTTGCTGAAAACACTAGAGGTCTATGATGGGACTGCAAAGTTCCTCAGAGAACT
AGATGTAGATGATGATGCTCTCACAAAAGCTATTATTGGAACCATCGGGGATGTTGATTC
CTACCAGCTACCAGATGCTAAAGGTTACAGCAGTCTGATGCGGTATTTGTTGGGCATCAC
CGAGGAGGAACGCCAGCAAAGGCGCGAAGAGATACTCGCAACCAGCGTGAAGGATTTCAA
GGAGTTTGCCGATGCTGTCGAAACGATCAATGACAATGGGGTTGTGGTGGCTGTAGCATC
GCCTGACGACGTCGAGGCAGCAAACAAAGAGAAGTCGTTATTTTCAGACATCAAGAAGTG
CCTGTGAGGCCTTTCCATTCCCACGCCGGCAGGGTTCAGCCTGGACCTGCGCCGAGCAGA
GGTTCTTCAGCGTTTTGCTTCACTGATGAAGAATGGTCTGGCGCTGTAACATGAGTTCGC
GTGGCGAGAGCACTGATCACCTTGTCTGTAGCCCGGTTACGGTTGTTTATTTTTCGCGCC
CATTTTTAGGGGAAATAATTAGTTCAGGGTTTTGAGCGAAGCACAATAGTTAAACAGAGT
TCCATTGCTGCCTTTCCCCCTCTGCAATCAGTGCTCAAACAAGCCGGTTGTACACCAAGT
TGCTGATAGGAGATTAAGAGCTGATGAGTGATGAGAACCTGCTCAAAATTTAGCTGAATA
CTCTGTAACTTGATATACCAAAGATATAAAGGTATTGGACTACTGATT

**FIGURE 20**

```
   1 atgaatttac aaccaatttt ctggattgga ctgatcagtt cagtttgctg tgtgtttgct
  61 caaacagatg aaaatagatg tttaaaagca aatgccaaat catgtggaga atgtatacaa
 121 gcagggccaa attgtgggtg gtgcacaaat tcaacatttt tacaggaagg aatgcctact
 181 tctgcacgat gtgatgattt agaagcctta aaaaagaagg gttgccctcc agatgacata
 241 gaaaatccca gaggctccaa agatataaag aaaaataaaa atgtaaccaa ccgtagcaaa
 301 ggaacagcag agaagctcaa gccagaggat attactcaga tccaaccaca gcagttggtt
 361 ttgcgattaa gatcagggga gccacagaca tttacattaa aattcaagag agctgaagac
 421 tatcccattg acctctacta ccttatggac ctgtcttact caatgaaaga cgatttggag
 481 aatgtaaaaa gtcttggaac agatctgatg aatgaaatga ggaggattac ttcggacttc
 541 agaattggat ttggctcatt tgtggaaaag actgtgatgc cttacattag cacaacacca
 601 gctaagctca ggaacccttg cacaagtgaa cagaactgca ccagcccatt tagctacaaa
 661 aatgtgctca gtcttactaa taaaggagaa gtatttaatg aacttgttgg aaaacagcgc
 721 atatctggaa atttggattc tccagaaggt ggtttcgatg ccatcatgca agttgcagtt
 781 tgtggatcac tgattggctg gaggaatgtt acacggctgc tggtgttttc cacagatgcc
 841 gggtttcact ttgctggaga tgggaaactt ggtggcattg ttttaccaaa tgatggacaa
 901 tgtcacctgg aaaataatat gtacacaatg agccattatt atgattatcc ttctattgct
 961 caccttgtcc agaaactgag tgaaaataat attcagacaa tttttgcagt tactgaagaa
1021 tttcagcctg tttacaagga gctgaaaaac ttgatcccta agtcagcagt aggaacatta
1081 tctgcaaatt ctagcaatgt aattcagttg atcattgatg catacaattc cctttcctca
1141 gaagtcattt tggaaaacgg caaattgtca gaaggcgtaa caataagtta caaatcttac
1201 tgcaagaacg gggtgaatgg aacaggggaa aatggaagaa aatgttccaa tatttccatt
1261 ggagatgagg ttcaatttga aattagcata acttcaaata agtgtccaaa aaaggattct
1321 gacagcttta aaattaggcc tctgggcttt acggaggaag tagaggttat tcttcagtac
1381 atctgtgaat gtgaatgcca aagcgaaggc atccctgaaa gtcccaagtg tcatgaagga
```

**FIGURE 21 (page 1 of 3)**

1441 aatgggacat ttgagtgtgg cgcgtgcagg tgcaatgaag ggcgtgttgg tagacattgt

1501 gaatgcagca cagatgaagt taacagtgaa gacatggatg cttactgcag gaaagaaaac

1561 agttcagaaa tctgcagtaa caatggagag tgcgtctgcg gacagtgtgt ttgtaggaag

1621 agggataata caaatgaaat ttattctggc aaattctgcg agtgtgataa tttcaactgt

1681 gatagatcca atggcttaat ttgtggagga aatggtgttt gcaagtgtcg tgtgtgtgag

1741 tgcaacccca actacactgg cagtgcatgt gactgttctt tggatactag tacttgtgaa

1801 gccagcaacg gacagatctg caatggccgg ggcatctgcg agtgtggtgt ctgtaagtgt

1861 acagatccga agtttcaagg gcaaacgtgt gagatgtgtc agacctgcct tggtgtctgt

1921 gctgagcata aagaatgtgt tcagtgcaga gccttcaata aaggagaaaa gaaagacaca

1981 tgcacacagg aatgttccta ttttaacatt accaaggtag aaagtcggga caaattaccc

2041 cagccggtcc aacctgatcc tgtgtcccat tgtaaggaga aggatgttga cgactgttgg

2101 ttctatttta cgtattcagt gaatgggaac aacgaggtca tggttcatgt tgtggagaat

2161 ccagagtgtc ccactggtcc agacatcatt ccaattgtag ctggtgtggt tgctggaatt

2221 gttcttattg gccttgcatt actgctgata tggaagcttt taatgataat tcatgacaga

2281 agggagtttg ctaaatttga aaaggagaaa atgaatgcca aatgggacac gtctctctct

2341 gtcgcccagc ctggagtgca gtggtgtgat atcagctcac tgcaacctct gacttccaga

2401 ttccagcaat tctcctgcct cagcctcccg agtacctggg attacagggt gaaaatccta

2461 tttataagag tgccgtaaca actgtggtca atccgaagta tgagggaaaa tgagtactgc

2521 ccgtgcaaat cccacaacac tgaatgcaaa gtagcaattt ccatagtcac agttaggtag

2581 ctttagggca atattgccat ggttttactc atgtgcaggt tttgaaaatg tacaatatgt

2641 ataattttta aaatgtttta ttattttgaa aataatgttg taattcatgc cagggactga

**FIGURE 21 Continued (page 2 of 3)**

2701 caaaagactt gagacaggat ggttactctt gtcagctaag gtcacattgt gcctttttga

2761 ccttttcttc ctggactatt gaaatcaagc ttattggatt aagtgatatt tctatagcga

2821 ttgaaagggc aatagttaaa gtaatgagca tgatgagagt ttctgttaat catgtattaa

2881 aactgatttt tagctttaca aatatgtcag tttgcagtta tgcagaatcc aaagtaaatg

2941 tcctgctagc tagttaagga ttgttttaaa tctgttattt tgctatttgc ctgttagaca

3001 tgactgatga catatctgaa agacaagtat gttgagagtt gctggtgtaa aatacgtttg

3061 aaatagttga tctacaaagg ccatgggaaa aattcagaga gttaggaagg aaaaaccaat

3121 agctttaaaa cctgtgtgcc attttaagag ttacttaatg tttggtaact tttatgcctt

3181 cactttacaa attcaagcct tagataaaag aaccgagcaa ttttctgcta aaaagtcctt

3241 gatttagcac tatttacata caggccatac tttacaaagt atttgctgaa tggggacctt

3301 ttgagttgaa tttattttat tatttttatt ttgtttaatg tctggtgctt tctgtcacct

3361 cttctaatct tttaatgtat ttgtttgcaa ttttggggta agactttttt tatgagtact

3421 ttttctttga agttttagcg gtcaatttgc ctttttaatg aacatgtgaa gttatactgt

3481 ggctatgcaa cagctctcac ctacgcgagt cttactttga gttagtgcca taacagacca

3541 ctgtatgttt acttctcacc atttgagttg cccatcttgt ttcacactag tcacattctt

3601 gttttaagtg cctttagttt taacagttca ctttttacag tgctatttac tgaagttatt

3661 tattaaatat gcctaaaata cttaaatcgg atgtcttgac tctgatgtat tttatcaggt

3721 tgtgtgcatg aaatttttat agattaaaga agttgaggaa aagcaaaaaa aaaa

**FIGURE 21 Continued (page 3 of 3)**

```
   1 gggactgagc atggatttcg gactggccct cctgctggcg gggcttctgg ggctcctcct
  61 cggccagtcc ctccaggtga agcccctgca ggtggagccc ccggagccgg tggtggccgt
 121 ggccttgggc gcctcgcgcc agctcacctg ccgcctggcc tgcgcggacc gcggggcctc
 181 ggtgcagtgg cggggcctgg acaccagcct gggcgcggtg cagtcggaca cgggccgcag
 241 cgtcctcacc gtgcgcaacg cctcgctgtc ggcggccggg acccgcgtgt gcgtgggctc
 301 ctgcgggggc cgcaccttcc agcacaccgt gcagctcctt gtgtacgcct tcccggacca
 361 gctgaccgtc tccccagcag ccctggtgcc tggtgacccg gaggtggcct gtacggccca
 421 caaagtcacg cccgtggacc ccaacgcgct ctccttctcc ctgctcgtcg gggccagga
 481 actggagggg gcgcaagccc tgggcccgga ggtgcaggag gaggaggagg agccccaggg
 541 ggacgaggac gtgctgttca gggtgacaga gcgctggcgg ctgccgcccc tggggacccc
 601 tgtcccgccc gccctctact gccaggccac gatgaggctg cctggcttgg agctcagcca
 661 ccgccaggcc atccccgtcc tgcacagccc gacctccccg gagcctcccg acaccacctc
 721 cccggagtct cccgacacca cctccccgga gtctcccgac accacctccc aggagcctcc
 781 cgacaccacc tccccggagc ctcccgacaa gacctccccg gagcccgccc cccagcaggg
 841 ctccacacac acccccagga gcccaggctc caccaggact cgccgccctg agatctccca
 901 ggctgggccc acgcagggag aagtgatccc aacaggctcg tccaaacctg cgggtgacca
 961 gctgcccgcg gctctgtgga ccagcagtgc ggtgctggga ctgctgctcc tggccttgcc
1021 cacctatcac ctctggaaac gctgccggca cctggctgag gacgacaccc acccaccagc
1081 ttctctgagg cttctgcccc aggtgtcggc ctgggctggg ttaaggggga ccggccaggt
1141 cgggatcagc ccctcctgag tggccagcct ttccccctgt gaaagcaaaa tagcttggac
1201 cccttcaagt tgagaactgg tcagggcaaa cctgcctccc attctactca aagtcatccc
1261 tctgttcaca gagatggatg catgttctga ttgcctcttt ggagaagctc atcagaaact
1321 caaaagaagg ccactgtttg tctcacctac ccatgacctg aagcccctcc ctgagtggtc
1381 cccacctttc tggacggaac cacgtacttt ttacatacat tgattcatgt ctcacgtctc
1441 cctaaaaatg cgtaagacca agctgtgccc tgaccaccct gggcccctgt cgtcaggacc
1501 tcctgaggct ttggcaaata aacctcctaa aatgataaaa aaaaaa
```

# FIGURE 22

```
  1 agattaattc acttccaggc atttcatctt cattcatttt ccaaaggggt accctgagat
 61 cacaaaggat acaaaattat gggcaaggga gttcgagtgt tgaacagcag cgagggtgtt
121 aaggggacca tcttcttcac tcaggaagga aacggtacca ccactgtgac aggaaccgtt
181 tctggcctta agcctggtct ccatggtttc catgtccatg ctcttggtga caccactaac
241 ggttgcatgt ctaccggtcc acatttcaac cctgaaggta aaacccacgg tgcacctgag
301 gatgctaatc gacatgctgg agatctagga aacatcactg ttggggatga tggaactgcc
361 accttcacaa tcactgacag ccagattcct cttgatggac caaactctat tgttggaagg
421 gctgttgttg tccacgcaga acctgatgac ctgggaaagg gaggccatga actcagcctt
481 actactggaa acgcaggtgg ccgtgttgct tgtggtatta ttggtcttca gggctaagct
541 gttgcttttc gaggacgaga gtgatgtaat aaggaggttc ttacctctag acatggctag
601 tttgtgtatt ctttggtgtg tggctgtatt aattgagctt agtggctcga tgcatttggt
661 ttaagacgga agaaaacaga aaatccaaac tttttctatt tcatgaataa cagaggacgt
721 ggttgaaaac gataaaatat tgaatatgaa aaaaaaaaaa aaaaaa
```

# FIGURE 23

```
  1 ctagagatag aattgtgact agaataaagg ctataattat tatagaggtt ttaattgttt
 61 gaattgctca tggtagtgga agtagaagag caatttctag gtcaaataat agaaatgtaa
121 ttgctaccaa gaaaaatttt attgagaatg gtagacgtgc agagcttgta gggtcgaatc
181 cgcattcata tggatttgaa gcatggcagt gtcagcaact ttgtctagag ccttccagaa
241 acagataatg acaaggctct aaaagggttc cagctgaagt ttctgagcgg agcacgctgt
301 gtggctccct aggctgagtt tccaagctgc tggttcatgc cgttgacaaa ctgcaggatg
361 gtgcccgttc gcaggccgct gtcgctgctc ctcaccttct tcctctgcgc ctgtgctgag
421 acaccccca ggtttacacg aactccggtt gatcagacag gggtctctgg aggagttgca
481 tcattcattt gtcaagctac aggagaccca agacctaaaa ttgtctggaa caaaaaagga
541 aagaaagtca gcaaccagag atttgaggta atagaatttg acgatgggtc tggatcagta
601 ctcagaatac agcccttaag gactccacgg gatgaggcca tttatgaatg tgtggcctcg
661 aataatgtgg gagaaatcag tgtgtccaca agactcacag ttttacgtga ggatcagatt
721 cctagagggt tccctacgat tgatatgggc ccgcagttga aggtggtgga acggacccgc
781 accgccacca tgctgtgtgc agccagcggt aatccggatc cagaaatcac ttggtttaaa
841 gatttcttac ctgttgacac aagcaacaac aatggtcgta ttaagcagtt acgatcagaa
901 tctattggag ccctgcagat cgaacagagc gaagaatccg accaaggaaa atacgagtgt
961 gttgccacca acagcgcggg cactcgctac tctgcccctg ccaatttata t
```

# FIGURE 24

1 gctgccggga cgggtccaag atggacggcc gctcaggttc tgcttttacc tgcggcccag
61 agccccattc attgccccgg tgctgagcgg cgccgcgagt cggcccgagg cctccgggga
121 ctgccgtgcc gggcgggaga ccgccatggc gaccctggaa aagctgatga aggccttcga
181 gtccctcaag tccttccagc agcagcagca gcagcagcag cagcagcagc agcagcagca
241 gcagcagcag cagcagcagc aacagccgcc accgccgccg ccgccgccgc cgcctcctca
301 gcttcctcag ccgccgccgc aggcacagcc gctgctgcct cagccgcagc cgcccccgcc
361 gccgcccccg ccgccacccg gcccggctgt ggctgaggag ccgctgcacc gaccaaagaa
421 agaactttca gctaccaaga aagaccgtgt gaatcattgt ctgacaatat gtgaaaacat
481 agtggcacag tctgtcagaa attctccaga atttcagaaa cttctgggca tcgctatgga
541 actttttctg ctgtgcagtg atgacgcaga gtcagatgtc aggatggtgg ctgacgaatg
601 cctcaacaaa gttatcaaag ctttgatgga ttctaatctt ccaaggttac agctcgagct
661 ctataaggaa attaaaaaga atggtgcccc tcggagtttg cgtgctgccc tgtggaggtt
721 tgctgagctg gctcacctgg ttcggcctca gaaatgcagg ccttacctgg tgaaccttct
781 gccgtgcctg actcgaacaa gcaagagacc cgaagaatca gtccaggaga ccttggctgc
841 agctgttccc aaaattatgg cttcttttgg caattttgca aatgacaatg aaattaaggt
901 tttgttaaag gccttcatag cgaacctgaa gtcaagctcc cccaccattc ggcggacagc
961 ggctggatca gcagtgagca tctgccagca ctcaagaagg acacaatatt tctatagttg
1021 gctactaaat gtgctcttag gcttactcgt tcctgtcgag gatgaacact ccactctgct
1081 gattcttggc gtgctgctca ccctgaggta tttggtgccc ttgctgcagc agcaggtcaa
1141 ggacacaagc ctgaaaggca gcttcggagt gacaaggaaa gaaatggaag tctctccttc
1201 tgcagagcag cttgtccagg tttatgaact gacgttacat catacacagc accaagacca
1261 caatgttgtg accggagccc tggagctgtt gcagcagctc ttcagaacgc ctccacccga

**FIGURE 25 (page 1 of 11)**

1321 gcttctgcaa accctgaccg cagtcggggg cattgggcag ctcaccgctg ctaaggagga
1381 gtctggtggc cgaagccgta gtgggagtat tgtggaactt atagctggag ggggttcctc
1441 atgcagccct gtcctttcaa gaaaacaaaa aggcaaagtg ctcttaggag aagaagaagc
1501 cttggaggat gactctgaat cgagatcgga tgtcagcagc tctgccttaa cagcctcagt
1561 gaaggatgag atcagtggag agctggctgc ttcttcaggg gtttccactc cagggtcagc
1621 aggtcatgac atcatcacag aacagccacg gtcacagcac acactgcagg cggactcagt
1681 ggatctggcc agctgtgact tgacaagctc tgccactgat ggggatgagg aggatatctt
1741 gagccacagc tccagccagg tcagcgccgt cccatctgac cctgccatgg acctgaatga
1801 tgggacccag gcctcgtcgc ccatcagcga cagctcccag accaccaccg aagggcctga
1861 ttcagctgtt accccttcag acagttctga aattgtgtta gacggtaccg acaaccagta
1921 tttgggcctg cagattggac agccccagga tgaagatgag gaagccacag gtattcttcc
1981 tgatgaagcc tcggaggcct tcaggaactc ttccatggcc cttcaacagg cacatttatt
2041 gaaaaacatg agtcactgca ggcagccttc tgacagcagt gttgataaat ttgtgttgag
2101 agatgaagct actgaaccgg gtgatcaaga aaacaagcct tgccgcatca aaggtgacat
2161 tggacagtcc actgatgatg actctgcacc tcttgtccat tgtgtccgcc ttttatctgc
2221 ttcgtttttg ctaacagggg gaaaaaatgt gctggttccg gacagggatg tgagggtcag
2281 cgtgaaggcc ctggccctca gctgtgtggg agcagctgtg gccctccacc cggaatcttt
2341 cttcagcaaa ctctataaag ttcctcttga caccacggaa taccctgagg aacagtatgt
2401 ctcagacatc ttgaactaca tcgatcatgg agacccacag gttcgaggag ccactgccat
2461 tctctgtggg accctcatct gctccatcct cagcaggtcc cgcttccacg tgggagattg
2521 gatgggcacc attagaaccc tcacaggaaa tacattttct ttggcggatt gcattccttt
2581 gctgcggaaa acactgaagg atgagtcttc tgttacttgc aagttagctt gtacagctgt

# FIGURE 25 Continued (page 2 of 11)

2641 gaggaactgt gtcatgagtc tctgcagcag cagctacagt gagttaggac tgcagctgat
2701 catcgatgtg ctgactctga ggaacagttc ctattggctg gtgaggacag agcttctgga
2761 aacccttgca gagattgact tcaggctggt gagctttttg gaggcaaaag cagaaaactt
2821 acacagaggg gctcatcatt atacagggct tttaaaactg caagaacgag tgctcaataa
2881 tgttgtcatc catttgcttg gagatgaaga ccccagggtg cgacatgttg ccgcagcatc
2941 actaattagg cttgtcccaa agctgtttta taaatgtgac caaggacaag ctgatccagt
3001 agtggccgtg gcaagagatc aaagcagtgt ttacctgaaa cttctcatgc atgagacgca
3061 gcctccatct catttctccg tcagcacaat aaccagaata tatagaggct ataacctact
3121 accaagcata acagacgtca ctatggaaaa taacctttca agagttattg cagcagtttc
3181 tcatgaacta atcacatcaa ccaccagagc actcacattt ggatgctgtg aagctttgtg
3241 tcttctttcc actgccttcc cagtttgcat ttggagttta ggttggcact gtggagtgcc
3301 tccactgagt gcctcagatg agtctaggaa gagctgtacc gttgggatgg ccacaatgat
3361 tctgaccctg ctctcgtcag cttggttccc attggatctc tcagcccatc aagatgcttt
3421 gattttggcc ggaaacttgc ttgcagccag tgctcccaaa tctctgagaa gttcatgggc
3481 ctctgaagaa gaagccaacc cagcagccac caagcaagag gaggtctggc cagccctggg
3541 ggaccgggcc ctggtgccca tggtggagca gctcttctct cacctgctga aggtgattaa
3601 catttgtgcc cacgtcctgg atgacgtggc tcctggaccc gcaataaagg cagccttgcc
3661 ttctctaaca aaccccccctt ctctaagtcc catccgacga aaggggaagg agaaagaacc
3721 aggagaacaa gcatctgtac cgttgagtcc caagaaaggc agtgaggcca gtgcagcttc
3781 tagacaatct gatacctcag gtcctgttac aacaagtaaa tcctcatcac tggggagttt
3841 ctatcatctt ccttcatacc tcaaactgca tgatgtcctg aaagctacac acgctaacta
3901 caaggtcacg ctggatcttc agaacagcac ggaaaagttt ggagggtttc tccgctcagc

FIGURE 25 Continued (page 3 of 11)

3961 cttggatgtt ctttctcaga tactagagct ggccacactg caggacattg ggaagtgtgt

4021 tgaagagatc ctaggatacc tgaaatcctg ctttagtcga gaaccaatga tggcaactgt

4081 ttgtgttcaa caattgttga agactctctt tggcacaaac ttggcctccc agtttgatgg

4141 cttatcttcc aaccccagca agtcacaagg ccgagcacag cgccttggct cctccagtgt

4201 gaggccaggc ttgtaccact actgcttcat ggccccgtac acccacttca cccaggccct

4261 cgctgacgcc agcctgagga acatggtgca ggcggagcag gagaacgaca cctcgggatg

4321 gtttgatgtc ctccagaaag tgtctaccca gttgaagaca aacctcacga gtgtcacaaa

4381 gaaccgtgca gataagaatg ctattcataa tcacattcgt ttgtttgaac ctcttgttat

4441 aaaagcttta aaacagtaca cgactacaac atgtgtgcag ttacagaagc aggttttaga

4501 tttgctggcg cagctggttc agttacgggt taattactgt cttctggatt cagatcaggt

4561 gtttattggc tttgtattga aacagtttga atacattgaa gtgggccagt tcagggaatc

4621 agaggcaatc attccaaaca tctttttctt cttggtatta ctatcttatg aacgctatca

4681 ttcaaaacag atcattggaa ttcctaaaat cattcagctc tgtgatggca tcatggccag

4741 tggaaggaag gctgtgacac atgccatacc ggctctgcag cccatagtcc acgacctctt

4801 tgtattaaga ggaacaaata aagctgatgc aggaaaagag cttgaaaccc aaaaagaggt

4861 ggtggtgtca atgttactga gactcatcca gtaccatcag gtgttggaga tgttcattct

4921 tgtcctgcag cagtgccaca aggagaatga agacaagtgg aagcgactgt ctcgacagat

4981 agctgacatc atcctcccaa tgttagccaa acagcagatg cacattgact ctcatgaagc

5041 ccttggagtg ttaaatacat tatttgagat tttggcccct tcctccctcc gtccggtaga

5101 catgctttta cggagtatgt tcgtcactcc aaacacaatg gcgtccgtga gcactgttca

5161 actgtggata tcgggaattc tggccatttt gagggttctg atttcccagt caactgaaga

**FIGURE 25 Continued (page 4 of 11)**

```
5221 tattgttctt tctcgtattc aggagctctc cttctctccg tatttaatct cctgtacagt
5281 aattaatagg ttaagagatg gggacagtac ttcaacgcta gaagaacaca gtgaagggaa
5341 acaaataaag aatttgccag aagaaacatt ttcaaggttt ctattacaac tggttggtat
5401 tcttttagaa gacattgtta caaaacagct gaaggtggaa atgagtgagc agcaacatac
5461 tttctattgc caggaactag gcacactgct aatgtgtctg atccacatct tcaagtctgg
5521 aatgttccgg agaatcacag cagctgccac taggctgttc cgcagtgatg gctgtggcgg
5581 cagtttctac accctggaca gcttgaactt gcgggctcgt tccatgatca ccacccaccc
5641 ggccctggtg ctgctctggt gtcagatact gctgcttgtc aaccacaccg actaccgctg
5701 gtgggcagaa gtgcagcaga ccccgaaaag acacagtctg tccagcacaa agttacttag
5761 tccccagatg tctggagaag aggaggattc tgacttggca gccaaacttg gaatgtgcaa
5821 tagagaaata gtacgaagag ggctctcat tctcttctgt gattatgtct gtcagaacct
5881 ccatgactcc gagcacttaa cgtggctcat tgtaaatcac attcaagatc tgatcagcct
5941 ttcccacgag cctccagtac aggacttcat cagtgccgtt catcggaact ctgctgccag
6001 cggcctgttc atccaggcaa ttcagtctcg ttgtgaaaac ctttcaactc caaccatgct
6061 gaagaaaact cttcagtgct tggaggggat ccatctcagc cagtcgggag ctgtgctcac
6121 gctgtatgtg gacaggcttc tgtgcacccc tttccgtgtg ctggctcgca tggtcgacat
6181 ccttgcttgt cgccgggtag aaatgcttct ggctgcaaat ttacagagca gcatggccca
6241 gttgccaatg gaagaactca acagaatcca ggaatacctt cagagcagcg ggctcgctca
6301 gagacaccaa aggctctatt ccctgctgga caggtttcgt ctctccacca tgcaagactc
6361 acttagtccc tctcctccag tctcttccca cccgctggac ggggatgggc acgtgtcact
6421 ggaaacagtg agtccggaca aagactggta cgttcatctt gtcaaatccc agtgttggac
6481 caggtcagat tctgcactgc tggaaggtgc agagctggtg aatcggattc ctgctgaaga
```

# FIGURE 25 Continued (page 5 of 11)

6541 tatgaatgcc ttcatgatga actcggagtt caacctaagc ctgctagctc catgcttaag
6601 cctagggatg agtgaaattt ctggtggcca gaagagtgcc ctttttgaag cagcccgtga
6661 ggtgactctg gcccgtgtga gcggcaccgt gcagcagctc cctgctgtcc atcatgtctt
6721 ccagcccgag ctgcctgcag agccggcggc ctactggagc aagttgaatg atctgtttgg
6781 ggatgctgca ctgtatcagt ccctgcccac tctggcccgg gccctggcac agtacctggt
6841 ggtggtctcc aaactgccca gtcatttgca ccttcctcct gagaaagaga aggacattgt
6901 gaaattcgtg gtggcaaccc ttgaggccct gtcctggcat ttgatccatg agcagatccc
6961 gctgagtctg gatctccagg cagggctgga ctgctgctgc ctggccctgc agctgcctgg
7021 cctctggagc gtggtctcct ccacagagtt tgtgacccac gcctgctccc tcatctactg
7081 tgtgcacttc atcctggagg ccgttgcagt gcagcctgga gagcagcttc ttagtccaga
7141 aagaaggaca aataccccaa aagccatcag cgaggaggag gaggaagtag atccaaacac
7201 acagaatcct aagtatatca ctgcagcctg tgagatggtg gcagaaatgg tggagtctct
7261 gcagtcggtg ttggccttgg gtcataaaag gaatagcggc gtgccggcgt ttctcacgcc
7321 attgctaagg aacatcatca tcagcctggc ccgcctgccc cttgtcaaca gctacacacg
7381 tgtgccccca ctggtgtgga agcttggatg gtcacccaaa ccgggagggg attttggcac
7441 agcattccct gagatccccg tggagttcct ccaggaaaag gaagtcttta aggagttcat
7501 ctaccgcatc aacacactag gctggaccag tcgtactcag tttgaagaaa cttgggccac
7561 cctccttggt gtcctggtga cgcagcccct cgtgatggag caggaggaga gcccaccaga
7621 agaagacaca gagaggaccc agatcaacgt cctggccgtg caggccatca cctcactggt
7681 gctcagtgca atgactgtgc ctgtggccgg caacccagct gtaagctgct tggagcagca
7741 gccccggaac aagcctctga aagctctcga caccaggttt gggaggaagc tgagcattat
7801 cagagggatt gtggagcaag agattcaagc aatggtttca aagagagaga atattgccac

# FIGURE 25 Continued (page 6 of 11)

```
7861 ccatcattta tatcaggcat gggatcctgt cccttctctg tctccggcta ctacaggtgc
7921 cctcatcagc cacgagaagc tgctgctaca gatcaacccc gagcgggagc tggggagcat
7981 gagctacaaa ctcggccagg tgtccataca ctccgtgtgg ctggggaaca gcatcacacc
8041 cctgagggag gaggaatggg acgaggaaga ggaggaggag gccgacgccc ctgcaccttc
8101 gtcaccaccc acgtctccag tcaactccag gaaacaccgg gctggagttg acatccactc
8161 ctgttcgcag tttttgcttg agttgtacag ccgctggatc ctgccgtcca gctcagccag
8221 gaggaccccg gccatcctga tcagtgaggt ggtcagatcc cttctagtgg tctcagactt
8281 gttcaccgag cgcaaccagt ttgagctgat gtatgtgacg ctgacagaac tgcgaagggt
8341 gcacccttca gaagacgaga tcctcgctca gtacctggtg cctgccacct gcaaggcagc
8401 tgccgtcctt gggatggaca aggccgtggc ggagcctgtc agccgcctgc tggagagcac
8461 gctcaggagc agccacctgc ccagcagggt tggagccctg cacggcgtcc tctatgtgct
8521 ggagtgcgac ctgctggacg acactgccaa gcagctcatc ccggtcatca gcgactatct
8581 cctctccaac ctgaaaggga tcgcccactg cgtgaacatt cacagccagc agcacgtact
8641 ggtcatgtgt gccactgcgt tttacctcat tgagaactat cctctggacg tagggccgga
8701 attttcagca tcaataatac agatgtgtgg ggtgatgctg tctggaagtg aggagtccac
8761 cccctccatc atttaccact gtgccctcag aggcctggag cgcctcctgc tctctgagca
8821 gctctcccgc ctggatgcag aatcgctggt caagctgagt gtggacagag tgaacgtgca
8881 cagcccgcac cgggccatgg cggctctggg cctgatgctc acctgcatgt acacaggaaa
8941 ggagaaagtc agtccgggta gaacttcaga ccctaatcct gcagcccccg acagcgagtc
9001 agtgattgtt gctatggagc gggtatctgt tctttttgat aggatcagga aaggctttcc
9061 ttgtgaagcc agagtggtgg ccaggatcct gccccagttt ctagacgact tcttcccacc
9121 ccaggacatc atgaacaaag tcatcggaga gtttctgtcc aaccagcagc catacccca
9181 gttcatggcc accgtggtgt ataaggtgtt tcagactctg cacagcaccg ggcagtcgtc
```

**FIGURE 25 Continued (page 7 of 11)**

```
 9241 catggtccgg gactgggtca tgctgtccct ctccaacttc acgcagaggg ccccggtcgc
 9301 catggccacg tggagcctct cctgcttctt tgtcagcgcg tccaccagcc cgtgggtcgc
 9361 ggcgatcctc ccacatgtca tcagcaggat gggcaagctg gagcaggtgg acgtgaacct
 9421 tttctgcctg gtcgccacag acttctacag acaccagata gaggaggagc tcgaccgcag
 9481 ggccttccag tctgtgcttg aggtggttgc agccccagga agcccatatc accggctgct
 9541 gacttgttta cgaaatgtcc acaaggtcac cacctgctga gcgccatggt gggagagact
 9601 gtgaggcggc agctggggcc ggagcctttg gaagtctgcg cccttgtgcc ctgcctccac
 9661 cgagccagct tggtccctat gggcttccgc acatgccgcg ggcggccagg caacgtgcgt
 9721 gtctctgcca tgtggcagaa gtgctctttg tggcagtggc caggcaggga gtgtctgcag
 9781 tcctggtggg gctgagcctg aggccttcca gaaagcagga gcagctgtgc tgcaccccat
 9841 gtgggtgacc aggtcctttc tcctgatagt cacctgctgg ttgttgccag gttgcagctg
 9901 ctcttgcatc tgggccagaa gtcctccctc ctgcaggctg gctgttggcc cctctgctgt
 9961 cctgcagtag aaggtgccgt gagcaggctt tgggaacact ggcctgggtc tccctggtgg
10021 ggtgtgcatg ccacgccccg tgtctggatg cacagatgcc atggcctgtg ctgggccagt
10081 ggctgggggt gctagacacc cggcaccatt ctcccttctc tcttttcttc tcaggattta
10141 aaatttaatt atatcagtaa agagattaat tttaacgtaa ctctttctat gcccgtgtaa
10201 agtatgtgaa tcgcaaggcc tgtgctgcat gcgacagcgt ccggggtggt ggacagggcc
10261 cccggccacg ctccctctcc tgtagccact ggcatagccc tcctgagcac ccgctgacat
10321 ttccgttgta catgttcctg tttatgcatt cacaaggtga ctgggatgta gagaggcgtt
10381 agtgggcagg tggccacagc aggactgagg acaggccccc attatcctag gggtgcgctc
10441 acctgcagcc cctcctcctc gggcacagac gactgtcgtt ctccacccac cagtcaggga
10501 cagcagcctc cctgtcactc agctgagaag gccagccctc cctggctgtg agcagcctcc
```

**FIGURE 25 Continued (page 8 of 11)**

10561 actgtgtcca gagacatggg cctcccactc ctgttccttg ctagccctgg ggtggcgtct
10621 gcctaggagc tggctggcag gtgttgggac ctgctgctcc atggatgcat gccctaagag
10681 tgtcactgag ctgtgttttg tctgagcctc tctcggtcaa cagcaaagct tggtgtcttg
10741 gcactgttag tgacagagcc cagcatccct tctgcccccg ttccagctga catcttgcac
10801 ggtgacccct tttagtcagg agagtgcaga tctgtgctca tcggagactg ccccacggcc
10861 ctgtcagagc cgccactcct atccccaggc caggtccctg gaccagcctc ctgtttgcag
10921 gcccagagga gccaagtcat taaaatggaa gtggattctg gatggccggg ctgctgctga
10981 tgtaggagct ggatttggga gctctgcttg ccgactggct gtgagacgag gcaggggctc
11041 tgcttcctca gccctagagg cgagccaggc aaggttggcg actgtcatgt ggcttggttt
11101 ggtcatgccc gtcgatgttt tgggtattga atgtggtaag tggaggaaat gttggaactc
11161 tgtgcaggtg ctgccttgag acccccaagc ttccacctgt ccctctccta tgtggcagct
11221 ggggagcagc tgagatgtgg acttgtatgc tgcccacata cgtgaggggg agctgaaagg
11281 gagcccctcc tctgagcagc ctctgccagg cctgtatgag gcttttccca ccagctccca
11341 acagaggcct cccccagcca ggaccacctc gtcctcgtgg cggggcagca ggagcggtag
11401 aaaggggtcc gatgtttgag gaggccctta agggaagcta ctgaattata acacgtaaga
11461 aaatcaccat tccgtattgg ttgggggctc ctgtttctca tcctagcttt ttcctggaaa
11521 gcccgctaga aggtttggga acgaggggaa agttctcaga actgttggct gctccccacc
11581 cgcctcccgc ctcccccgca ggttatgtca gcagctctga gacagcagta tcacaggcca
11641 gatgttgttc ctggctagat gtttacattt gtaagaaata acactgtgaa tgtaaaacag
11701 agccattccc ttggaatgca tatcgctggg ctcaacatag agtttgtctt cctcttgttt
11761 acgacgtgat ctaaaccagt ccttagcaag ggctcagaa caccccgctc tggcagtagg

FIGURE 25 Continued (page 9 of 11)

11821 tgtcccccac ccccaaagac ctgcctgtgt gctccggaga tgaatatgag ctcattagta
11881 aaaatgactt cacccacgca tatacataaa gtatccatgc atgtgcatat agacacatct
11941 ataattttac acacacacct ctcaagacgg agatgcatgg cctctaagag tgcccgtgtc
12001 ggttcttcct ggaagttgac tttccttaga cccgccaggt caagttagcc gcgtgacgga
12061 catccaggcg tgggacgtgg tcagggcagg gctcattcat tgcccactag gatcccactg
12121 gcgaagatgg tctccatatc agctctctgc agaagggagg aagactttat catgttccta
12181 aaaatctgtg gcaagcaccc atcgtattat ccaaattttg ttgcaaatgt gattaatttg
12241 gttgtcaagt tttgggggtg ggctgtgggg agattgcttt tgttttcctg ctggtaatat
12301 cgggaaagat tttaatgaaa ccagggtaga attgtttggc aatgcactga agcgtgtttc
12361 tttcccaaaa tgtgcctccc ttccgctgcg ggcccagctg agtctatgta ggtgatgttt
12421 ccagctgcca agtgctcttt gttactgtcc accctcattt ctgccagcgc atgtgtcctt
12481 tcaaggggaa aatgtgaagc tgaaccccct ccagacaccc agaatgtagc atctgagaag
12541 gccctgtgcc ctaaaggaca cccctcgccc ccatcttcat ggagggggtc atttcagagc
12601 cctcggagcc aatgaacagc tcctcctctt ggagctgaga tgagccccac gtggagctcg
12661 ggacggatag tagacagcaa taactcggtg tgtggccgcc tggcaggtgg aacttcctcc
12721 cgttgcgggg tggagtgagg ttagttctgt gtgtctggtg ggtggagtca ggcttctctt
12781 gctacctgtg agcatccttc ccagcagaca tcctcatcgg gctttgtccc tcccccgctt
12841 cctccctctg cggggaggac ccgggaccac agctgctggc cagggtagac ttggagctgt
12901 cctccagagg ggtcacgtgt aggagtgaga agaaggaaga tcttgagagc tgctgaggga
12961 ccttggagag ctcaggatgg ctcagacgag gacactcgct tgccgggcct gggcctcctg
13021 ggaaggaggg agctgctcag aatgccgcat gacaactgaa ggcaacctgg aaggttcagg
13081 ggccgctctt cccccatgtg cctgtcacgc tctggtgcag tcaaaggaac gccttcccct

# FIGURE 25 Continued (page 10 of 11)

13141 cagttgtttc taagagcaga gtctcccgct gcaatctggg tggtaactgc cagccttgga

13201 ggatcgtggc caacgtggac ctgcctacgg agggtgggct ctgacccaag tggggcctcc

13261 ttgtccaggt ctcactgctt tgcaccgtgg tcagagggac tgtcagctga gcttgagctc

13321 ccctggagcc agcagggctg tgatgggcga gtcccggagc cccacccaga cctgaatgct

13381 tctgagagca aagggaagga ctgacgagag atgtatattt aattttttaa ctgctgcaaa

13441 cattgtacat ccaaattaaa ggaaaaaaat ggaaaccatc a

**FIGURE 25 Continued (Page 11 of 11)**

```
   1 atccacccgc ctctgcctcc caaagtgcta ggattacagg catgagccac catgtctggc
  61 caggaaaaat gggaagtttt aaatgctttt ccagtagcac tggagacagg gtgagatgtc
 121 tgctctcatc atttatattg tcacggtgct gggtttctat acagtcctct caggcaagaa
 181 gagaaataaa aggtggctgg gcgtggtgac tcaccctgta atcccagcac tttgggaggc
 241 cgaggtggga ggatcccttc agctcaggag tttgagacct tcaagaccag cctggacaac
 301 acagtgagat cccatctcta aaaaaaaaaa aaatacaaaa attagccggg tatggtggct
 361 tgcacctgta gtcccagata cttaggaggc tgatgtggga ggctcacctg agcctggggg
 421 gttggaactg cagttagctg agatcatgcc actgcactcc agcctgggtg atagagcaag
 481 acgctgtctc aaaaaaaaaa aaaaaaaaaa aaaggctggg cacaatgact cacacctgta
 541 atccctgcac tttgggaggc caaggcgggc agatcaactg aggtcaggag ttcaagacca
 601 acctggccaa aatggtgaaa ccctgtcttt actaaaaata caaaaattag ccgggcgtga
 661 tggcgggcac ctgtaattcc agctactcag gaggctaagg caggagaatc actcgaaccc
 721 gggaggtgga ggttgcagtg agccaagatt gcaccactgc actccagctt gggtgacagg
 781 gtgagatcct gtctaaaaaa aaaaaaaaaa atccagtcag gaacaggaat gctaaggaat
 841 caagagcagc aacaacaagc tagcagaagt aacaactaac gaacaagttc ataaagatca
 901 cagggaggac cagaagacct gcattcactg tcaattttat ttatacttat taacaataaa
 961 tggtctgaaa ataaaattaa gaagacagct tcattcacaa ttgcatagaa aaataaaata
1021 ctttagatta aaattaagaa aataaatgca aaccttgtac attaaaatct acaaaacatt
1081 gctgagagaa agcaaagaca gcctaaaaaa atggggagtg atcctatgtt cacagattag
1141 aagattcaat attgtggccg cgtgtggtga ctcatgcctg aaatcccagc actttgggag
1201 gccgggggcg gggggggggg tggattatga ggtcaggagt tcgagaccag cctggccaag
1261 agaccagcct ggccaatatg gtgaaaccct gtctccacta aaaatacaaa aattagccgg
1321 gcatgatggt gggcgcctat agtcccagct actcgggagg ctgaggaagg agaatggcgt
1381 gaacccagga agcagagctt gcagtgagcc gagattgcag cactgcactc cagcctgggc
1441 aacagagtga gactctgtct caaaaaaaaa tatatatata tatatattat ttttattttt
```

**FIGURE 26 (Page 1 of 24)**

1501 agtagagaca gggtcttacc atgttggtca ggctggtctc gaactcctga cctcaggtga

1561 tcctcccatc tcggcctccc aaagtgctgg gatgacaggt gggagccgcc gcgcccggct

1621 gggaggtgtt ctttctagac ctcacctggg agtcacgcac cattacctct accacgttcc

1681 atttgtaagt gcaggccatg tatgcctgga gggaaatcaa tcttctgccg aacagggtgg

1741 tgttcaaagc accaccggct ccaccacact ctgcctttta ttctgcattc tgtttcttga

1801 gaccacgcgc cgctacgtgg aatggtctca ggagctcatg tgtttgtgct ccatgaagtc

1861 agaaagtcca gcctttgcac tgccacatac ccacccttag aacagcgtct ggtacacggt

1921 aggtgctcag tgaatgtgcc tagtggagta aacgtgcggt gcggtgctgc ctgcggtcgg

1981 atctgtgggg atcctgtgat ggggaagacg gctaccagga aaggtggaat ttggaaagtc

2041 aggacaggga gagaggaggg actttgtgga ggcaggaagg tatgggagcc agtccagcac

2101 aagggctggc gggaaagcct cgtgggtgtc agagactata cctctgtgag ggtccctggg

2161 ctcccccagg tcagggcaga ggtcctgacc ccagtctagc tctttagcgg gggccaggcc

2221 cgagatggcc agctccgttt cccctgcgtg gcctggcagc ccctccagga gctggcacgg

2281 gaagcaggcc tgtcctccac gcccttggcc ggccttggct gcgatgggtg gagacgcttc

2341 tgcccgcccc acccctgtct gtctgcctcc tcctctcaag caggcttcct ctcgacagaa

2401 atgtgtctgc aggtggctct tgagccccag ttctcaggga ttcacctgca gaggatgtag

2461 agccggggtc ccttccctgc ttctgatctg cccaaaatcc tagggagagc cctgattggc

2521 aacccctcgg ccaatggact gaggccaggg cgcgtctgca cccgggttga cagccctgac

2581 tagaaccgcg aggctggggt cagggaggag cagccgcctc ccgccggggc cgcagctgtc

2641 agagtgggca aggggatctc cacagaggga gactgtccca cagtggcccg gggtagaaca

2701 gcgcttccct ccccacccag cgcctgactg ctggccccgg gcccatccca gggcagtgcc

2761 ccaggtctct ggtcctcccc acagcctttg tctcaccagg tctcagaggg gtccgagtcc

2821 aggctcatct gttgtggtac cactgccccc tccctcatgg ttgtggaaga agcccacggg

2881 gggaaaatgc agagcagaat ccaggaaaga ctcgctcaca ggaggagctg tggcctggag

2941 acgcggcctc caccccatct ccatcatgga caacctgcca aggggtcttg aacagaagct

# FIGURE 26 Continued (Page 2 of 24)

3001 tggggacgca catacggtcc ggtggggcag tgacccactg gcagaccagc tccctgagta
3061 gaaaagaaaa agccctggtt tgtggggttt gatgattccg tggcagcaga gataatccca
3121 ctgtggccaa tttcgaagcc tgactgtaac agctgctggc aggtgtctga acatttagcg
3181 atcagctctc cagggtcagc gggagtcgct ccagcacccc acggggtggg ggtgggtggg
3241 tggagccagg gcctctgctg cagcctccaa ccccttttctt ccacgctgat gaggcgctgt
3301 ctgggctcag gtctaccccc acgcagctgc caggctctgg cctgcctgag gcggatactt
3361 tttttttct tttttttgat ggagtgttgc tctgtcgccc gggctggagt gcaatggtgc
3421 gatcttggct cactgcaaca tccgcctccc agattcaagc gattctcctg cctcagtctc
3481 ccaagtagct gggattacaa atgcctgccg ccacgcccgg ctaattttg tatttttagt
3541 agagacaggg tttcaccatg ttggccaggc tggtctcgaa ctcctgacct taggtgatcc
3601 acctgccctt gcctcccaaa gtattgagat tacaggcatg agccactgtg ccctgccgag
3661 gtggaagttt ggagatgggc cacaaactcc tggagatagg gccagctcag tccccctgag
3721 cacccacaca caccctcctg agagccaaca gaaggagtga gggccccgag gcggatgacc
3781 cgtgtgcctc aacccgaacg gggagaggcg gggtctgcag cagggtacgg gcaggtgatc
3841 ccccaaggaa agattttcct gtattgagag agaaggggcc aagaggagga gcttgtcaaa
3901 caccacagcc cctccccctc ctctcagctc caggggtcc ctggtgccag tgttcggctg
3961 atggagagaa cggcaagcgg gagagagagt gtgacccctg tgggcacatg acttcccttg
4021 ctgcactgct gcacatagca gaggtgtggt gacgaccctg ttttgtccca ttgggggcgt
4081 ttgctgttag gtctgcagaa tcctcagttg ctattggaaa tggtgacatc actggcaggg
4141 gcggagcttc agccatcctt caagttaggg aggggcacgc acactccagg ggtggagggg
4201 gacaaagaca gggtggtgtg gaccagaggg atgggtaagg ctctggaaaa gggggcgctg
4261 ggagcgcatt gcgagggggc tggagaggga gagaggagcg gaagctgagg gtgtgaaacg
4321 gctggccccg aacacacctc gcggcgctcc agtgattcct ggtgtccgac ctcagcccca
4381 gtcagtgcgg gtccagtttc caggctctcg cggaaggcct ggctgagcac atgcggcagc
4441 cacggtcacc ctccctattc ctcttagccc gaggaggggg gtcccaagtt acatggccac
4501 gcagatgggg cctctccctc atttctgaac cttgtgggga ggggaacctt gaagggagcg

**FIGURE 26 Continued (Page 3 of 24)**

4561 ccccccagag ccatggctta gggcctcccc caccccctctg gagctccagt ctgcaagagt

4621 caggagccga aatatcgctg actgtgggtg acgactcttg cgcgcacaca cacatacaag

4681 cgggcacgac gcgttcggtc ctattaaaag gcacgcaagg gtgcgggctg cacgcggtga

4741 cacggacccc tctaacgttt ccaaactgag ctccctgcag gtccccgaca gcacaggccc

4801 ctgtcccagg acccctccag gcacgcgctc acacgcacac gcgcgctccc cggctcacgc

4861 gcgctccgac acacacgctc acgcgaacgc aggcgcacgc tctggcgcgg gaggcgcccc

4921 cttcgcctcc gtgttgggaa gcgggggcgg cgggaggggc aggagacgtt ggccccgctc

4981 gcgtttctgc agctgctgca gtcgccgcag cgtccggacc ggaaccagcg ccgtccgcgg

5041 agccgccgcc gccgccgccg ggccctttcc aagccgggcg ctcggagctg tgcccggccc

5101 cgcttcagca ccgcggacag cgccggccgc gtggggctga gccccgagcc cccgcgcacg

5161 cttcagcgcc ccttccctcg gccgacgtcc cgggaccgcc gctccggggg agacgtggcg

5221 tccgcagccc gcggggccgg gcgagcgcag gacggcccgg aagccccgcg ggggatgcgc

5281 cgagggcccc gcgttcgcgc cgcgcagagc caggcccgcg gcccgagccc atgagcacca

5341 tgcgcctgct gacgctcgcc ctgctgttct cctgctccgt cgcccgtgcc gcgtgcgacc

5401 ccaagatcgt caacattggc gcggtgctga gcacgcggaa gcacgagcag atgttccgcg

5461 aggccgtgaa ccaggccaac aagcggcacg gctcctggaa gattcagctc aatgccacct

5521 ccgtcacgca caagcccaac gccatccaga tggctctgtc ggtgtgcgag gacctcatct

5581 ccagccaggt gccctccccc acctccgcca cccacctccc ctctcctcca tcctgcaacc

5641 ccacaccccc agtttcattc catcctttcc gtgccccctt cctccctgta agacaccacc

5701 ccagagtcag ctggctgctt ccgggaggcc tcgtctcact aggaaccaaa caccagggtc

5761 tgctggctcc cctatcttgg cctgagacca gtcacctgcc accttggctg gtcctcagag

5821 ggcccctggg gctccaggcc ctgactggtg tgtgtagacg tggggctgga gtgtgtcagt

5881 gtgggggtgg gcattccggg taagagagta gaagcgcctg tccagctaca tgcccgccct

5941 gcagagcttt aaacaggacg gggcctgggg ccatctttgt ttctgcttcc aggttctcct

6001 gccctttctt tcgtcccttc cccctaccga tgggtccgcc tgggaagaga aatggctcag

6061 gtgccacggc aggacgcttt gtgggggtgg gagtgggggt gcacacgcga gaggcatcag

**FIGURE 26 Continued (Page 4 of 24)**

6121 ggcatgggag ctgtcggcag ccagcgctgc gggggaggac gtggctcctg ggattttgcc

6181 tgtcggagct gtccgcccct gggccgagcg cctgctgaat tccaatgagg ctgcaaggat

6241 ctgcaatgca gccctttatg taagaggcaa gacagacatc cagcctagca ccgctcacac

6301 gtgcctacct gatggacaca ccacatctgt ggacacacat gctcacactc acaccaaatg

6361 ttacattagc acacactcat gcacctcagc atcacacaat caatttcata tgctcatctg

6421 cacacatgca gatccattga cacctgctca tgtgccacac acggcttggc atgcattccc

6481 agaggcacgt gcaaacatgc acatttacac acatggttcc agtcattcac acgcatgtac

6541 acgaacagac atgccagggc atgtgatgca cataaccata ccctagcaca cgcgtgaaca

6601 cctgcatggt cacacacgga cctacgggtc tttgccaagc acctctgggt gcaggctgga

6661 agcaagagct gggggaggga gaaccacttc aaacagctgc agctgcaggg cccacaccag

6721 agttttctca gaaatcctcc ctccccactt cacaagccac ccccgtgccc cagcccagga

6781 caccatggga tgggactggg gggatgcatc tgtagccagt ggctgcagtc acatattcca

6841 tctgggactg gggagggaca cggaaggtgg actcaggaaa tccaggaggg gccattcctg

6901 gggaattgct tcaactcacg cccatgttgc tgtctgtctg tgggcatggc ctctgcagca

6961 aaggcaatgc ctgcagctac cactcacgga acacaccccc ggccaggtac tgtcctgcca

7021 cgtggggcca tgcagtgcac gcccccattc gccaaagctc taagaggcac aggcagactt

7081 ggggacagac gcaggtcctt gctgtgtgaa ggtggtgtgg accaccagct gcctgcctcc

7141 ctgccttggg aggctgggga gagagggagg catcagctcc agggggctga gccgctggct

7201 ttagatctgc cccatggggc cttggtcatg ggcaggaagg ctgggctgca cccccaatgc

7261 ctcccttccc ttccttgagg atgaggccag cactcaaagt aagggcttgg tgttgttcag

7321 acagagcccg tcacaggccc tgcccctgga gacaccagca aaagggatct cggcctcttt

7381 ggcagctcct agctgcttcc ccctgaagtc cggtaccacc cttcagagct gcccgccctg

7441 tctcgggatg tgggctggcc ccacccctgg cccatcagga aggacgggtg ggttctgaga

7501 atcaaggcca tcatgatgca ggaccagcca tcctccccgg tccaccttgg gtgcgtcccg

7561 tgctccaggc ccccaggaca tcccaagggc agtccttcac ctggcccttg agcacaacac

7621 ctgcagggcc ctagtcagca gtgtgagagg agtgagggga ggtccgggtg ggtctccct

FIGURE 26 Continued (Page 5 of 24)

7681 cccctgccct gtgggcatgt gtgcatctgg gcctgggcat gtagcatgta cccgaatcat

7741 gcccccagcc ccccttagcc tgctgggttc agcccctgct gcttccagat ctcagcctct

7801 aacccagtgc ctggctcacc cctgactcaa gctgatcatg tctcctgtgt ccacaggtct

7861 acgccatcct agttagccat ccacctaccc ccaacgacca cttcactccc acccctgtct

7921 cctacacagc cggcttctac cgcatacccg tgctggggct gaccacccgc atgtccatct

7981 actcggacaa ggtaagcctg actgccagac caggccttcc ggccctcggc cccagggcac

8041 agcctggcca ctccaggagc agcgggccga cccgctcaca tggaactcac acaccacaaa

8101 cagccacaca gctcccccac attcatgcac gtccacacgc tctcacgtgt ccaactcaca

8161 catctgcaaa catgctcaca tgcacactca tgtgctctta cacacacaat acacactctc

8221 ttgcacatag agggctcacg tggagcccag cacgtgcccc cagcccagag caggccaaag

8281 ggagggggca cacatcacac actcacacat cacacacaca tcacacactc acacattcat

8341 acagcaccca cacgctacac tgctatgctc accctcccca cacatgaaca ctgacacacc

8401 catggattcg cacaaagtca cacacactca ctggcacagg caccagtgac accccctcag

8461 gacgagaggg cccgtgggct aggagaaggg atggctggga ggctttctag acaggtggac

8521 tttgaagggg agtttggaga gctgggggtt gctccaggag gaaaggggtg tgcacgcagc

8581 cagggtggtg gggccagcct ttcccactgc aggcatgggt ggagagcaat gtctgtggtt

8641 gcagctcagg gtcgggggcg ctggcctggg ggcttccagc ctctagggct gagggcacct

8701 tggcttagcc tcctgcagac cctcctggcc cacaggctat gaggagggct tctgtccaat

8761 cctggaacat cagctggaag agaggagggt catccagtca gttttgcagg aatctccaag

8821 ccagagagcc atgggggctt gctctaggtc acacagccct ccgtctaccc aggatgcaaa

8881 ctgggcactg agaggctgac caacctgggg ccactggcag acagacctgc agggccactt

8941 ggcaggggac atccagtttg gtgccagcgc tgaggagcca gagggctggg ctgtgcagcc

9001 aggcttctgg gtcccccacc ttctccaaat tcctcctgcc cagagtccac agtccttggt

9061 aacactgcct taaagcacag gggtcgcccc agccaggccc aggctcttct gggaggatgg

9121 aaggccccag aggcaggaac tgagacagag gctggaacag ccaccttcct gaggctctga

9181 aagccctggc gtgccccctc ggcacccaaa ctgctcctcc caggtgactt cacctctggc

**FIGURE 26 Continued (Page 6 of 24)**

9241 catgggaagg tgggggtctc attcctgtgc cctcaggcac gacctccttc gcctctctgg
9301 gcaccagttt cctcacatgt gaaggagaga agatggcctt acccaggaaa gcagccagtg
9361 gtcaaatgaa aggcgggga aacggatgcc cctggcccag agcaggccaa agggaggggg
9421 cacagctcac agctgcaccc tggccttacc acagtctcta cactacaacc aacctgtgcc
9481 caaaacatga accggcaagg ccaggtcaga gctagtccaa gacctcaagc acagcctgcc
9541 ttgccaccac gtcaccaggt ggatagacag aagcagggga catttttgca ccccaaggca
9601 ctgccccagg ccacaaagag ggagcaggtg aaaaataacc tggaagcctc agaggaccac
9661 aagatcagca agagtccaca gggacactga aggaaccagg gcttacctgg acagacacag
9721 agaactgagg cagaggggg cagagcctgc tccactcccg gccatgccac ggcactccgt
9781 ggcagcttga agccaggaaa agcaagccag ggcaagcaag caccacgctc tcgcctgggg
9841 agatgaggcc tttagcccca agagtgaatt cttcttcata catagagttg tttaaatttg
9901 ggaggactct atgggcagcc ccaggggat cttcgaggcg ctatgtgtca tcaagaattt
9961 cctgagctca gcttgtccaa aggtggtggg ctgcagggga agaggtgagc tcaccccagg
10021 cacaattcca cagaaaccca cgtcccttag ggtgctatgg ggccaacact aaacctcctc
10081 catttccgag attatatgtg ggaggagagg ccggggtggg agagaggttc ccagggtcta
10141 aaaagtgtcc ccaggatggt ggggacaggg gtgggaaaaa ggaggggtcc cagtgtctag
10201 aaagtgtccc caggttggcc gggcgcggtg gctcacgcct gtaatcccag cactttggga
10261 ggccgaggcg ggcggatcac gaggtcagga gatcgagacc atcctggcta atacggtgaa
10321 accccatctc cactaaaaat acaaaaaaat tagccgggcg tggtggcggg cgcctgtagt
10381 ctcagctact tgggaggctg aggcaggaga atggtgtgaa cccaggaggc ggagcctgca
10441 gtgagccgag attgcactcc agcctgggta acagtgcgag actgtttaaa aaaaaaaaaa
10501 agtgtcccca gggtggtggg gacaggggtg ggagacagga gggggtccca gggtctagaa
10561 agtgtcccca ggggtgtggg gacaggagtg agaggaaggg ggtcccaggg tccagaaagt
10621 gtccccaggg tggtagggac aggggtggga gacacgaggg ggtcccaggg tctagaaagt
10681 gtccccaggg tggcagggat gggatgggag acacgagggg gtcccagagt ctagaaagtg
10741 tccccagggg tgtggggacc ggggtgagag gaagggggtc ccagggtcca gaaagtgtcc

**FIGURE 26 Continued (Page 7 of 24)**

10801 ccagaggggt ggggacagga gtgagaggaa gggggtccca gggtccagaa agtgtcccca

10861 gaggggtggg gacaggagtg agaggaaggg ggtcccaggg tccagaaagt gtccccagag

10921 gggtggggac cggggtgaga ggaagggggt cccagggtct agaaagtgtc cccagagggg

10981 tggggaccgg ggtgagagga agggggtccc agggtctaga aagtgtcccc aggggtgtgg

11041 ggaccggggt gagaggaagg gggtcccagg gtccagaaag tgtccccaga ggggtgggga

11101 caggagtgag aggaaggggg tcccagggtc cagaaagtgt ccccagaggg gtggggacag

11161 gagtgagagg aagggggtcc cagggtccag aaagtgtccc cagggtggta gggacagggg

11221 tgggagacac gagggggtcc cagggtctag aaagtgtccc caggtggca gggatgggat

11281 gggagacacg agggggtccc agagtctaga aagtgtcccc agcggggtgg ggacaggggt

11341 gggagacacg agggggtccc agggtctaga aagtgtcccc agggtggtag ggacggggtg

11401 ggagacacga gggggtccca gggtctagaa cgtgtcccca tgggggtggg gacaggggtg

11461 ggaggagggg ggttcccagc ctccggcggg tgttccggca gtgggaggcg ggtgggaggg

11521 cgggtccccg cgggtccacc tcagcccgcc gtgcccccgc ctcccgcaga gcatccacct

11581 gagcttcctg cgcaccgtgc cgccctactc ccaccagtcc agcgtgtggt ttgagatgat

11641 gcgtgtctac agctggaacc acatcatcct gctggtcagc gacgaccacg agggccgggc

11701 ggctcagaaa cgcctggaga cgctgctgga ggagcgtgag tccaaggtga gggtcggcgc

11761 cgcgggtggg cgcctggcgg agccgaggtg caggacgggc cgccttgtgt ctgtggctcc

11821 gtgtgtgaca ccctcttctt tccatcgtgc atggtcagca ccaccacgtc tggcgagcgc

11881 ccgccccagc ctgtcctcgg ctcatttcac tcgcttttgc cattagtcga aatctccttc

11941 gtgtcagtcc ctgcggggcg agggccagac cacctggagc tccgcaaaca cccctgcccc

12001 gcgctgccga gcgccctcgt cccctcctcc tgcccatgcc cctcgctccc tggaggcccc

12061 agccgggctt gggactcgtc acccttcccg cccaccctgt cctgagtccc cagcagcctc

12121 cctctgggca aggtctcccc tgtacacgac ccccacatac cgcccctgca ggcctgtccc

12181 ctcctggctg ggcccattcc ctgtcctccc ccgcgtggcc tcccctgaag ctctgcacct

12241 catggctcag caaagccctg tccacagaca cctgcccccc agcctggacc gcccctgtgg

12301 gctccactcc cctccttgcc cccaccacag ggctccctct gcactcctca ccaagaccca

**FIGURE 26 Continued (Page 8 of 24)**

12361 ttagccacct ggttctagga cacactgacc cccaacacag ccaggcgtcc actctgtggg

12421 gctgcagaga gatcagagct gggatttggg ggggtccgag cgaccccttt ccccttcctt

12481 accactccca tttgcagtct ggggcagagc tggttctcgg ctacagaccc ccggagccct

12541 gggtgctcag cacctgggcc agctcctgat caagcagtgg gaggaggccc aggctgagga

12601 gggccagacc tatgggtggc tgggagcatg tttcgtgtca gagctggctt catcggactt

12661 agggccaacc tagcaccccc caaggcaccc caggccccgg gagggaccag agggcatggg

12721 tcgggggtaa agccaggggc agaccagagg gtcctgggag tactgtcgtg gggggtctgc

12781 tgagtcttgg gggggagggg catgggcacc aagggcccca cccaagacag tgcccctcac

12841 cccagtgccc gacaggcccc tccccccagc gcccgacagg cccctccccc ccagcacccg

12901 acaggcccct caccccagcg ctcgacaggc ccctcacctt ctggcatgtc cacccacggc

12961 cacggactcc catcacacac tcacccctgc gcacccaact gcctgctttc actcacaggt

13021 gcatgcacac attcccatca cactccacac ctctggccac aggctcaggc ttgcctccat

13081 gcagctccag cgccacacac acacctggac acgtactcag gtgcgctcct cacacacaca

13141 cctggacacg cacaggtgcg ctcctcacac acacacctgg acacacgctc aggtgcgctc

13201 ctcatgtgca tgctcaccct tacttgcgcc agccagcaca gacacacatg cacacacgca

13261 cacacgtgca caggcacaca catgcacaca tgcacacgca cacacatgca cacacgcaca

13321 cacaagcacg cacacacgca catgcacaca tgcacacaca caagcatgct cagaccatct

13381 ggcccttccc caaccttcac aggcctttgt ggactaaccc tcccatgctg acacccacag

13441 gcgcatgcca cccctgcagg cgcacataac gcacacacac cctcttgggc gcatatgcga

13501 gccgaagggc gtgggcaccc cagttagtga ggatacctcg gtctcttgag aggcagaggg

13561 agaccaagga gagggagggg gagggacatg gggacaggcc ccgggggggc tgctcacctc

13621 ccactcagga ctgacacagg ttggagtggg cactgctggg gccacacagc aggtgcacgg

13681 cagggtgggg gcggcaggtg gggctccctc cgaacggtgg acgcggacag ggcctccttt

13741 tctcccgaga gcgaccgttt ccaagagcac agcttcgtgg cagggagcct ccacggcccc

13801 gcccctacga ccctcacccc cagctccacc ccggccccca gccccgcccc gggcctcggt

13861 gtttgcgagc tccaggtagg agcccgtctg cagacgtgcc gaggaggtgg tgtgattgct

**FIGURE 26 Continued (Page 9 of 24)**

13921 ttagcgccgt cattttcaac cgtttataat cttcttctgt gtctgcatat tttctctgtg

13981 cacattattc atcagagtaa aaaaaggaac tatgaaaacc tcgaccaact gtcctatgac

14041 aacaagcgcg gacccaaggt atatatgcat ggacgtgcac gccacccacg gctagggagc

14101 cctggcctcg gcgcctcggc cactagggcc actgtctggc ccagccgccg agccgcaggc

14161 ccaagcaatg aggagaggca gccggcagca ggcaggagag ggcaggcagg agagggcagg

14221 cgtggcggcg tgggtgcctg aggcccaccc gccgtgaccc tagcctgcac cctcgaggca

14281 ggcgcggctg caggaggagc tgctctccgg gaagtgcatg cgaatctccg agaccccaag

14341 ggtttcctcg tggaacccgg gagggagccc gcccgcctgg ccacccactc gccggggccg

14401 ggctgctcct caggggcctg cggaatcaaa cctcagagga cctcccatgg ttttggaaaa

14461 gtcagcccca tctcttttcc tggttgcatt ccaaaactct tttctgtttc ccgtccgctg

14521 cacgcctcct gagtctgggt ccacttcagc ttgcatgctc agtgcaaaga tgaggacagg

14581 agtgaggtgg agagagagat ccagagagag cagagagagc acagaacgag cacaggtgag

14641 cgcgcaggct gaagacagga caggaccgga gaggcgaggc caggccaggc aaggactgag

14701 gaaggcaggc ggaggcgcag gtggcaggcg cagaccatgg cagccctagc taagctgcct

14761 cggggttccc agcggctccg gcccaactct cacccctgag gcgctatgtc ccctgcccca

14821 gccgcctgct aacactcttg ctcacaccgc aggcagagaa ggtgctgcag tttgacccag

14881 ggaccaagaa cgtgacggcc ctgctgatgg aggcgaaaga gctggaggcc cgggtcatca

14941 tcctttctgc caggtgaggc tgggcagggc cctacacact ccacacagga tggtacctga

15001 gccaagtacc cgccatctga gccagagctg ggacattgtt gggcacagtg accttcagct

15061 tccaaagcac cttcaccaag gacagccacc cccaccccca cccgcaccca cactcctatc

15121 ggcatggctg atgtgacacc ctccatctgt ccctcccttc ctgggccctt ccccattcca

15181 cagtcacatg ctgctgctgc cctgagctgg gctgtgggag agggatatgg aagagaccct

15241 gcccttggga agccccgcaa ctcaaggggg caaacctgtg caaacaaggc ccaggcctgg

15301 ggccagggac taggcccagg gaagtgatgt gcaggtgtgc caggcgaaaa ggcccacagc

15361 agggagaggg ccagatttcc caactgaagg attgcaacag ctgcagcagt agcttagggg

15421 gaaatcagtt aggtacccgg gaaatcaagc tgctctggac aggtccggtg ccacagagca

**FIGURE 26 Continued (Page 10 of 24)**

15481 accttggggt ggagcccact gtaaaaagct ccctatttgc aaatggctag gttctcccgg
15541 gaaggaaaag cctgggtgac tgggaatagg aaaagcaaga gtggggaagg gcagggtgag
15601 gagccttgcc ccatgggaca gccaaaaccc cactgtccct gacctaaaag ctctgctagg
15661 ctccaacaga gcggagcaaa acagcagtga aacacccgga ggaacacagc ccagccctcc
15721 agccctgcat atgggaaaga gccggcgaca ctctcagtcc cagggcagac caccatttcc
15781 acggtccatc aaatgaccct ctagcacgga gacagatgca gccccctcac cagggcagaa
15841 cgcagggtgg ggccagccag ggctcctcgg actagaaggc aggaatctcc ccagcccaag
15901 gtagggttgt tgcttagagc tgcccacggg gccttacatg ctcctcctgc agctgctata
15961 aaaaggcact aatcgtcccc cattacgccc cctgcactcg gctttaagct cacaggtcac
16021 ttgtccactc cactcatcca actgcaagcc ccagggaggg ttggcctggg ctccaggaaa
16081 aaagattttt aaagacgtga ccaggcaaag tcccaagggt atgcacaggt cccaaagaag
16141 gaatgccccg cccagggaaa gacccgccca gaaaaaaaga cccgccaagg gaaagctccg
16201 cccagaaaaa gacctgccca gggaaagccc cgcccagaaa aaaggtcctc ccagggaaag
16261 ccccacccag ggaaagctcc acccagataa aagcccgccc agggaaagcc ccgcccagaa
16321 aaaaagatcc gccaagagaa gtctccgccc agataaaagc cccgcccaga aaaagacccg
16381 ccaaaggaaa gcccagccca gaaaaaagac ctgcccaggg aaagccccac ccagggaaag
16441 ctccgcccag ataaaagccc gcccagggaa agccccgccc agaaaaaaga ccagcccaag
16501 gaaagctccg cccagggaaa gccccgccca gaaaaaaaga cctgcccagg gaaagctctg
16561 cccagataaa agcccgccca gggaaagccc cgcccagaaa aaagaccagc ccaaggaaag
16621 ccctgcccag aaaaaagacc gcccagggaa agccccgccc agaaaagacc cgcccaggaa
16681 aagctctggc cagataaaag ctcagcccag ggaaagcccc gcccataaaa aagccccgcc
16741 cagataaaag ccctgcccag cgaaagcctc gcccagggaa agccccaccc agaaaaagac
16801 ccgcccaggg aaagcccagc ccagagaaaa gacccgccca gggaaaactc tgcccagata
16861 aaagacccgc ccagggaaag ccccgcccag aaaaagtccc gcccagggaa agccccgccc
16921 agaaaaaaaa cccgcccagg gaaagccccg cccagaaaaa agtcgcgccc ggggaaagcc
16981 ctgcccagaa aaagtcccgc ccagggaaag ccccgcccag ggaaagaccc gcccagaaaa

**FIGURE 26 Continued (Page 11 of 24)**

17041 aagtcccgcc cagaaaaaag tcgcgcctgg tgaaagccct gcccagaaaa aagaccagcc

17101 cagggaaagc ccctcccaga aaaaaagacc cgcccaggaa aaagctctgg ccagataaaa

17161 gctccgccca gggaaagctc cgcccaggga aagccccgcc cagaaaaaaa gccccaccca

17221 gggaaagccc agcccagaaa aagacccgcc cagggaaaac tccacccaga aaaaagacca

17281 gcccagagaa agcccaaccc agaagaaagc cccacccagg gaaagccccg cccagggaaa

17341 gcttcaccca gagaaattcc cacttagaga aattcccact cagagaaagc cccacccaga

17401 aacgtgccac ccagggaaag ccccacccag tgaaagcccc taccagaaaa agccccgccc

17461 agaaaaagcc cctcttagag aaagccccgc cagactctca gaagttaatt tcctttttcg

17521 ttgttttgag agggagtctt gctttgtcac ccaggctgga gtgcagtagt acaatctcag

17581 ctcactgcaa cctctgcctc ctgggttcaa gcgattctcc tgcctcagcc tcccgagtag

17641 ctgggactac aggcacaagc caccacaccc ggctaatttt tgtattttta gtagagacgg

17701 ggtttcacca tgttggccag actggtctcg aacttgtggc ctcttttttt tttttttttt

17761 ttttctttga gatggagtct cactctgtca cccaggctgg agtgcagtgg ctcgatctcg

17821 gctcattgca agctccacct cccgggctca cgccattctc ctgcctcagc ctcccaagta

17881 gctgggacta caggcccctg ccaccacgcc ctgctacttt tttgtatttt tagtagagac

17941 ggggtttcac catgtcagcc aggatggcct caatcttctg gcctcatgat ctgcccgcct

18001 cggcctccca aagtgctggg attacaggag tgagccaccg tgcccagcca cttgtggcct

18061 cttctgttat tttctgaatt gtttacactt cccttactca tcacagagct tgagagaaat

18121 tctgtagctg tgatgggata aaaggatgga tgggcaggtg aacaaatgga cagacagctg

18181 gctgggaagt ggaatttctt catccaagat gggtcaactc aaggaatcga ttgccctaaa

18241 acatacctgt tccactgttg gccacttttg caatagacaa gttcacatca agctagacct

18301 gcctgatccc tacattctaa ctggagtgac caacaggaca cgggagagaa aaccacatac

18361 aaaaccaatc cacagaaccc cgccatgccc aggtccccac agagagggga gggggcgttt

18421 tctccacttt tttttctcgg cctgtgagcc cctgagccgt gcactgcggt cccacaggtt

18481 gaccccgcgt tgccagcctc agggccaagg tcacgtttcc agacatggcc ctaagaaaaa

18541 ggccagccca ggggaaggga catggtcagg gcacacagga accacgtgca taccacacat

**FIGURE 26 Continued (Page 12 of 24)**

18601 ccatgcccat gagcacacac cacacaccac atgtgtatgc acataccata cacacgtgca
18661 caaatgcatg tatacacact atagtcacac catgcataca tctctaccca cacatgcaga
18721 atcatgtaca ggtcatacac aacacacacg catgtatgca catgccatat acacaccaca
18781 tgtaccatgc atacaccata cacacatgtg cgcaaatgca tgtacacaca ccatcacacc
18841 actcatacat ctctactcac acatgcagaa tcatgtacag gtcatacaca acacatacac
18901 gtgcacacat gccatataca caccacgtgt acacacatgc accatgcata caccacacac
18961 acacgtgcac aaatgtatgt acacacacca tagtcacacc actcatagat ctctacccac
19021 acatgcacca tcatgtacag gtcacacaca acacatacac atgtgcacac atgccacata
19081 ccacgtgtac acacatgcac catgcataca ccatacacat gtgcacaaat gtatgtacac
19141 acaccatagt cacaccatgc atcatctcta cccacacatg cagaattgtg tataggtcat
19201 acacaacaca tacgcatgta tgcacatgcc acatacacac cacatgctcc atgcatacac
19261 acacaaatgt atgtacgcac catagtcaca ctacacatac atctctaccc acacatgcac
19321 catcatgtac aggtcataca caacacatac acatgtgcac acatgccata taccacatgt
19381 gtacacacat gcaccatgca tacaccatac acacgtgcat ctacacatac agatacaggc
19441 acacacacca ccatatacat cacatacaaa gacatccact gatatacgca cacctacata
19501 catgtacaca cagcacacat gcacatacat cacaacacac atgcgcacca cacaccatgt
19561 gcacacccat acacccatgc tacatgcaca ccatacccca cacatatgca tacactgacc
19621 acagcacaca tcaaccacac acccgccaac ataactcttt ctctttttg gggagataga
19681 gtcttgctct gttgcccagg ctggagtgca gtggcgtgat ctcagcttac tgcaacctct
19741 gccccctggg attcaagcga ttctcctgcc tcagcctcct gagtagcagg aattacaggt
19801 gtgtgccacc atgcccggct aatttttgta tttttagtag agatgaggtt tcaccatgtt
19861 ggccaggctg gtcttgaact cctgacctca ggtgatccgc ctgcctcagc cttccaaagt
19921 gctggcatta caggcatgag ccaccgtgcc tgcctttttt tttttctttt ttttttgtgt
19981 gtgtgtgtga gacagtcttg ctctgtcacc caggctggag tacagtggca caatcttcgc
20041 tcactgcaac ctcggcctcc caggttcaag tgattctcgt gcctcagcct cccgagtagc
20101 tggaattaca ggtgcaggcc accatgcctg gctaagtttt gtatttttag tagagactgg

**FIGURE 26 Continued (Page 13 of 24)**

20161 gtttcgccat gttggccagg ctggtctcga actcctggcc tcaagtgatc cacccacctc

20221 ggcctcccaa agggctggga tgacaggcat cagccaccac ccccagctcc aacaactttt

20281 tttttttttt tttttttttt tttttttttt tttttaagat ggagtctcac tctgtcaccc

20341 aggctggagt gcagtggcgc gatcttggct cactgcaacc tccgcctccc agattcaacc

20401 gattctcctg ccacggcctc ccgagtagct gggattacag gcatgcgcca ccaccccggc

20461 taattttttt gtttttgtag agacggggtt tctccatgtt ggtcaggctg gtctcaaatt

20521 cctgacctca ggagacctgc ccgcctcggc ctcccaaagt gctgggatta caggcatcag

20581 ttactgcgcc tggcctcaaa atacttcaga gcaatggctc tggactgtca gggctggaaa

20641 ggaccctcct ctcacatgct gagctccttg gagtggaggc caaagtccca cagcagaggc

20701 cgactggccc acccagcatg cccatctccc aggagtgcag agaggcaagc cctgggcagt

20761 ggcaggcaca ggccttcttg accccagacc ttcagcctgt catattttgg ctcctcctta

20821 gtgaaggttg ttgagggtgt tttgcagaga gacatgacgc caatcttaat ttttgacaat

20881 tttccatagc atgcagataa tttgtttcca aaacttttca ttttcctgaa gtcatcttga

20941 ttggtatcag ctatttccat aaaacgatcg gatgagtttt gatggacaga tcaggctttt

21001 gtttacaact gttttgctcc taatcattcc accacatcac atgtcatgga cctgaattgc

21061 gtcaagaaga cgggcttgtc tgtcaggccc tggtgggcac tttgatagcg ggcatgctgt

21121 gccatgacac gtgtggtgtt gggtcttgct ggacaagctg tgctgtgttc agtgtgcgga

21181 gcctctgcta gatgctctca tttggggcac tgggccagtg ctactgggag cacttctgtt

21241 ttgtgtcact gacatccaat agcatcgtta tgtagagcaa acaccgaagg gctgcatttc

21301 tttgtgggct tattctcgag aaaactgggg gcagatccct cctcaaggag gggagggcca

21361 ccttggtttc cagtcaagta ttgtgaaaat tatccaacac tcaggcaatc cacccaaccc

21421 tgctgcccat gtctggagaa gcaaagtgtc aggggtagtc caggcccacc tggagacagg

21481 tcaggccctg cagagaaagg tctgacagac gggggtgagg gaagaccccc caaaggcctc

21541 cagagtccca ccaggtctcc aggtccttgt cataaccaga gaggccccag ccccagagga

21601 ccaggtcccc tgctccactg tccacagggg cccacctgca agcacactgg cagagctcaa

21661 gaccacacat gcctgcaagg tgaggcctgt ctgggctctg tcctcctgca ggccccaggc

**FIGURE 26 Continued (Page 14 of 24)**

21721 ctgggtggct gggcgaaggc agctgcttat gcagactcca gggggaaagc cgcctctcat

21781 ctctggccgt ccccaggacg ctggatccac caatatctca ccaacctgga gagccactca

21841 acccctcatt tcacatgttt gaacatagag gaccagaggg gtgtggcctg tctaggaggt

21901 cttaggagct cgggtcctga ctctgccact taccaactct tgtgtgtccc atgtgcctcc

21961 gcttcccctc gggacacaga gatattgtga aagttaaaca acataatccc cgtaaaacac

22021 ttcgagcagt gcctggtatc tggccagcaa gtgatcaatg gtgatccatt accatcctgg

22081 gaccccatca gagccttctg aggtggaggg aagggcgtgc tggggagcac aggtgcaggt

22141 cacaagaagg aagtcagtcc cataagccag gtatctaacc ccatccctgc tcccccaagg

22201 taagggccag catctaaccc cacccctgct cccccaaggt aagggccagc atctaacccc

22261 atccctgctc ccccaaggta agggggccag catctaaccc catccctgct cccccaaggt

22321 aagagccagc aacggaggcc tgggaggctc ctgggttctg ggccgcagcg cctctgcgag

22381 gtctgcaggc ttcgctctag gaggggatgg gggctgggca ggtccctgct ccagaggagg

22441 aggacctggg cctgcggagc gccgcggtgg gagtgctgga gtcctggccc gtcatccccg

22501 tctgccccac agcgaggacg atgctgccac tgtataccgc gcagccgcga tgctgaacat

22561 gacgggctcc gggtacgtgt ggctggtcgg cgagcgcgag atctcgggga acgccctgcg

22621 ctacgcccca gacggtgagt gctgggcctt ggcggggtcc ccgaacgggg aggaccccac

22681 gggctctgag tcgcatgctc gcctaggcat cctcgggctg cagctcatca acggcaagaa

22741 cgagtcggcc cacatcagcg acgccgtggg cgtggtggcc caggccgtgc acgagctcct

22801 cgagaaggag aacatcaccg acccgccgcg gggctgcgtg ggcaacacca acatctggaa

22861 gaccgggccg ctcttcaaga ggtgggcggg gcctccccgg agctgggcgg ggctgctctt

22921 ggggaggtgg gcggggtcac tccagagatg ggcggggccg ctcttgggga ggtgggcggg

22981 gccactctcc agagctgggc ggagcagctc tcaggactag gcggggccgc tcttagggag

23041 ctgggggagc gctcctcaag agatgggtgg gggcactctc ggggaggtgg gcggggtcgc

23101 ttccaggagg tgggcggcgt cgctctcagg ggtactgcag tggagcctgc tgccaacatc

23161 ctctggacac tgttacttct ctcctctccc cccacacccc cagcaccacc acatctaatg

23221 gcacaatcat ctgccctctt ctcaacactg acaccagtac ctgggccgtc actggagtgg

**FIGURE 26 Continued (Page 15 of 24)**

23281 ggactggctc cactgcctcc gcccctactt tccacactgc agcccacccct gaaacagcac
23341 ccctctccct gtgtggctgg cagcctttgg gaggaggctc ttgatgcaga tggggactga
23401 aagcttccag ggacccagga ggccagacaa gcagcccaag aacagcacac gagccttaga
23461 cagccagggt tggccaaggc ccagagaccc aagtgaacat ctgcagtgtg gcaggagtta
23521 gctcacagca cgcctggaca ccatgccatg ccagctcacc cccagatccc caaccactga
23581 gtagcacgtg cagagccacc atccacaacg cccacataag tgcagatgta ggcagcacgt
23641 gtgcacacac acgacacaca tacacagaac catgtgtgca cacagactca ggcacatgac
23701 acacatgtga cacaagcaca tgcatggggt gcaccccaca tagggatcac gtgtgcacac
23761 aggttcactg atgtggcccg atgggtacaa tgcacacgtg cacacacagc cggacaggac
23821 agcctggtgg ttagagctgg ctcaacctcg ccttcacttg ctggcaagag ggcaggcatc
23881 cttctgagct tctgcctccg tctctgtaag gcaggatggt tctgaggaca acgtcctaac
23941 ccacagaaag ccgggtctgg cacccataaa ccactcagct gtcatgaacc acaccgtcca
24001 tctggtgcag gcagatacca cggtgtccag ggtctggcgt ctgctgatct ttccgttctt
24061 gggactggga caagggaaaa gcccaagctg ctcagccggc aggagaagga gcagggagaa
24121 ggagcagggg gaaggagcag ggagaagcag caggggggaag gagcagggag gagcaggaga
24181 aggagcatct ctgagaagcc tcagctatgc ttccttccct agagtgctga tgtcttccaa
24241 gtatgcggat ggggtgactg gtcgcgtgga gttcaatgag gatggggacc ggaagttcgc
24301 caactacagc atcatgaacc tgcagaaccg caagctggtg caagtgggca tctacaatgg
24361 cacccacgta ggtgggggtc atgagggggt gggggctggg gccttagggt cctggggcca
24421 agacccctgc gtggccaccc tccatctcat actcccaccc ccaggtcatc cctaatgaca
24481 ggaagatcat ctggccaggc ggagagacag agaagcctcg agggtaccag atgtccacca
24541 gactgaaggt gggggcccca cagacctccc tcagtgtccc caccccagac agcccatcca
24601 ccccctctgg cctgaaggag gagggtgcgg tgaggtcaat gaaagccact aaaggaagtg
24661 ggggtggggc ctgcttcccc tggacaccgt ccagcacacc tggcacagca caggaagcag
24721 agagaacagg agggaggaga ggaagctgcc cccatcccac agggggtctc cagtgcccct
24781 cttgacccag ccctacttaa gtctggggca gttagttgtc tgacaggacc ctgctgggga

# FIGURE 26 Continued (Page 16 of 24)

24841 agagcagatg ggggacagca ggcagacctc agcttcagca ctcgctgtcc ccagtcctgg

24901 tcctccacac ccctcatccc tcctccagcc tgcattgctc ttgatgggac cgggtcaaac

24961 tgtcctcttc caccgtgtgg gacagccctt cctgactccc ctgggcctct gagagcctct

25021 gccctcgccg gcttcctcct ccagaacatc tttcccttgg ctccctactc cagggtgctc

25081 tcctggccat tcctccccgg gcagagccac actaccccca ctccacacac actccagtcc

25141 tggtagcatc acagaccacc aaaggcaagg acctcacagg cgacacgccc accaaccttc

25201 tctcggtcat tccaagccct caaatgtctc ttgaccctgt ctgttttctg agcccacccc

25261 tgaagcttgg tgtcagcccc tgtgacctct cacccaggct ccctcccctg ctctgcaccg

25321 gcccctgtgg cctctcaccc aagctccctt ccctgctctg cagacagggt ggggttttcc

25381 agtgccagtg tgggtttcat tgcagcccag acacctcaca ctgaaaagtc tgaaagcagc

25441 ggtcaaacgc taatggccaa aaggccccat ctaggctgtg agatggagat ggctttttac

25501 aaatttgttt ctggcctcac taattttta aaaatactag catatatatt acctgtataa

25561 caggaaaatg taatgaaagt tttataaagc aaatcaactt cttcaatggc tcctgattcc

25621 cctggggata aaagacaaaa tgctgcctgg aggctgaggg tgggcgggcc tgcccccctc

25681 tcaggctcac cctgcctagg acatgccggg agggtgcctc tcccaccacc cccacgcctc

25741 cctgcctttg cagttctgga ctgcagactc ctctgctcca cctgccctca ggcacctgct

25801 tgatccctgc ccaccttgag ggctcagctc tgacaccatc tcccctcaca gttctggcag

25861 tgtgctatgc tctattccag ccccctgtgc cccagatccc ttccccaccc ccatgccatg

25921 gtcccttgaa ggacagacag gagggcgagc ccaagcagga gtgtgggtcg aagaggccac

25981 ggcgcggtgg agcacgtaca cacgggcaag agaaaggagc cagagaccta cattcaaagc

26041 ctgagggctt cgggactggg ggccgggaca ggcagtgcgc cgggatgaag ggaggcacgg

26101 gtgggtggcc ccacgggtcc caggtcctgt gcaggtgcag ggtcggcttt gtggacatgc

26161 ccctgtcctc gtggcacagc agggtggggg tcagcctgca ggctgggctg tttctcaccc

26221 caggaagatg cctggcatac acgggacatc agcggctcct ctgctggagg gaatcatgtc

26281 ttttttttt tgagacagag tctcgctctg tcgcccaggc tggagtgcag tggcgcggtc

26341 tccgctcact gcaagctccg cctcccgggt tcacgccatt ctcctgcctc agcctcctga

**FIGURE 26 Continued (Page 17 of 24)**

26401 gtagctggga ctacaggtgc ccaccaccac gcccggctaa tttttttgta ttttcagtag

26461 agacggggtt tcaccgtgtt agccaggatg gtctcgatct cctgacctcg tgatccggcc

26521 gcctcggcct cccaaagtgc tgggattaca ggcgtgagcc acagcacctg gcggggaatc

26581 atgtctaagc caagactgga gaaaaagtgg ccaagagaag ggtccagctc tccaggagtc

26641 ttttctgagc ccccagcccc caccccccg gggctgcagg cagacgatgc tgacggtggc

26701 tggggaggac gtgtcctgaa cacttgggct cgtgaagaag ctccagagag gggcagtggc

26761 cggcggcgca gggcgggggg tgtgaggggt gcgtcgggga ttaagagggg cgccagggga

26821 ggctgggagc tgagaagaga ctgccgccct gggcagcctt aggtcggtgg tccaggctgg

26881 gtctcccctt cccccccaga ttgtgacgat ccaccaggag cccttcgtgt acgtcaagcc

26941 cacgctgagt gatgggacat gcaaggagga gttcacagtc aacggcgacc cagtcaagaa

27001 ggtgatctgc accgggccca acgacacgtc gccgggcagc cgtgagtgcg cggggcaggg

27061 cgcggggcgc ggggcagggc gcggggcgtg gggcggtctg gagcccagca gttaccgccc

27121 gcacctaccc agcccgccac acggtgcctc agtgttgcta cggcttttgc atcgacctgc

27181 tcatcaagct ggcacggacc atgaacttca cctacgaggt gcacctggtg gcagatggca

27241 agttcggcac acaggagcgg gtaggctgga cggcgggggt ggggaccagc gtgagagggg

27301 cctgcaggcg cggtcggagt gggtggggca tggagtaggc ggggcttgca gatggtgggg

27361 ggtcctgggg tgagtggggc atggagtgag cggagcctgc gggctgggtc ctggcgtggg

27421 taaagcatgg ggtgggcggg gcctgagggc tgggtggggc ctgacatggg aggggcctga

27481 cgtgggggtc ggagtgggtg gggcacggag tgggcagggc ctgcaggcgg gggtctggag

27541 tgggcgggac gtggagtggg cggggcctgc tggctgtggt ggggcccgcc cggcgtggga

27601 ggggtctgcg agccagggcg gggctggagt ggggtggggc ctgcgagctg ggtagggtct

27661 tggggagaag acccccggag tgctctaggg cggcttcagt cgggggtacc tgtggcggga

27721 gctgggagga cgctgcctgc atgcccgccg gctctgtcgc ctcgcaggtg aacaacagca

27781 acaagaagga gtggaatggg atgatgggcg agctgctcag cgggcaggca gacatgatcg

27841 tggcgccgct aaccataaac aacgagcgcg cgcagtacat cgagttttcc aagcccttca

27901 agtaccaggg cctgactatt ctggtcaaga aggtgggcag gggccgggtg gcggggtggc

**FIGURE 26 Continued (Page 18 of 24)**

27961 ggcgggggga gtccctggag ggcccgggcc gcgctgacct cgcgtccctc cgcaggagat
28021 tccccggagc acgctggact cgttcatgca gccgttccag agcacactgt ggctgctggt
28081 ggggctgtcg gtgcacgtgg tggccgtgat gctgtacctg ctggaccgct tcaggtgagc
28141 gcgacccggg gctcagacac ctccatctgc ggggcgcgga gccggccagg ggcggggcag
28201 ggccgcctct cccgccctct ctcccgcccg ccctctgcgc cccgcagccc cttcggccgg
28261 ttcaaggtga acagcgagga ggaggaggag gacgcactga ccctgtcctc ggccatgtgg
28321 ttctcctggg gcgtcctgct caactccggc atcggggaag gtaaggcccc gcccggcccg
28381 cctggtcccg cctcggccct ctagggtctg acagagcccc ccgcccgccc acaggcgccc
28441 ccagaagctt ctcagcgcgc atcctgggca tggtgtgggc cggctttgcc atgatcatcg
28501 tggcctccta caccgccaac ctggcggcct tcctggtgct ggaccggccg gaggagcgca
28561 tcacgggcat caacgaccct cgggtgaggc ctggccgggc tgggggaggg aatgcgaggt
28621 gagctggggt cggcctcggt taggggcctg gggagccgcc gccgcgatcc ctgccctccg
28681 accctgcagc tgaggaaccc ctcggacaag tttatctacg ccacggtgaa gcagagctcc
28741 gtggatatct acttccggcg ccaggtggag ctgagcacca tgtaccggca tatggagaag
28801 cacaactacg agagtgcggc ggaggccatc caggccgtga gagacaagtg aggcgcgggc
28861 ggccaccctg gcggggcggg acaggtgcgg ggaggggggag ggtggcctcc accgggcagg
28921 agagcgtccg ggccgggcac cccggagggc gcgggcgtgg ggcttccagg ctggcaggac
28981 caaggccccc gtgactccgc ctctgccggc agcaagctgc atgccttcat ctgggactcg
29041 gcggtgctgg agttcgaggc ctcgcagaag tgcgacctgg tgacgactgg agagctgttt
29101 ttccgctcgg gcttcggcat aggcatgcgc aaagacagcc cctggaagca gaacgtctcc
29161 ctgtccatcc tcaagtgagt gtccgtgcgc ccgcgtccct cctccgcccc tctccgccag
29221 aggtggacgc cctccccagt gccagaccac tccgaggcca ccactgattt cccacccagg
29281 ccgggcgctg cccactccac gccgcaccct accccgcagg ccccgccccg gccccgcccc
29341 cagcttgctc cttcccgtcc tgggccccgc ctcactgcag gctcacttgt tcccaccgcc
29401 aggtcccacg agaatggctt catggaagac ctggacaaga cgtgggttcg gtatcaggaa
29461 tgtgactcgc gcagcaacgc ccctgcgacc cttacttttg agaacatggc cggtgcgttc

**FIGURE 26 Continued (Page 19 of 24)**

29521 tccttcatcc attctcgggt gggttctccg tgggctgcgg cctccctggc cagcaactga
29581 ggctctgggt cccggcacac aggggtcttc atgctggtag ctgggggcat cgtggccggg
29641 atcttcctga ttttcatcga gattgcctac aagcggcaca aggatgctcg ccggaagcag
29701 atgcagctgg cctttgccgc cgttaacgtg tggcggaaga acctgcaggt agggcaggcc
29761 accctccgag gcctggtgcc cagggcccgg cctggccacg gccctcctcc atccccgaag
29821 gccgtggcac tggctctggc tctggtgggc aggactggag ctaggagcca tggccagggg
29881 cagtggtgag tgctcccagg gcacgggggc agcaccggtg gggggctgcc tgcaggtggc
29941 tgcccactgc aaagccgggg ccgagggagg ccacgcaccc tgctccaagc ctccgcctgg
30001 cccctctgtc tccagagtcg cccgccggta cccattccat aggaaggcaa tcaggcaggg
30061 taagacaggg gcccgcctgt gtatggcacg tgagtccaag atgcattttg ccctccgccg
30121 acccaagccc cttgacaccc ttcggagacc cccccctttc ctgctatgtc cttgtgctcc
30181 gtgactctaa tccgaattgg gccaggtccg gtcctgcctg gtgcccaggt tgtatccatg
30241 agaatttgcc accagcaagg gcagccacgg cccacctggg acagggtggg cagtgggcct
30301 gtacaggcct aagggctcgt ggcccgcggt cgagttccgg ttcactccgt ctcttctctt
30361 tctctgggtg ccgtcctgga gcctgtgtcc tgagatgaag ccgacagtgc ggccagggct
30421 gctgggggat gggggttgct ggaggctcca cacctctcat ccgcccgctc ttgctcttgg
30481 cccccacagg tcccctgggg acctggccgc tgccagcact ggcgggcaca ggccacctgg
30541 ccatcagacc tgaggccaga gtcccgggag ctgcctctgt cactccaatt ccacctcgac
30601 acctgcctcc agccctcggc cccttcctga atcttggtgt gtgccccttg gggtcagtg
30661 gcctccacgc agacagctgg tgtggcctga ggggcaactc ctccagtcct cagaggactc
30721 ctcctcctcg ggacgcctgt aagccagggc cacccaggag ccagggagcc aggcggacct
30781 cccaggaaga gccagccgag agcccccaag cccagcccca gcacgagcaa ggtcaggccc
30841 gagaccccgg gcaggagaag aggccaccct cgaacgtccg ctgtcggccc gtctgtccag
30901 cacagggagg caggcaggag cgagggccca agtggccggc caggctgggc agcggcccat
30961 gcaggagcag gcgagggcag gtgtggccac caccctagcc atctaatcac ttatacatat
31021 tcattttagg atagaaagag tggtagagca gagcctgacc ctaaaaagaa agccacattt

**FIGURE 26 Continued (Page 20 of 24)**

31081 agggctatca cctccaccct ggcttccagc ttcaagaggc gtaggtcctc caaagacacg

31141 gtaaggggga gagcacccca gtcccgcgtc cgactccacc tgccctgccc tgcgtgtgtc

31201 tcccgcccca tcaccccgcc ccggaccctg ggctcctgtg gcccactctg cccctgtctc

31261 cctgtggcgg ccgctctgcc cagcccgccc atgctgctct ctctcactct ctggaccttt

31321 ctccccggcc ctcctgggtc ctcggctttc cccgtgtgtc tccgttagtc tgcccgccca

31381 cctcccctgc catgacccac acgccatctt gaagcctgtc atctcgttgg tcagtcagtc

31441 agccacacca cctctcgggg ccaggtctgg ggccctggga gcccagcgtg gccccatcct

31501 ggactcctca gctgccggga ggccacacca cttctctgtt atgtccccgt ttctctcgcc

31561 tctcccagag gggcccgccg ccctcacttc gcccctgcga cggccctgga gggggtggct

31621 gtgatgtccc atcccgtccg tctgtctggc cactggcccc gccccccaga cacctgtctc

31681 acctgtctca ccagagccat gcgtgttgca tcttcatgtg gtctctgtgt gggccggggg

31741 ctgggggccg ggcctgggtc cgtctgggtg gacggctggg gcctggagtt ggaactggcc

31801 ccggccacag gggactgtca ggcagggagt ggggtgggac caaaaggggt ggctcccacc

31861 ccaggctgag cgggggccct gcaggaggtg tggcggcagc tcccagaggg tctgagaatg

31921 ggtaggggcg gccccacaag ccctggcctg cagagcccag gacgacactg aggttcccag

31981 acagggaggc ctctggaagg gaaacgacca cctcagctcc tgaccccagc aaccccacaa

32041 ggcccacccc aaagagccag gccttctgcc ctttggagcc cagaatcccc cacctcctgc

32101 tcggggcagc ttgtccctgt agcggatatg cacactcgga ccagaggccc ccagagcgaa

32161 cccagccttg ctagaggcac cccaggccca ggcaccatgg tggggagggg ctgcccagag

32221 aggcagcgga gacctcagcc ccgtggccac cctgcagtcc agggaccagt ctggcccaca

32281 ggaagccccc agcccataag cagcatcacc agagagaagc ttacgcccgg gggaggaagt

32341 gcgatttgca gccacctgcc cctcagtgca ctggaagcgg ggcagacctc cagggcacag

32401 acaggacttg gcatcaagca agccaaatcc cgagatgaag ccaccagggt gccccaagag

32461 ggacccatga ggcctggctg ctcagcttcc tggggaaggg acttggcatg caggatgggt

32521 ggacagtgag agcctgtagg cctgggggcc actggaggct caaggagcag gtggaagcac

32581 cattcctgga gccacctctg ctgcggaaag cgggcagagc tgatgctgca aagtctgagc

**FIGURE 26 Continued (Page 21 of 24)**

32641 caggagtccc gcagggaaca gggaggggga atagcgcagg gatcgtgggc tgggcaggct

32701 ggggaagagg gggtgtccag gcagacagga gaaacagcga tttggggcag gcagccacgg

32761 ggggcaagca caaatgtcgt gcaggtgatg ggccactttc agagggtgac actgggtccc

32821 agggccctgc ctggagcgag gccaggtgca gctcagagac cctcatggtg ccctcccagg

32881 gacatgttcc cagcggaacc ctcacccgag cctctctggg caccagggac cgtcctctgg

32941 ggccagttct ggcatcacgt ggcatctggg gctggccccg ccctgcaagg ctgaactgtg

33001 gggggcactg ccagctgggg gtctgggcag gggagggcag cccagctccc acctggtctc

33061 tggggctgcg agcttattca gagggaggcg tgggtggggg gctcctttgg gtagggtggg

33121 gtcagtccgg ctgcggagat cccctgcccc tgtcctgtgg ccggtccggg ccagggcggc

33181 actgggcgct gagggctggg gtccctggcg gccggcgggg ccagcgggta ttgattgttg

33241 gttcttattt atagagcacc gggggtggac gcggcgcttt gcaaaaccaa aaagacacag

33301 tgctgccgcg acgcgctatt gagagggagg agggccagct gcagctgtgt tcccgtcata

33361 gggagagctg agactccccg cccgccctcc tctgcccccct cccccgcaga cagacagaca

33421 gacggacggg acagcggccc ggcccacgca gagccccgga gcaccacggg gtcgggggag

33481 gagcaccccc agcctccccc aggctgcgcc tgcccgcccg ccggttggcc ggctggccgg

33541 tccaccccgt cccggccccg cgcgtgcccc cagcgtgggg ctaacgggcg ccttgtctgt

33601 gtatttctat tttgcagcag taccatccca ctgatatcac gggcccgctc aacctctcag

33661 atccctcggt cagcaccgtg gtgtgaggcc cccggaggcg cccacctgcc cagttagccc

33721 ggccaaggac actgatgggt cctgctgctc gggaaggcct gagggaagcc cacccgcccc

33781 agagactgcc caccctgggc ctcccgtccg tccgcccgcc caccccgctg cctggcgggc

33841 agcccctgct ggaccaaggt gcggaccgga gcggctgagg acggggcaga gctgagtcgg

33901 ctgggcaggg ccgcagggcg ctccggcaga ggcagggccc tggggtctct gagcagtggg

33961 gagcgggggc taactggccc caggcggagg ggcttggagc agagacggca gccccatcct

34021 tcccgcagca ccagcctgag ccacagtggg gcccatggcc ccagctggct gggtcgcccc

34081 tcctcgggcg cctgcgctcc tctgcagcct gagctccacc ctcccctctt cttgcggcac

34141 cgcccaccca caccccgtct gccccttgac cccacacgcc ggggctggcc ctgccctccc

FIGURE 26 Continued (Page 22 of 24)

34201 ccacggccgt ccctgacttc ccagctggca gcgcctcccg ccgcctcggg ccgcctcctc

34261 cagactcgag agggctgagc ccctcctctc ctcgtccggc ctgcagccca gaacgggcct

34321 ccccgggggt ccccggacgc tggctcggga ctgtcttcaa ccctgccctg caccttgggc

34381 acgggagagc gccacccgcc cgcccccgcc ctcgctccgg gtgcgtgacc ggcccgccac

34441 cttgtacaga accagcactc ccagggcccg agcgcgtgcc ttccccgtgc ggcccgtgcg

34501 cagccgcgct ctgcccctcc gtccccaggg tgcaggcgcg caccgcccaa cccccacctc

34561 ccggtgtatg cagtggtgat gcctaaagga atgtcacgca gttttcggtc tgtgtcgctt

34621 gttgacgccg gcagacagtg taaagggagg gcaaaggcat gggggaagct tcgagcgctc

34681 caggcggccg cggccgctca ggcttgggcg gcagcggcgg ggctccccgg gtccgcgggc

34741 gaggcacagc cgtgggggtc gggatcgggg ttcgggtctg gcggtctcgg cgggcggagg

34801 gcggcggtgc ggaggcggcg gcggcgcgca cggcaggcgg tgagcccaga gcccagcgcc

34861 aggcaggaag ccaggctgac gaggaaggag gccggcccga gcgtgtaaac cacggccagg

34921 tcccgcaggg cgagcggctg cgcgcaatgg ctaaaggcgg cgtcggaaaa ggcagtcagg

34981 gggctgagcg tcaggcgtcc cggccacacg cagagcaccg tctcggcctc tgtgggacac

35041 agacaaaggc gcggcgtcag gtggcggctg ccgcaccgcc ctgcagaccc cgacccgcgt

35101 ccccagcagc tcacctgacg cgggcagcgg gtgccggcgc agccaggcgc agagcgggcg

35161 cagcgcgcac ccgcagcccc aagggttgcc gcgcaggtgc agcgcgtcta gagcgggcag

35221 gcggcccagc agccccggcg cgagtgccgc cagctcgttg tcctgcaggc tgagtgagcg

35281 cagcagcggg agcgcgccta gcgccgcggg ctccaggcgc gccagccggt tgccggccaa

35341 tgagaggttg cgcagcgcgc gcagcggcgc gaaagtccct ggtgccagtg cttccagctg

35401 gttggcgctc aggtccagca gctgcagcgc gcccaggccc cagaaggctc gcacatgcac

35461 cgagtgcagc ccgttctcgc gcaggtccag gcgctgtagc gcgcccgctc ccgcgaaggc

35521 acctggcggc agcgcacgga cgcggttgtg gtccagcagc agcgcgcgca ggcgcaggct

35581 caggcccggg ggcacggcgg gcagcgagag tgccgagcag ctggccaggc ctcccggcac

35641 gcacgtgcac acctcggggc agtccggggc gcccagggac cccgagggcg aggccgtggc

35701 cgacacctgg gcccagacag gccaaggcga cagcagcagc aacagcagca gcagcggccg

**FIGURE 26 Continued (Page 23 of 24)**

35761 aggccgcgac caggaagggc cccgcatggg ggcagccccc ccgcccccgg cacccgcggt

35821 gggaggcccg ctgcctgtgc gtccctggag cccgtcgtcc gcggagcccg ttcctgcggc

35881 gcctgcacaa atattaactc tctggcccga gctcaggcag ttcctgtccc acggctggat

35941 ccacgcttgg ggcgggggca gggcaaacag ccaacgcccg gcaccgccag ccacctgtcc

36001 gggagctcca aactactctt ggctcagcgc cggccacagc gctatcaaga ccacctcacc

36061 ccgccttgtc caccaccggg cgcccgccga gccctgacct gagcagcagg acaccgccca

36121 cctgcacccg cccagctcac ggctgcaaca ggcgcccaca cgcaatgcaa cccagaggtc

36181 ctgctcctca cccgtctcga ccccacccag gctccgcaaa gtgatcccaa cgtgcacatg

36241 cggagtggcc cccacgcagg gatggtccca ttcccatgct ggatagggcc tgggttggag

36301 acaggccttg ggtggaggg agacagcaca cccgaggcag gagaggcgtg cctgccccac

36361 ccctcccccc caggactctc ctgggataga ggtacagacc agtgcagtgg ggcaggggta

36421 tcagcccaag tcctgctgtt aaacccagcg cccttcacag ttgccagttg caggtcctgt

36481 tctaggggct ttccaaagct gggtgcagga aggagccggg ctgaccagct gtggcagaga

36541 aggtggaaac attaggggta tggcttactg ccagggggca gaggaacagg ggaacttgct

# FIGURE 26 Continued (Page 24 of 24)

```
   1 gaggtattgt ggccagtatt ttggttttct tatgttttgg agtcacacaa gctaaagacg
  61 ggccattttc cagacctcat ccagcttact ggcggttttg gctgtgtgtt agtgtggtct
 121 acgaattgtt tctcatcttc atccttttcc agacagtcca ggatggccga cagtttctga
 181 agtatgtgga tcccaggctg ggagtcccat tgccagagag ggactacggg ggcaactgcc
 241 tcatctatga tgctgacaac aagactgacc ctttccacaa catctgggac aagctggatg
 301 gctttgttcc tgcacacttc attggctggt atctgaagac gctcatgatc cgtgactggt
 361 ggatgtgcat gatcatcagt gtgatgttcg agttcctgga gtacagcctg gagcaccagc
 421 tgcccaactt cagcgagtgc tggtgggacc attggatcat ggacgtcctc gtctgcaacg
 481 ggctgggcat ctactgtggc atgaagaccc tcgagtggct gtccctgaag acatataagt
 541 ggcagggcct ctggaacatt ccaacctaca agggcaagat gaagaggatt gcctttcagt
 601 tcacgcctta cagctgggta cgctttgagt ggaagccagc ctccagcctg caccgctggc
 661 tggccgtgtg tggcatcatc ctggtgttcc tgctggcaga gctgaacacc ttctacctga
 721 agtttgtgct atggatgccc cctgaacact acctggtcct tctgaggctg gtcttcttcg
 781 tgaacgtggg tggtgtggcc atgcgtgaga tctacgactt catggatgaa ttgaagcccc
 841 acaggaagct gggccagcag gcctggctgg tggcagccat cacagtcaca gagcttctca
 901 tcgtggtgaa gtatgacccg cacacactca ccctgtcact gcccttctac atctcccagt
 961 gctggactct tggctccatc ctggtgctta catggactgt ctggcgcttc ttcctgcggg
1021 acatcaccat gaggtacaag gagacccggc gacagaagca gcagagtcac caggccagag
1081 ccgtcaacaa ccgggatggg caccctgggc cagatgatga cctgctaggg actggaactg
1141 cagaagaaga ggggaccacc aatgacggtg tgactgctga ggaggggacc tcagccgcgt
1201 catgagcctc accctcgtca ctggccttgt gccaaggggc tgtcccattt ctcccttcct
1261 cgtgctccct gcttcagagg caggggtggg gggggcatcc acactccagg aggggcacct
1321 gagaatacat gtttgtgtgc aggtaggtgt atgcacatat tggctcctga cactactttg
1381 gggccatgag ctgaacagtg agcctggaac ttgctctaga gcaagagtcc tttctacctg
1441 gtcaacaaag gccctggtcc atggtctccc ttgtgctcaa tctcagcacc ggctagggga
```

**FIGURE 27 (page 1 of 2)**

1501 ggtatcttgg tatctcggta ctctttctcc actctttatg gggtaggcag aagccccatg

1561 agaccctgtg gtcccaccca cttacagcag cataagtgaa ggatatcata ataccaacat

1621 gtctgcaaag tggtgggtct agagtcagca ctgagccatt tcctttggag ccttccttta

1681 accacgcagg actataaact gaatggtgta tacatgacag tcacagattg gccttttgcg

1741 gccagagagc ccaacttgga gacctgtacc ccaggtgcca gggtgctgtc accatggctc

1801 ccttgagcaa atggaacaaa taaagtgatg atgaaggtga aaaaaaaaaa aaaaaaaaa

**FIGURE 27 continued (page 2 of 2)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100092470 A
- WO 2010033913 A
- US 7371849 B
- US 6838254 B1
- US 6765087 B
- US 6005079 A
- US 5800988 A
- WO 2002048193 A
- EP 1264885 A
- WO 2001090190 A
- WO 2000043507 A
- EP 1024191 A
- WO 1999042077 A
- US 20070117151 A
- EP 1978035 A
- US 20060246477 A


### Non-patent literature cited in the description


- *Nature,* 2003, vol. 422, 226
- **GREENBERG, A. S. ; AVILA, D. ; HUGHES, M. ; HUGHES, A. ; MCKINNEY, E. ; FLAJNIK, M. F.** *Nature,* 1995, vol. 374, 168-173
- *Mol. Immunol.,* 2001, vol. 38, 313-326
- *Comp. Biochem. Physiol. B.,* 1973, vol. 15, 225
- **DIAZ, M. ; STANFIELD, R. L. ; GREENBERG, A. S. ; FLAJNIK, M. F.** *Immunogenetics,* 2002, vol. 54, 501-512
- **NUTTALL, S. D. ; KRISHNAN, U. V. ; DOUGHTY, L. ; PEARSON, K. ; RYAN, M. T. ; HOOGENRAAD, N. J. ; HATTARKI, M. ; CARMICHAEL, J. A. ; IRVING, R. A. ; HUDSON, P. J.** *Eur. J. Biochem.,* 2003, vol. 270, 3543-3554
- **AZWAI SM et al.** Serology of Orthopoxvirus Camel Infection in Dromedary camels. *Comp. Immun. Microbiol. Infect. Dis.,* 1996, vol. 19, 65
- **KELLY PJ et al.** Isolation and characterization of immunoglobulin-G and IgG Subclasses of the African elephant. *Comp. Immun. Microbiol. Infect. Dis.,* 1998, vol. 2J, 65
- **LINDEN RICHARD VAN DER et al.** Induction of immune responses and molecular cloning of the heavy-chain antibody repertoire of Lama glama. *J. Immun. Methods,* 2000, vol. 240, 185
- **RIVERA H et al.** Serological survey of viral antibodies in the Peruvian alpacas. *Am. J. Vet. Res.,* 1987, vol. 48, 189
- **KUMAR M et al.** Biochemical studies on Indian Camels: Blood proteins and lipids. *J. Sci. Industrial Res.,* 1961, vol. 20c, 236
- **UNGAR-WARON H. ; ELIAS E. et al.** Dromedary IgG: Prurification, characterization and quantitation in Sera of Dams and newborns. *Isr. J. Vet. Med.,* 1987, vol. 43 (3), 198
- **AZWAI SM et al.** The isolation and characterization of camel immunoglobulin classes and subclasses. *J. Comp. Path.,* 1993, vol. 109, 187
- **HAMERS-CASTERMAN C ; ATARHOUCH T. et al.** Naturally occurring antibodies devoid of light chains. *Nature,* 1993, vol. 363, 446
- **ZHANG J ; TANHA J. et al.** Pentamerization of single-domain antibodies from phage libraries: a novel strategy for the rapid generation of high-avidity antibody reagents. *J. Mol. Biol,* 2004, vol. 335, 49
- **SAERENS D et al.** Engineering Camel Single-Domain Antibodies and Immobilization Chemistry for Human Prostate-Specific Antigen Sensing. *Anal. Chem.,* 2005, vol. 77, 7547-7555
- **SAERENS D. et al.** Identification of a Universal VHH Framework to Graft Non-canonical Antigen-binding Loops of Camel Single-domain antibodies. *JMB,* 2005, vol. 352, 597-607
- **SHERWOOD, L J.** Rapid assembly of Sensitive Antigen-Capturi Assays for Marburg Virus, Using In Vitro Selection of Llama Single-Domain Antibodies, at Biosafety level 4. *JID,* 2007, vol. 296 (2), 213-219
- **RYAN S.** Single-Domain Antibody-Nanoparticles: Promising Architechtures for Increased Staphylococcus aureus Detection Specificity and Sensitivity. *Bioconjugate Chem,* 2009, vol. 20, 1966-1974
- Intracellular Antibodies: Research and Disease Applications. Springer-Verlag New York, Inc, 1998
- Intracellular Antibodies: Research and Disease Applications. Springer-Verlag, 1998
- *Nature Biotechnology,* 2005, vol. 23, 1126
- *Biochim. Biophys. Acta.,* 1999, vol. 141, 7
- *Protein Engineering,* 1994, vol. 7, 1129

- *Nature Biotech.,* 2005, vol. 23, 1126
- **S. MUYLDERMANS.** *Reviews in Mol. Biotech.,* 2001, vol. 74, 277
- *PNAS USA,* 2004, vol. 101, 12444
- *Proteins,* 2005, vol. 55, 187
- **K. DECANNIERE et al.** *Structure,* 2000, vol. 7, 361
- *Nature Structural Biol,* 1996, vol. 9, 803
- *J. Mol. Biol,* 2001, vol. 311, 123
- *Eur. J. Immunol,* 2005, vol. 35, 936
- **NGUYEN et al.** *EMBO J.,* 2000, vol. 19, 921
- **HARMSEN et al.** *Mol. Immun.,* 2000, vol. 37, 579
- **M. LAUWEREYS et al.** *EMBO, J.,* 1998, vol. 17, 3512
- **F. MARTIN et al.** *Prot. Eng.,* 1997, vol. 10, 607
- **MURPHY et al.** *Am. J. Vet. Res.,* 1989, vol. 50, 1279
- *EMBO, J.,* 1998, vol. 17, 3512
- *J. Immunol. Methods,* 2000, vol. 240, 185
- **SURAN et al.** *J. Immunology,* 1967, vol. 99, 679
- **ANDRIS-WIDHOPF J. et al.** *J. Immunology Methods,* 2002, vol. 242, 159